(19) Europäisches Patentamt
European Patent Office
Office européen des brevets

(11) Publication number: 0 514 192 A1

(12) # EUROPEAN PATENT APPLICATION

(21) Application number: 92304404.4

(22) Date of filing: 15.05.92

(51) Int. Cl.[5]: C07D 405/14, C07D 457/04, C07D 405/06, A61K 31/55, A61K 31/505, A61K 31/415

(30) Priority: 16.05.91 GB 9110634
16.05.91 GB 9110638
16.05.91 GB 9110621

(43) Date of publication of application:
19.11.92 Bulletin 92/47

(84) Designated Contracting States:
AT BE CH DE DK ES FR GB GR IT LI LU MC NL PT SE

(71) Applicant: GLAXO GROUP LIMITED
Glaxo House, Berkeley Avenue
Greenford, Middlesex UB6 0NN (GB)

(72) Inventor: Ross, Barry Clive
Glaxo Group Research Limited, Park Road
Ware, Hertfordshire SG12 0DG (GB)
Inventor: Middlemiss, David
Glaxo Group Research Limited, Park Road
Ware, Hertfordshire SG12 0DG (GB)
Inventor: Scopes, David Ian Carter
Glaxo Group Research Limited, Park Road
Ware, Hertfordshire SG12 0DG (GB)
Inventor: Jack, Torquil Iain MacLean
Glaxo Group Research Limited, Park Road
Ware, Hertfordshire SG12 0DG (GB)
Inventor: Cardwell, Kevin Stuart
Glaxo Group Research Limited, Park Road
Ware, Hertfordshire SG12 0DG (GB)
Inventor: Dowle, Michael Dennis
Glaxo Group Research Limited, Park Road
Ware, Hertfordshire SG12 0DG (GB)
Inventor: Montana, John Gary
Glaxo Group Research Limited, Park Road
Ware, Hertfordshire SG12 0DG (GB)
Inventor: Watson, Stephen Paul
Glaxo Group Research Limited, Park Road
Ware, Hertfordshire SG12 0DG (GB)
Inventor: Panchal, Terence Aaron
Glaxo Group Research Limited, Park Road
Ware, Hertfordshire SG12 0DG (GB)
Inventor: Hirst, Gavin Charles, Glaxo Inc.
Five Moore Drive, Box 13398
Res. Triangle Park, North Carolina 27709 (US)

(74) Representative: James, Stephen Richard, Dr. et al
Glaxo Holdings plc Glaxo House Berkeley Avenue
Greenford, Middlesex UB6 0NN (GB)

(54) **Antihypertensive benzofuran derivatives, substituted by varied N-pyrimidinyl- or N-imidazolyl-methyl groups.**

(57) The invention provides compounds of the general formula (I):

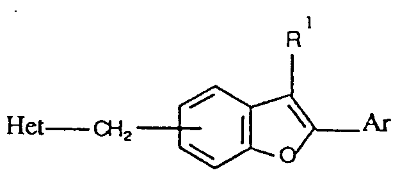

(I)

or a physiologically acceptable salt, solvate or metabolically labile ester thereof wherein $R^1$ and Ar are described in the Claims and
Het represents a group selected from

(a) 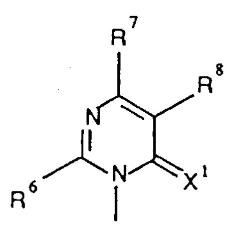 ,

(b) 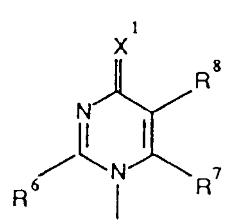

EP 0 514 192 A1

(c) 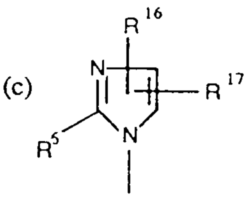 ,

(d) 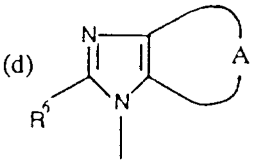

or

(e) 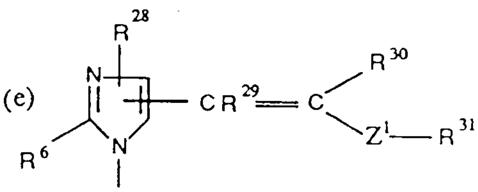

and $R^6$, $R^7$, $R^8$, $R^{16}$, $R^{17}$, $R^{28}$, $R^{29}$, $R^{30}$, $R^{31}$, $X^1$, $Z^1$ and A are a variety of substituents and groups.

The compounds may be used in the treatment or prophylaxis of hypertension and diseases associated with cognitive disorders.

This invention relates to benzofuran derivatives, processes for their preparation and pharmaceutical compositions containing them. According to a first aspect of the invention we provide a compound of the general formula (I):

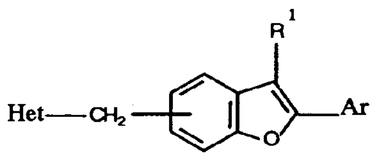

(I)

or a physiologically acceptable salt, solvate (e.g. hydrate) or metabolically labile ester thereof in which

$R^1$ represents a hydrogen atom or a halogen atom or a group selected from $C_{1-6}$alkyl, $C_{2-6}$alkenyl, fluoro$C_{1-6}$alkyl, $C_{1-6}$alkoxy ,-CHO, $-CO_2H$ or $-COR^2$;

Ar represents the group

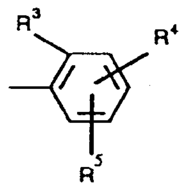

;

$R^2$ represents a group selected from $C_{1-6}$alkyl, $C_{2-6}$alkenyl, $C_{1-6}$ alkoxy or the group $-NR^{14}R^{15}$;

$R^3$ represents a group selected from $-CO_2H$, $-NHSO_2CF_3$ or a C-linked tetrazolyl group;

$R^4$ and $R^5$ which may be the same or different each independently represent a hydrogen atom or a halogen atom or a $C_{1-6}$alkyl group;

Het represents a group selected from

(a) 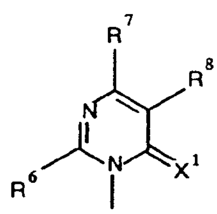 ,

(b) 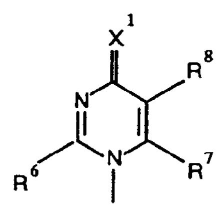

(c) 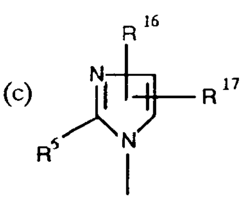 ,

(d) 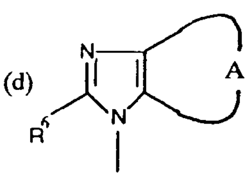 or

(e) 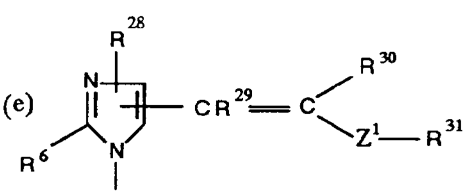

A represents, when read in a clockwise or anti-clockwise direction, a group selected from

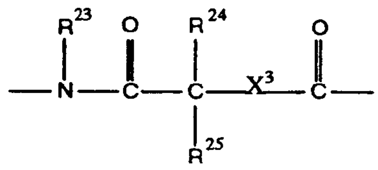 ,

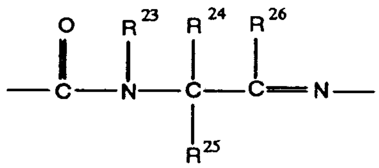 , 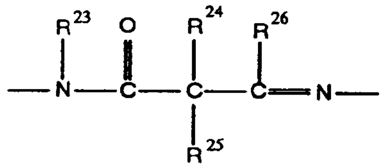 ,

or 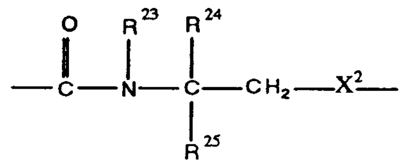 ;

$R^6$ represents a hydrogen atom or a group selected from $C_{1-6}$alkyl, $C_{2-6}$ alkenyl $C_{1-6}$alkylthio, $C_{1-6}$alkoxy, $C_{3-7}$cycloalkyl or $C_{3-7}$cycloalkyl$C_{1-4}$alkyl;
$R^7$ represents a hydrogen atom, a halogen atom or a group selected from $C_{1-6}$alkyl, $C_{2-6}$alkenyl, fluoro$C_{1-6}$alkyl $-(O)_m(CH_2)_nR^{10}$, $-(CH_2)_pCOR^{11}$ or $-(CH_2)_qNR^{12}COR^{13}$;
$R^8$ represents a hydrogen atom, a halogen atom or a group selected from $C_{1-6}$alkyl, $C_{2-6}$alkenyl, fluoro$C_{1-6}$alkyl, $-(CH_2)_rR^{10}$ or $-(CH_2)_pCOR^{11}$;
$R^9$ represents a hydrogen atom or a $C_{1-4}$alkyl group;
$R^{10}$ represents a group selected from hydroxy, $C_{1-6}$alkoxy, $C_{1-6}$alkylthio, phenyl or the group $-NR^{14}R^{15}$;
$R^{11}$ represents a hydrogen atom or a group selected from hydroxy, $C_{1-6}$alkyl, $C_{1-6}$alkoxy, phenyl, phenoxy or the group $-NR^{14}R^{15}$;
$R^{12}$ represents a hydrogen atom or a $C_{1-6}$alkyl group,
$R^{13}$ represents a hydrogen atom or a group selected from $C_{1-6}$alkyl, $C_{1-6}$alkoxy, phenyl, phenoxy or the group $-NR^{14}R^{15}$;
$R^{14}$ and $R^{15}$, which may be the same or different, each independently represent a hydrogen atom or a $C_{1-4}$alkyl group or $-NR^{14}R^{15}$ forms a saturated heterocyclic ring which has 5 or 6 ring members and may optionally contain in the ring one oxygen atom;
$R^{16}$ represents a group selected from $-NR^{18}COR^{19}$, $-NHCOR^{19}$, $-NR^{18}SO_2R^{19}$, $-NHSO_2(C_{1-4}alkyl)$ or $-NR^9R^{20}$;
$R^{17}$represents a group selected from $-CO_2R^{12}$, $-CONHSO_2R^{21}$ or $-CONR^9R^{20}$;
$R^{18}$ represents a group selected from $C_{1-6}$alkyl, aryl or arylmethyl, wherein 'aryl' represents a phenyl group optionally substituted by 1 or 2 substituents selected from a halogen atom or a group selected from $-NO_2$, $-CF_3$, $C_{1-4}$alkyl, $C_{1-4}$alkoxy, $-NR^9R^9$, $-NHCO_2(C_{1-4}alkyl)$, $-CO_2R^9$ or hydroxy;
$R^{19}$ represents a group selected from aryl (as defined above), fluoro$C_{1-4}$alkyl, $-NR^9R^9$, $C_{3-5}$cycloalkyl, heteroaryl,

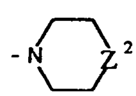

or $C_{1-4}$ optionally substituted by aryl (as defined above), $-NR^9R^9$, $-NCO_2R^{22}$ or $-CO_2R^9$, wherein 'heteroaryl' represents a 5 or 6 membered aromatic ring containing from 1 to 3 heteroatoms selected from the group consisting of N, O or S, said aromatic ring being optionally substituted by 1 or 2 substituents selected from a halogen atom or a group selected from hydroxy, -SH, $C_{1-4}$alkyl, $C_{1-4}$alkoxy, $CF_3$, $-NO_2$, $-CO_2R^9$ or $-NR^9R^9$;
$R^{20}$ represents a hydrogen atom or a group selected from aryl (as defined above) or $C_{1-6}$alkyl optionally substituted by a group selected from aryl (as defined above), hydroxy, $-CO_2R^9$ or $-NR^9R^9$;
$R^{21}$ represents a group selected from aryl (as defined above), heteroaryl (as defined above), $C_{3-7}$cycloalkyl, fluoro$C_{1-4}$alkyl or a $C_{1-4}$alkyl group optionally substituted by a halogen atom or a group selected from aryl (as

defined above), heteroaryl (as defined above), hydroxy, -SH, $C_{1-4}$alkyl, $C_{1-4}$alkoxy, $C_{1-4}$alkylthio, $-CF_3$, $-NO_2$, $-CO_2R^9$, $-NR^9R^9$, $-PO_3H$, -PO(OH)($C_{1-4}$alkoxy);

$R^{22}$ represents a hydrogen atom or a group selected from $C_{1-6}$alkyl, $C_{3-7}$cycloalkyl, aryl or arylmethyl (wherein 'aryl' is as defined above);

$R^{23}$ represents a hydrogen atom or a $C_{1-6}$alkyl group optionally substituted by a hydroxy group;

$R^{24}$ and $R^{25}$, which may be the same or different, each independently represent a hydrogen atom or a group selected from $-CO_2R^{12}$, a $C_{1-6}$alkyl, $C_{2-6}$alkenyl or $C_{2-6}$alkynyl group each of which is optionally substituted by a group selected from hydroxy, $C_{1-4}$alkoxy, $-NR^9R^9$, $-CONR^9R^9$, $-CO2R^{27}$, $-OC(O)R^{27}$, guanidino or $C_{1-4}$alkylthio, a phenyl or phenyl-$C_{1-4}$alkyl group, each of which is optionally substituted by a halogen atom or a group selected from hydroxy, $C_{1-4}$alkyl or $C_{1-4}$alkoxy, or an imidazolyl-$C_{1-4}$alkyl or indolyl- $C_{1-4}$alkyl group;

$R^{26}$ and $R^{27}$, which may be the same or different, each independently represent a hydrogen atom or a group selected from $C_{1-6}$alkyl, phenyl or benzyl;

$R^{28}$ represents a hydrogen atom or a halogen atom or a group selected from $C_{1-6}$alkyl, $C_{2-6}$alkenyl or fluoro$C_{1-6}$alkyl;

$R^{29}$ and $R^{30}$, which may be the same or different, each independently represent a hydrogen atom or a group selected from $C_{1-6}$alkyl, $C_{3-7}$cycloalkyl, thienyl-Y-, pyrazolyl-Y-, imidazolyl-Y, thiazolyl-Y, furyl-Y-, pyrrolyl-Y-, triazolyl-Y-, oxazolyl-Y-, isoxazolyl-Y, pyridyl-Y-, phenyl-Y- or tetrazolyl-Y- wherein each aryl or heterocyclic ring is optionally substituted by a halogen atom or group selected from $C_{1-6}$alkyl, fluoro$C_{1-6}$alkyl, $C_{1-6}$alkoxy, hydroxy, $-NR^{27}R^{27}$, $-CO_2R^{27}$, $-CONR^{27}R^{27}$, $-SO_2NHR^{27}$ or $-SO_3H$;

$R^{31}$ represents $-COR^{11}$, a C-linked tetrazol-5-yl group or the group $-NHSO_2CF_3$;

$X^1$ represents an oxygen atom or sulphur atom or the group $-NR^9$-;

$X^2$ represents an oxygen or sulphur atom or the group -N($R^{26}$)-;

$X^3$ represents an oxygen atom or the group -N($R^{23}$)-;

Y represents a bond or an oxygen or sulphur atom, or a $C_{1-6}$alkyl linkage which is optionally substituted by phenyl or benzyl wherein each aryl ring is optionally substituted by a halogen atom or a group selected from $-NO_2$, $-CF_3$, $C_{1-4}$alkyl, $C_{1-4}$alkoxy, -CN or $CO_2R^{27}$;

$Z^1$ represents a bond or a methylene, ethylene or vinyl linkage, each being optionally substituted by a $C_{1-4}$alkyl group;

$Z^2$ represents an oxygen or sulphur atom or a group selected from -N($R^9$)-, -S(O)- or $-S(O)_2$-;

m represents zero or 1;

n represents zero or an integer from 1 to 4;

p represents zero or an integer from 1 to 4;

q represents an integer from 1 to 4; and

r represents an integer from 1 to 4,

provided that when m is 1, n is an integer from 1 to 4;

Where optical isomers may exist formula (I) is intended to cover all enantiomers, diastereoisomers and mixtures thereof including racemates. Compounds containing one or two double bonds may exist in the cis or trans configuration.

The invention also includes within its scope the solvates, especially the hydrates of compounds of general formula (I).

Within the above definition the term 'alkyl', 'alkoxy' 'alkylthio' as a group or part of a group means that the group is straight or branched. The term 'alkenyl' as a group or part of a group means that the group is straight or branched and contains at least one carbon-carbon double bond. The term 'cycloalkyl' as a group or part of a group may be, for example, a cyclopropyl, cyclobutyl, cyclopentyl or cyclohexyl group.

The term 'halogen' means a fluorine, chlorine, bromine or iodine atom.

The term 'fluoro$C_{1-6}$alkyl' means a $C_{1-6}$alkyl group in which one or more hydrogen atoms have been replaced by a fluorine atom, for example, $-CH_2CF_3$.

Within the above definition when $-NR^{14}R^{15}$ represents a saturated heterocyclic ring, this contains 5 or 6 ring members, one of which may be an oxygen atom. Suitable heterocyclic groups are a pyrrolidino, piperidino or morpholino group.

A particularly preferred class of compounds of general formula (I) is that wherein $R^6$ represents a hydrogen atom or a $C_{1-5}$alkyl, $C_{3-5}$alkenyl, $C_{1-5}$alkoxy, $C_{3-5}$cycloalkyl or $C_{3-5}$cycloalkyl$C_{1-2}$alkyl group. Particularly preferred are those compounds wherein $R^6$ is a $C_{2-4}$alkyl group (especially an ethyl, n-propyl or n-butyl group).

In preferred classes of compounds of general formula (I) in which Het represents either group (a) or group (b),

$X^1$ represents an oxygen atom;

$R^6$ represents a hydrogen atom, a $C_{1-5}$alkyl group, particularly a $C_{2-4}$alkyl group, especially an ethyl, n-propyl or n-butyl group, a $C_{3-5}$alkyenyl group, a $C_{3-5}$cycloalkyl group, especially a cyclopropyl or cyclobutyl group, or a $C_{3-5}$cycloalkyl$C_{1-2}$alkyl group, especially a cyclopropylmethyl group;
$R^7$ represents a hydrogen atom a $C_{1-4}$alkyl, especially a methyl group, $-(O)_m(CH_2)_nR^{10}$ or $-(CH_2)_pCOR^{11}$;
$R^8$ represents a hydrogen atom, a halogen, especially chlorine, atom, a $C_{1-4}$alkyl, especially a methyl, group, $-(CH_2)_rR^{10}$ or $-(CH_2)_pCOR^{11}$;
$R^{10}$ represents hydroxy, a $C_{1-4}$alkoxy, especially a methoxy, group, a $C_{1-4}$alkylthio, especially a methylthio group, phenyl or $-NR^{14}R^{15}$;
$R^{11}$ represents a hydroxy, a $C_{1-4}$alkoxy, especially a methoxy or ethoxy, group or $-NR^{14}R^{15}$;
$R^{14}$ and $R^{15}$, which may be the same or different, each independently represent a hydrogen atom or a $C_{1-4}$alkyl, especially a methyl or, ethyl, group or $-NR^{14}R^{15}$ forms a morpholino group;
m represents zero or 1;
n represents zero, 1 or 2;
p represents zero, 1 or 2, especially zero or 1; and
r represents 1 or 2.

In particularly preferred compounds of general formula (I) in which Het represents either group (a) or group (b),
$X^1$ represents an oxygen atom;
$R^6$ represents a $C_{2-4}$alkyl, especially an ethyl or n-propyl, group;
$R^7$ represents a hydrogen atom, a $C_{1-4}$alkyl, especially a methyl, group, or $-(CH_2)_pCOR^{11}$;
$R^8$ represents a hydrogen atom, a halogen, especially a chlorine, atom, or a $C_{1-4}$alkyl, especially a methyl, group;
$R^{11}$ represents hydroxy or $-NR^{14}R^{15}$;
$R^{14}$ and $R^{15}$ represents a hydrogen atom or a $C_{1-4}$alkyl, especially a methyl or ethyl, group; and
p represents zero.

Another preferred class of compounds of general formula (I) in which Het represents the group (c) is that wherein $R^{16}$ represents $-NR^9R^{20}$ wherein, in particular, $R^9$ represents a hydrogen atom and $R^{20}$ represents a hydrogen atom or a $C_{1-6}$alkyl group optionally substituted by a hydroxy group.

A further preferred class of compounds of general formula (I) in which Het represents the group (c) is that wherein $R^{17}$ represents the group $-CO_2R^{12}$ or $-CONR^9R^{20}$. In particular, $R^{12}$ preferably represents a hydrogen atom or a $C_{1-4}$alkyl group, $R^9$ preferably represents a hydrogen atom and $R^{20}$ preferably represents a hydrogen atom or a $C_{1-6}$alkyl group optionally substituted by a hydroxy group.

A yet further preferred class of compounds of general formula (I) in which Het represents the group (d) is that wherein A represents the group

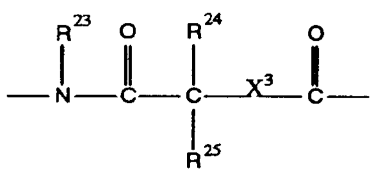

or

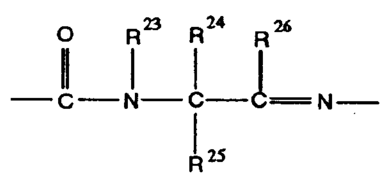

;

In particular, $R^{23}$ preferably represents a hydrogen atom, $R^{24}$ preferably represents a hydrogen atom, $R^{25}$ preferably represents a hydrogen atom, a $C_{1-6}$alkyl group (optionally substituted by a group selected from hydroxy, amino, guanidino, $C_{1-4}$alkylthio, $-CONR^9R^9$ (wherein $-NR^9R^9$ represents an $NH_2$ group), $-CO_2R^{27}$ (wherein $R^{27}$ represents a hydrogen atom or a $C_{1-4}$alkyl group) or $-OC(O)R^{27}$ (wherein $R^{27}$ represents a hydrogen atom or a $C_{1-4}$alkyl group)), or a phenyl, benzyl, 4-hydroxybenzyl, 4-imidazolylmethyl or 3- indolylmethyl group, $R^{26}$ preferably represents a hydrogen atom, and $X^3$ preferably represents $-N(R^{23})-$.

A further preferred class of compounds of general formula (I) in which Het represents the group (e) is that wherein $R^{28}$ represents a hydrogen atom, a halogen atom (especially chlorine) or a $C_{1-4}$alkyl group (especially methyl).

Another preferred class of compounds of general formula (I) in which Het represents the group (e) is that wherein $R^{29}$ represents a hydrogen atom or a $C_{1-4}$alkyl group (especially methyl or ethyl).

A further preferred class of compounds of general formula (I) in which Het represents the group (e) is that wherein $R^{30}$ represents a group selected from thienyl-Y-, imidazolyl-Y-, furyl-Y-, Pyridyl- Y-, or phenyl-Y- wherein each aryl or heterocyclic group is optionally substituted by a hydroxy group or a $C_{1-4}$alkyl (especially

methyl) or $C_{1-4}$alkoxy (especially methoxy) group, and Y represents a bond or a methylene linkage.

Another preferred class of compounds of general formula (I) in which Het represents the group (e) is that wherein $R^{31}$ represents the group $COR^{11}$ wherein $R^{11}$ represents a hydroxy, $C_{1-4}$alkoxy (especially methoxy or ethoxy) or the group $-NR^{14}R^{15}$ (especially wherein $R^{14}$ and $R^{15}$ each independently represent a hydrogen atom or a $C_{1-4}$alkyl (e.g. methyl or ethyl) group), or $R^{31}$ represents a C- linked tetrazol-5-yl group, and $Z^1$ represents a bond or a methylene linkage.

In another preferred class of compounds of general formula (I), $R^1$ represents a hydrogen atom or a halogen atom or a group selected from $C_{1-6}$alkyl, fluoro$C_{1-6}$alkyl or $C_{1-6}$alkoxy, and in particular a halogen atom, especially bromine.

Conveniently, in the compounds of general formula (I), the group Het-$CH_2$- is attached at the 5- or 6-position on the benzofuran ring, and especially the 5-position.

Also conveniently, in the compounds of general formula (I), $R^3$ may be a C-linked tetrazolyl group or the group $NHSO_2CF_3$.

Still conveniently, in the compounds of general formula (I), $R^4$ and $R^5$ may each independently represent a hydrogen atom or a halogen atom. In particular $R^4$ and $R^5$ each represent hydrogen atoms.

Particularly preferred compounds of the present invention in which Het represents either group (a) or group (b) include:

3-[[3-bromo-2-[2-(1H-tetrazol-5-yl)phenyl]-5-benzofuranyl]methyl]-2-ethyl-4(3H)-pyrimidinone;
3-[[3-bromo-2-[2-(1H-tetrazol-5-yl)phenyl]-5-benzofuranyl]methyl]-2-ethyl-6-methyl-4(3H)-pyrimidinone;
3-[[3-bromo-2-[2-(1H-tetrazol-5-yl)phenyl]-5-benzofuranyl]methyl]-2-ethyl-5-methyl-4(3H)-pyrimidinone;
3-[[3-bromo-2-[2-(1H-tetrazol-5-yl)phenyl]-5-benzofuranyl]methyl]-2-ethyl-5,6-dimethyl-4(3H)-pyrimidinone;
3-[[3-bromo-2-[2-[[(trifluoromethyl)sulphonyl]amino]phenyl]-5-benzofuranyl]methyl]-2-ethyl-4(3H)-pyrimidinone;
3-[[3-bromo-2-[2-[[(trifluoromethyl)sulphonyl]amino]phenyl]-5-benzofuranyl]methyl]-2-ethyl-6-methyl-4(3H)-pyrimidinone;
3-[[3-bromo-2-[2-[[(trifluoromethyl)sulphonyl]amino]phenyl]-5-benzofuranyl]methyl]-2-ethyl-5-methyl-4(3H)-pyrimidinone;
3-[[3-bromo-2-[2-[[(trifluoromethyl)sulphonyl]amino]phenyl]-5-benzofuranyl]methyl]-2-ethyl-5,6-dimethyl-4(3H)-pyrimidinone;
3-[[3-bromo-2-[2-(1H-tetrazol-5-yl)phenyl]-5-benzofuranyl]methyl]-2-propyl-4(3H)-pyrimidinone;
3-[[3-bromo-2-[2-(1H-tetrazol-5-yl)phenyl]-5-benzofuranyl]methyl]-6-methyl-2-propyl-4(3H)-pyrimidinone;
3-[[3-bromo-2-[2-(1H-tetrazol-5-yl)phenyl]-5-benzofuranyl]methyl]-5-methyl-2-propyl-4(3H)-pyrimidinone;
3-[[3-bromo-2-[2-(1H-tetrazol-5-yl)phenyl]-5-benzofuranyl]methyl]-5,6-dimethyl-2-propyl-4(3H)-pyrimidinone;
3-[[3-bromo-2-[2-[[(trifluoromethyl)sulphonyl]amino]phenyl]-5-benzofuranyl]methyl]-2-propyl-4(3H)-pyrimidinone;
3-[[3-bromo-2-[2-[[(trifluoromethyl)sulphonyl]amino]phenyl]-5-benzofuranyl]methyl]-6-methyl-2-propyl-4(3H)-pyrimidinone;
3-[[3-bromo-2-[2-[[(trifluoromethyl)sulphonyl]amino]phenyl]-5-benzofuranyl]methyl]-5-methyl-2-propyl-4(3H)-pyrimidinone;
3-[[3-bromo-2-[2-[[(trifluoromethyl)sulphonyl]amino]phenyl]-5-benzofuranyl]methyl]-5,6-dimethyl-2-propyl-4(3H)-pyrimidinone;
1-[[3-bromo-2-[2-(1H-tetrazol-5-yl)phenyl]-5-benzofuranyl]methyl]-2-ethyl-4(1H)-pyrimidinone;
1-[[3-bromo-2-[2-(1H-tetrazol-5-yl)phenyl]-5-benzofuranyl]methyl]-2-propyl-4(1H)-pyrimidinone;
1-[[3-bromo-2-[2-[[(trifluoromethyl)sulphonyl]amino]phenyl]-5-benzofuranyl]methyl]-2-ethyl-4(1H)-pyrimidinone;
1-[[3-bromo-2-[2-[[(trifluoromethyl)sulphonyl]amino]phenyl]-5-benzofuranyl]methyl]-2-propyl-4(3H)-pyrimidinone;
3-[[3-bromo-2-[2-(1H-tetrazol-5-yl)phenyl]-5-benzofuranyl]methyl]-2-ethyl-3,6-dihydro-6-oxo-4-pyrimidinecarboxylic acid;
3-[[3-bromo-2-[2-(1H-tetrazol-5-yl)phenyl]-5-benzofuranyl]methyl]-3,6-dihydro-6-oxo-2-propyl-4-pyrimidinecarboxylic acid;
3-[[3-bromo-2-[2-(1H-tetrazol-5-yl)phenyl]-5-benzofuranyl]methyl]-5-chloro-2-ethyl-3,6-dihydro-6-oxo-4-pyrimidinecarboxylic acid;
3-[[3-bromo-2-[2-(1H-tetrazol-5-yl)phenyl]-5-benzofuranyl]methyl]-5-chloro-3,6-dihydro-6-oxo-2-propyl-4-pyrimidinecarboxylic acid;

3-[[3-bromo-2-[2-[[(trifluoromethyl)sulphonyl]amino]phenyl]-5-benzofuranyl]methyl]-2-ethyl-3,6-dihydro-6-oxo-4-pyrimidinecarboxamide;

3-[[3-bromo-2-[2-[[(trifluoromethyl)sulphonyl]amino]phenyl]-5-benzofuranyl]methyl]-2-ethyl-3,6-dihydro-N-methyl-6-oxo-4-pyrimidine carboxamide;

3-[[3-bromo-2-[2-[[(trifluoromethyl)sulphonyl]amino]phenyl]-5-benzofuranyl]methyl]-N,2-diethyl-3,6-dihydro-6-oxo-4-pyrimidine carboxamide;

3-[[3-bromo-2-[2-[[(trifluoromethyl)sulphonyl]amino]phenyl]-5-benzofuranyl]methyl]-3,6-dihydro-6-oxo-2-propyl-4-pyrimidine carboxamide;

3-[[3-bromo-2-[2-[[(trifluoromethyl)sulphonyl]amino]phenyl]-5-benzofuranyl]methyl]-3,6-dihydro-N-methyl-6-oxo-2-propyl-4-pyrimidinecarboxamide;

3-[[3-bromo-2-[2-[[(trifluoromethyl)sulphonyl]amino]phenyl]-5-benzofuranyl]methyl]-N-ethyl-3,6-dihydro-6-oxo-2-propyl-4-pyrimidinecarboxamide;

3-[[3-bromo-2-[2-[[(trifluoromethyl)sulphonyl]amino]phenyl]-5-benzofuranyl]methyl]-5-chloro-2-ethyl-3,6-dihydro-6-oxo-4-pyrimidinecarboxamide;

3-[[3-bromo-2-[2-[[(trifluoromethyl)sulphonyl]amino]phenyl]-5-benzofuranyl]methyl]-5-chloro-2-ethyl-3,6-dihydro-N-methyl-6-oxo-4-pyrimidinecarboxamide;

3-[[3-bromo-2-[2-[[(trifluoromethyl)sulphonyl]amino]phenyl]-5-benzofuranyl]methyl]-5-chloro-N,2-diethyl-3,6-dihydro-6-oxo-4-pyrimidinecarboxamide;

3-[[3-bromo-2-[2-[[(trifluoromethyl)sulphonyl]amino]phenyl]-5-benzofuranyl]methyl]-5-chloro-3,6-dihydro-6-oxo-2-propyl-4-pyrimidinecarboxamide;

3-[[3-bromo-2-[2-[[(trifluoromethyl)sulphonyl]amino]phenyl]-5-benzofuranyl]methyl]-5-chloro-3,6-dihydro-N-methyl-6-oxo-2-propyl-4-pyrimidinecarboxamide;

3-[[3-bromo-2-[2-[[(trifluoromethyl)sulphonyl]amino]phenyl]-5-benzofuranyl]methyl]-5-chloro-N-ethyl-3,6-dihydro-6-oxo-2-propyl-4-pyrimidinecarboxamide;

and their physiologically acceptable salts, solvates and metabolically labile esters.

Particularly preferred compounds of the invention in which Het represents either the group (c) or group (d) include:

1-[[3-bromo-2-[2-(1H-tetrazol-5-yl)phenyl]-5-benzofuranyl]methyl]-2-ethyl-1,4,6,7-tetrahydro-4-methyl-5,8-dioxoimidazo[4,5-e][1,4] diazepine-6-carboxylic acid;

1-[[3-bromo-2-[2-(1H-tetrazol-5-yl)phenyl]-5-benzofuranyl]methyl]-1,4,6,7-tetrahydro-4-methyl-5,8-dioxo-2-propylimidazo[4,5-e][1,4] diazepine-6-carboxylic acid;

1-[[3-bromo-2-[2-[[(trifluoromethyl)sulphonyl]amino]phenyl]-5-benzofuranyl]methyl]-2-ethyl-1,4,6,7-tetrahydro-4-methyl-5,8-dioxoimidazo[4,5-e][1,4]diazepine-6-carboxylic acid;

1-[[3-bromo-2-[2-[[(trifluoromethyl)sulphonyl]amino]phenyl]-5-benzofuranyl]methyl]-1,4,6,7-tetrahydro-4-methyl-5,8-dioxo-2-propylimidazo[4,5-e][1,4]diazepine-6-carboxylic acid;

1-[[3-bromo-2-[2-(1H-tetrazol-5-yl)phenyl]-5-benzofuranyl]methyl]-2-ethyl-1,4,6,7-tetrahydro-4-methylimidazo[4,5-e][1,4]diazepine-5,8-dione;

1-[[3-bromo-2-[2-(1H-tetrazol-5-yl)phenyl]-5-benzofuranyl]methyl]-1,4,6,7-tetrahydro-4-methyl-2-propylimidazo[4,5-e][1,4]diazepine-5,8-dione;

N-[2-[3-bromo-5-[(2-ethyl-1,4,6,7-tetrahydro-4-methyl-5,8-dioxoimidazo[4,5-e][1,4]diazepin-1-yl)methyl]-2-benzofuranyl]phenyl]-2,2,2-trifluoromethanesulphonamide;

N-[2-[3-bromo-5-[(1,4,6,7-tetrahydro-4-methyl-5,8-dioxo-2-propylimidazo[4,5-e][1,4] diazepin-1-yl)methyl]-2-benzofuranyl]phenyl]-2,2,2-trifluoromethanesulphonamide;

4-amino-1-[[3-bromo-2-[2-[[(trifluoromethyl)sulphonyl]amino]phenyl]-5-benzofuranyl]methyl]-2-ethyl-1H-imidazole-5-carboxamide;

4-amino-1-[[3-bromo-2-[2-[[(trifluoromethyl)sulphonyl]amino]phenyl]-5-benzofuranyl]methyl]-2-propyl-1H-imidazole-5-carboxamide;

1-[[3-bromo-2-[2-[[(trifluoromethyl)sulphonyl]amino]phenyl]-5-benzofuranyl]methyl]-2-ethyl-4-(methylamino)-1H-imidazole-5-carboxamide;

1-[[3-bromo-2-[2-[[(trifluoromethyl)sulphonyl]amino]phenyl]-5-benzofuranyl]methyl]-4-(methylamino)-2-propyl-1H-imidazole-5-carboxamide;

1-[[3-bromo-2-[2-[[(trifluoromethyl)sulphonyl]amino]phenyl]-5-benzofuranyl]methyl]-4-(dimethylamino)-2-ethyl-1H-imidazole-5-carboxamide;

1-[[3-bromo-2-[2-[[(trifluoromethyl)sulphonyl]amino]phenyl]-5-benzofuranyl]methyl]-4-(dimethylamino)-2-propyl]-1H-imidazole-5-carboxamide;

4-amino-1-[[3-bromo-2-[2-[[(trifluoromethyl)sulphonyl]amino]phenyl]-5-benzofuranyl]methyl]-2-ethyl-N-methyl-1H-imidazole-5-carboxamide;

4-amino-1-[[3-bromo-2-[2-[[(trifluoromethyl)sulphonyl]amino]phenyl]-5-benzofuranyl]methyl]-N,2-diethyl-1H-imidazole-5-carboxamide;

4-amino-1-[[3-bromo-2-[2-[[(trifluoromethyl)sulphonyl]amino]phenyl]-5-benzofuranyl]methyl]-N-ethyl-2-propyl-1H-imidazole-5-carboxamide;

1-[[3-bromo-2-[2-[[(trifluoromethyl)sulphonyl]amino]phenyl]-5-benzofuranyl]methyl]-N,2-diethyl-4-(methylamino)-1H-imidazole-5-carboxamide;

1-[[3-bromo-2-[2-[[(trifluoromethyl)sulphonyl]amino]phenyl]-5-benzofuranyl]methyl]-N-ethyl-4-(methylamino)-2-propyl-1H-imidazole-5-carboxamide;

1-[[3-bromo-2-[2-[[(trifluoromethyl)sulphonyl]amino]phenyl]-5-benzofuranyl]methyl]-4-(dimethylamino)-N,2-diethyl-1H-imidazole-5-carboxamide;

1-[[3-bromo-2-[2-[[(trifluoromethyl)sulphonyl]amino]phenyl]-5-benzofuranyl]methyl]-4-(dimethylamino)-N-ethyl-2-propyl-1H-imidazole-5-carboxamide;

4-amino-1-[[3-bromo-2-[2-[[(trifluoromethyl)sulphonyl]amino]phenyl]-5-benzofuranyl]methyl]-N-methyl-2-propyl-1H-imidazole-5-carboxamide;

1-[[3-bromo-2-[2-[[(trifluoromethyl)sulphonyl]amino]phenyl]-5-benzofuranyl]methyl]-2-ethyl-N-methyl-4-(methylamino)-1H-imidazole-5-carboxamide;

1-[[3-bromo-2-[2-[[(trifluoromethyl)sulphonyl]amino]phenyl]-5-benzofuranyl]methyl]-N-methyl-4-(methylamino)-2-propyl-1H-imidazole-5-carboxamide;

1-[[3-bromo-2-[2-[[(trifluoromethyl)sulphonyl]amino]phenyl]-5-benzofuranyl]methyl]-4-(dimethylamino)-2-ethyl-N-methyl-1H-imidazole-5-carboxamide;

1-[[3-bromo-2-[2-[[(trifluoromethyl)sulphonyl]amino]phenyl]-5-benzofuranyl]methyl]-4-(dimethylamino)-N-methyl-2-propyl-1H-imidazole-5-carboxamide;

and physiologically acceptable salts, solvates and metabolically labile esters thereof.

Particularly preferred compounds of the invention in which Het represents the group (e) include:

(E)-α-[[1-[[3-bromo-2-[2-(1H-tetrazol-5-yl)phenyl]-5-benzofuranyl]methyl]-2-propyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-4-chloro-2-propyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-4-methyl-2-propyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-ethyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-4-chloro-2-ethyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-ethyl-4-methyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-butyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-butyl-4-chloro-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-butyl-4-methyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-2-propyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-4-chloro-2-propyl-1H-imidazol-5-yl]methylene]-2-thiophenepropanoic acid;

(E)-α-[[1-[[(3-bromo-2-[2-[[(trifluoromethyl)sulphonyl]amino]phenyl]-5-benzofuranyl]methyl]-4-methyl-2-propyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-2-ethyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-4-chloro-2-ethyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-2-ethyl-4-methyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-2-propyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-4-chloro-2-propyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-4-methyl-2-propyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-2-ethyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-4-chloro-2-ethyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-2-ethyl-4-methyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-2-propyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-4-chloro-2-propyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-4-methyl-2-propyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-2-ethyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-4-chloro-2-ethyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-2-ethyl-4-methyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-2-propyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-4-chloro-2-propyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-4-methyl-2-propyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-2-ethyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-4-chloro-2-ethyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-2-ethyl-4-methyl-1H-imidazol-5-yl]methylene]-N-ethyl-2-thiophenepropanamide;

and physiologically acceptable salts, solvates and metabolically labile esters thereof.

The physiologically acceptable acid addition salts of the compounds of formula (I) may be derived from inorganic or organic acids. Examples of such salts include hydrochlorides, hydrobromides, sulphates, phosphates, benzoates, methanesulphonates or trifluoroacetates.

The compounds may also form salts with suitable bases. Examples of such salts are alkali metal (e.g. sodium or potassium), alkaline earth metal (e.g. calcium or magnesium), ammonium and substituted ammonium (e.g. dimethylammonium, triethylammonium, 2-hydroxyethyldimethylammonium, piperazinium, N,N-dimethylpiperazinium, tetraethylammonium, piperidinium, ethylenediammonium and choline).

It will be appreciated that, for pharmaceutical use, the salts referred to above will be physiologically acceptable, but other salts may find use, for example, in the preparation of the compounds of general formula (I) and the physiologically acceptable salts thereof.

It will be further appreciated that the compounds of general formula (I) may be chemically modified in the form of compounds which in vivo (for example, by enzymic attack) will provide the parent compounds of general formula (I). Such prodrugs may be, for example, physiologically acceptable metabolically labile ester derivatives. These may be formed by esterification, for example of any of the carboxylic acid groups in the parent compound of general formula (I), with prior protection of any other reactive groups present in the molecule. Examples of such esters include lower alkyl esters (e.g. methyl or ethyl esters), alkenyl esters (e.g. vinyl or allyl esters), alkynyl esters(e.g. ethynyl or propynyl esters), alkoxyalkyl esters, (e.g. methoxymethyl or 2-methoxyethyl esters), alkylthioalkyl esters (e.g. methylthiomethyl esters) haloalkyl esters (e.g. 2-iodoethyl or 2,2,2-trichloroethyl esters), alkanoyloxyalkyl esters (e.g. acetoxymethyl, 1-acetoxyethyl or pivaloyloxymethyl esters), alkoxycarbonyloxyalkyl esters (e.g. 1-ethoxycarbonyloxyethyl or 1-methoxycarbonyloxyethyl esters), aroyloxyalkyl esters (e.g. benzoyloxymethyl or 1-benzoyloxyethyl esters), substituted or unsubstituted aralkyl esters (e.g. benzyl or 4-amidobenzyl esters), substituted or unsubstituted aminoalkyl esters (e.g aminoethyl or 2-N,N-dimethylaminoethyl esters) or hydroxyalkyl esters (e.g. 2-hydroxyethyl or 2,3-dihydroxypropyl esters).

In addition to the above ester derivatives the present invention includes within its scope compounds of general formula (I) in the form of other physiologically acceptable equivalents, i.e. physiologically acceptable compounds which, like the metabolically labile esters, are converted in vivo into the parent compounds of general

formula (I).

According to a second aspect of the present invention we provide a compound of general formula (I) or a physiologically acceptable salt, solvate or metabolically labile ester thereof for use in therapy.

In particular, the compounds of the present invention may be used in the treatment or prophylaxis of hypertension (for example, essential, malignant or resistant, caused by oral contraceptives, coarctation of the aorta or renal vascular disease) and pulmonary hypertension.

The compounds of the present invention may also be used in the treatment or prophylaxis of congestive heart failure, acute or chronic heart failure, aortic or cardiac insufficiency, post- myocardial infarction, renal insufficiency and renal failure (for example, as a result of diabetic nephropathy, glomerular nephritis, scleroderma or renal crisis), proteinuria, Bartter's syndrome, secondary hyperaldosteronism, Reynaud's syndrome, cerebrovascular insufficiency, peripheral vascular disease, diabetic retinopathy, atherogenesis and for the improvement of vascular compliance.

They are also potentially useful for the treatment of cognitive disorders such as dementia (e.g. Alzheimer's disease) and other CNS disorders, such as anxiety disorders, schizophrenia, depression and alcohol or drug (e.g. cocaine) dependency.

According to a further aspect of the present invention we provide a compound of general formula (I) or a physiologically acceptable salt, solvate or metabolically labile ester thereof for use in the treatment of the aforementioned diseases, especially hypertension.

According to another aspect of the present invention we provide a compound of general formula (I) or a physiologically acceptable salt, solvate or metabolically labile ester thereof for the manufacture of a therapeutic agent for the treatment of the aforementioned diseases, especially hypertension.

According to a further aspect of the present invention we provide a method of treating the aforementioned diseases, especially hypertension, which method comprises administering an effective amount to a patient in need of such treatment of a compound of general formula (I) or a physiologically acceptable salt, solvate or metabolically labile ester thereof.

It will be appreciated that the compounds of general formula (I) or a physiologically acceptable salt, solvate, or metabolically labile ester thereof may advantageously be used in conjunction with one or more other therapeutic agents, such as for example diuretics and/or different antihypertensive agents such as B-blockers, calcium channel blockers or ACE inhibitors. It is to be understood that such combination therapy constitutes a further aspect of the present invention.

It will be further appreciated that reference herein to treatment extends to prophylaxis as well as to the treatment and relief of established symptoms.

While it is possible that a compound of general formula (I) may be administered as the raw chemical it is preferable to present the active ingredient as a pharmaceutical formulation.

The compounds of general formula (I) and their physiologically acceptable salts, solvates and metabolically labile esters may be formulated for administration in any convenient way, and the invention also includes within its scope pharmaceutical compositions comprising at least one compound of general formula (I) or a physiologically acceptable salt, solvate or metabolically labile ester thereof adapted for use in human or veterinary medicine. Such compositions may be presented for use in conventional manner in admixture with one or more physiologically acceptable carriers or excipients. The carrier(s) must be 'acceptable' in the sense of being compatible with the other ingredients of the formulation and not deleterious to the recipient thereof.

Thus, the compounds according to the invention may be formulated for oral, buccal, parenteral or rectal administration or in a form suitable for administration by inhalation or insufflation. Oral administration is preferred.

Tablets and capsules for oral administration may contain conventional excipients such as binding agents, for example mucilage of starch or polyvinylpyrrolidone; fillers, for example, lactose, microcrystalline cellulose or maize-starch; lubricants, for example, magnesium stearate or stearic acid; disintegrants, for example, potato starch, croscarmellose sodium or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup or carboxymethyl cellulose; emulsifying agents, for example, sorbitan mono-oleate; non-aqueous vehicles (which may include edible oils), for example, propylene glycol or ethyl alcohol; and preservatives, for example, methyl or propyl p-hydroxybenzoates or sorbic acid. The compounds or their salts or esters may also be formulated as suppositories, e.g. containing conventional suppository bases such as cocoa butter or other glycerides. For buccal administration the composition may take the form of tablets or lozenges formulated in conventional manner.

It will be appreciated that both tablets and capsules may be manufactured in the form of sustained release formulations, such that they provide a controlled continuous release of the compounds according to the invention over a period of hours.

The compounds of general formula (I) and their physiologically acceptable salts, solvates and metabolically labile esters may be formulated for parenteral administration by bolus injection or continuous infusion and may be presented in unit dose form in ampoules, or in multi-dose containers with an added preservative. The compositions may take such forms as suspensions, solutions, or emulsions in oily or aqueous vehicles, and may contain formulatory agents such as suspending, stabilising and/or dispersing agents. Alternatively the active ingredient may be in powder form for constitution with a suitable vehicle, e.g. sterile, pyrogen-free water, before use.

For administration by inhalation the compounds according to the invention are conveniently delivered in the form of an aerosol spray presentation from pressurised packs or a nebuliser, with the use of a suitable propellant, e.g. dichlorodifluoromethane, trichlorofluoro methane, dichlorotetrafluoroethane or other suitable gas. In the case of a pressurised aerosol the dosage unit may be determined by providing a valve to deliver a metered amount.

Alternatively, for administration by inhalation or insufflation, the compounds according to the invention may take the form of a dry powder composition, for example a powder mix of the compound and a suitable powder base such as lactose or starch. The powder composition may be presented in unit dosage form in, for example, capsules or cartridges of e.g. gelatin, or blister packs from which the powder may be administered with the aid of an inhaler or insufflator.

The pharmaceutical formulations according to the invention may also contain other active ingredients such as antimicrobial agents, or preservatives.

It will be appreciated that the amount of a compound of general formula (I) required for use in treatment will vary not only with the particular compound selected but also with the route of administration, the nature of the condition being treated and the age and condition of the patient and will ultimately be at the discretion of the attendant physician or veterinarian. In general, however, when the compositions comprise dosage units, each unit will preferably contain 5mg to 500mg, advantageously where the compounds are to be administered orally 25mg to 400mg of the active compound. The daily dosage as employed for adult human treatment will preferably range from 5mg to 3g, most preferably from 25mg to 1g which may be administered in 1 to 4 daily doses.

The compounds of the invention may be prepared by a number of processes as described below wherein the various groups are as defined for general formula (I) unless otherwise specified.

Thus, according to a further aspect of the present invention we provide a process (A) for preparing the compounds of general formula (I) wherein Het represents group (a), (c) or (e) which comprises treating a compound of general formula (II)

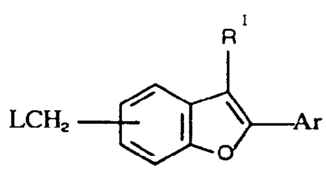

(II)

(wherein L is a leaving group, for example a halogen atom such as chlorine, bromine or iodine, or an alkyl- or aryl sulphonyloxy group such as methanesulphonyloxy, or p-toluenesulphonyloxy and $R^1$ and Ar are as defined in general formula (I)) with a heterocycle of formula (IIIa), (IIIb) or (IIIc)

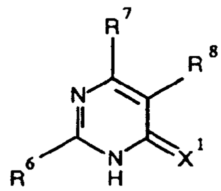

(IIIa)

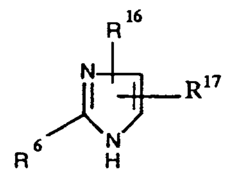

(IIIb)

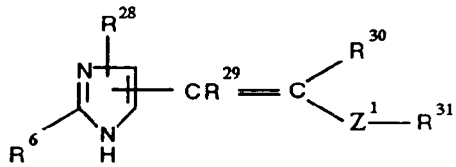

(IIIc)

(wherein $R^6$, $R^7$, $R^8$, $R^{16}$, $R^{17}$, $R^{28}$, $R^{29}$, $R^{30}$, $R^{31}$, $X^1$ and $Z^1$ are as defined in general formula (I)) followed by the removal of any protecting groups where present, as described hereinafter.

The reaction is preferably effected under basic conditions, for example, in the presence of sodium hydride, potassium carbonate or sodium methoxide. The reaction is conveniently effected in a solvent such as acetonitrile or an alcohol e.g. methanol, an ether e.g. tetrahydrofuran or dioxan, a ketone e.g. butanone or methyl isobutyl ketone, or a substituted amide e.g. dimethylformamide, at a temperature between 0°C and the reflux temperature of the solvent.

In another general process (B) a compound of general formula (I) may be obtained by deprotection of a protected intermediate of general formula (IV)

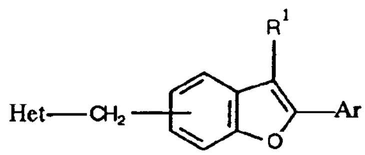

(IV)

(wherein $R^1$, Ar and Het are as defined in general formula (I) except that at least one reactive group is blocked by a protecting group).

The protecting groups may be any conventional protecting groups, for example as described in "Protective Groups in Organic Synthesis" by Theodora Greene (John Wiley and Sons Inc., 1981). Examples of carboxyl protecting groups include $C_{1-6}$alkyl such as methyl or t-butyl, or $C_{7-10}$aralkyl such as benzyl.

When $R^3$ is a tetrazole group, this may be protected with, for example, the trityl group -C(phenyl)$_3$, or a p-nitrobenzyl or l-ethoxyethyl group.

Deprotection to yield the compound of general formula (I) may be effected using conventional techniques. Thus, for example, aralkyl groups may be cleaved by hydrogenolysis in a suitable organic solvent such as an alcohol, e.g. ethanol, in the presence of a noble metal catalyst such as palladium or an oxide thereof on a support such as charcoal, and conveniently at room temperature and pressure. Carboxyl protecting groups such as alkyl groups may be cleaved by hydrolysis using a base such as an alkali metal hydroxide (e.g. sodium hydroxide or potassium hydroxide) in a suitable solvent (e.g. an aqueous alcohol such as methanol or ethanol) at any suitable temperature up to reflux. Deprotection of the tetrazole group when protected with a trityl group may be effected by acid hydrolysis using trifluoroacetic acid or a mineral acid such as hydrochloric acid in a suitable solvent such as ethanol conveniently at room temperature. Alternatively, when possible, deprotection of the tetrazolyl group can be effected by catalytic hydrogenation as previously described.

In another general process (C) a compound of general formula (I) in which the substituent $R^3$ in the group Ar represents a C-linked tetrazolyl group may be prepared from a compound of general formula (Ia)

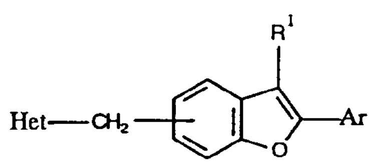

(Ia)

(wherein $R^1$, Ar and Het are as defined in general formula (I) except that in the group Ar, $R^3$ represents a nitrile group) by reaction with a suitable azide such as sodium azide, ammonium azide (preferably prepared in situ from sodium azide and ammonium chloride), trialkyl-(e.g triethyl) ammonium azide (preferably prepared in situ from sodium azide and a trialkylamine (e.g. triethylamine)), a trialkylsilyazide (e.g. trimethylsilylazide) or an alkyl tin azide e.g. tributyl tin azide. The reaction is conveniently effected in a solvent such as xylene, an ether, for example, dimethoxyethane or tetrahydrofuran, or a substituted amide, for example, dimethylformamide at an elevated temperature, such as the reflux temperature of the solvent, for between 1 and 10 days. Where the

azide is tributyl tin azide the reaction may conveniently be effected in the absence of a solvent at a temperature between room temperature and 180°C. Such a reaction leaves the tetrazolyl group protected with a tributyl tin group, which can readily be removed using aqueous base or acid. Where aqueous base is used to effect this deprotection, the compound may be treated with an aqueous acid to liberate the free tetrazole.

Compounds of general formula (Ia) may be prepared by processes analogous to those described herein commencing from a compound of formula (XIa)) and a corresponding benzofuran intermediate.

The intermediate compounds of general formula (Ia) and their acid addition salts are novel compounds and form a further aspect of the present invention.

In another general process (D) a compound of general formula (I) in which the substituent $R^3$ in the group Ar represents $-NHSO_2CF_3$, may also be prepared from a compound of general formula (Ib)

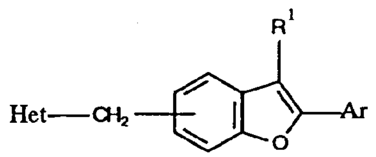

(Ib)

(wherein $R^1$, Ar and Het are as defined in general formula (I) except that in the group Ar, $R^3$ represents an amino group) by reaction with trifluoromethanesulphonic anhydride or trifluoromethylsulphonyl chloride, in a suitable solvent such as a halogenated hydrocarbon, for example, chloroform or dichloromethane in the presence of a base e.g. triethylamine.

Compounds of general formula (Ib) may be prepared by processes analogous to those described herein commencing from a compound of formula (XIb) and a corresponding benzofuran intermediate.

Alternatively, compounds of general formula (Ib) may be prepared by a Curtius rearrangement of a compound of formula (I) wherein $R^3$ in the group Ar is $-CO_2H$ (provided that this is the only carboxyl group in the molecule) using, for example, diphenylphosphorylazide in the presence of a base such as triethylamine and in a solvent such as an alcohol (e.g. tert- butanol) to form a carbamate followed by deprotection of the amine in a conventional manner, for example by acid hydrolysis using hydrochloric acid in a solvent such as ethanol.

The intermediate compounds of general formula (Ib) and their acid addition salts are novel compounds and form a further aspect of the present invention.

In another general process (E) a compound of general formula (I) wherein Het represents the group (b) may be obtained by treating a compound of formula (II) with a heterocycle of formula (VIa)

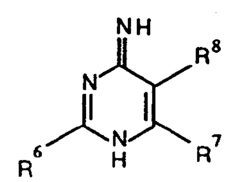

(VIa)

wherein $R^6$, $R^7$ and $R^8$ are as defined in general formula (I) followed by hydrolysis of the amine using aqueous hydroxide e.g. sodium hydroxide to give the pyrimidinone. The reaction is effected in the presence of a base such as sodium hydride or potassium carbonate in a suitable solvent such as acetonitrile, or a substituted amide e.g. dimethylformamide at a temperature between 0° and the reflux temperature of the solvent.

Alternatively, where $X^1$ is an oxygen atom, $R^6$ as a $C_{1-6}$alkoxy group may be prepared by transetherification using, for example $(C_{1-6}alkoxy)_3CH$ in a suitable solvent such as a substituted amide e,g, dimethylformamide.

In another general process (F) a compound of general formula (I) in which Het represents the group (a) may be obtained by treating a compound of formula (V)

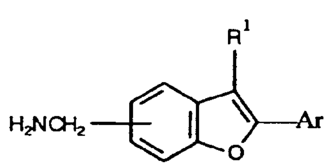

(V)

wherein $R^1$ and Ar are as defined in general formula (I)) with a compound of formula (VIb)

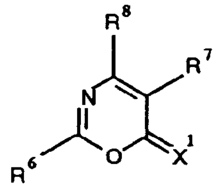

(VIb)

wherein $R^6$, $R^7$, $R^8$ and $X^1$ are as defined in general formula (I)), optionally in the presence of a dehydrating agent such as p-toluenesulphonic acid, in a solvent such as an aromatic hydrocarbon e,g, benzene or toluene, or a halogenated hydrocarbon e.g. dichloromethane, at a temperature between room temperature and the reflux temperature of the solvent.

In another general process (G) a compound of general formula (I) wherein Het represents structure (d) may be obtained by treating a compound of formula (Ic)

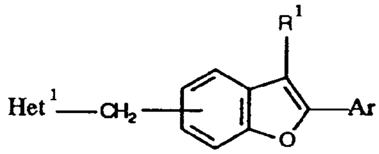

(Ic)

(wherein $R^1$ and Ar are as defined in general formula (I) and $Het^1$ represents the group

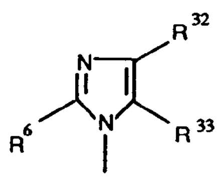

wherein $R^6$ is as defined in general formula (I) and $R^{32}$ and $R^{33}$ represent, respectively, $-CONR^{23}$ and $-NHR^{23}$; $-NHR^{23}$ and $-CONR^{23}$; $-CO_2R^{12}$ and $-NHR^{23}$; $-NHR^{23}$ and $-CO_2R^{12}$; $-CO_2R^{12}$ and halogen; halogen and $-CO_2R^{12}$; -SH and $-CO_2R^{12}$; $-CO_2R^{12}$ and -SH) with one of the compounds of general formula (VIIa) to (VIIe)

(a) 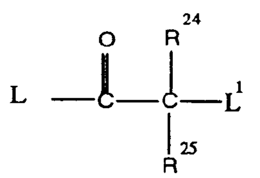 , (b) 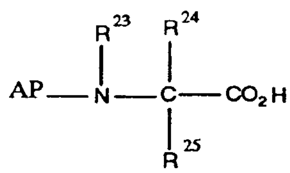 ,

(c) 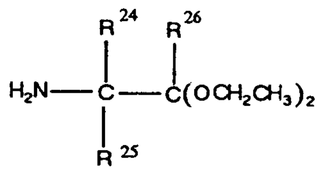 , (d) 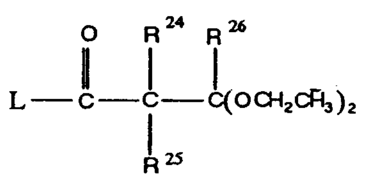 ,

(e) 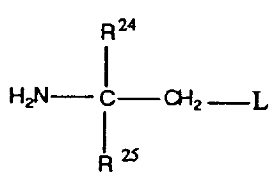

(VII)

(wherein $R^{23}$, $R^{24}$, $R^{25}$ and $R^{26}$ are as defined in general formula (I), L and $L^1$ are leaving groups as defined in

general process (A) and AP is a suitable amino-protecting group) followed by the removal of any protecting group where present, as described above.

It will be appreciated by a person skilled in the art, that the choice of substituents $R^{32}$ and $R^{33}$ in the compound of formula (Ic) combined with a given structure of formula (VII) will enable the synthesis of the group A in structure (d) of Het as defined in general formula (I).

Thus, for example, compounds of general formula (I) wherein Het represents

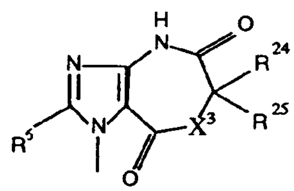

and $X^3$ represents $-NR(R^{23})-$ or oxygen may be prepared by the reaction of a compound of formula (Ic) (wherein $R^{32}$ represents $-NH_2$ and $R^{33}$ represents $-CONHR^{23}$ or $-CO_2H$ where $X^3$ = oxygen) with a compound of formula (VIIa) (wherein L and $L^1$ are leaving groups, such as halogen atoms, for example, chlorine atoms) in the presence of an organic base, such as a tertiary amine, for example, triethylamine.

Similarly, A may be prepared in the reverse orientation simply by interchanging the groups represented by $R^{32}$ and $R^{33}$.

Compounds of general formula (I) wherein Het is shown as above (wherein $X^3$ represents $-N(R^{23})-$) may also be prepared by reacting a compound of formula (VIIb) (wherein AP is an amino-protecting group such as a carbobenzyloxy, t-butoxycarbonyl or fluorenylethylmethoxyloxycarbonyl group) in the presence of an acyl halide or a caiboxyl activating agent such as dicyclohexylcarbodiimide. Final formation of the seven-membered ring is conveniently effected by the action of heating the reaction mixture in an alcoholic solvent.

Similarly, A may be prepared in the reverse orientation by interchanging the groups represented by $R^{32}$ and $R^{33}$.

Compounds of general formula (I) wherein Het represents

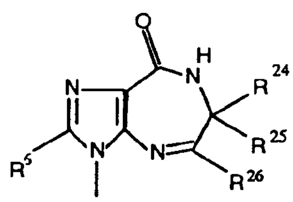

may be prepared by reacting a compound of formula (Ic) (wherien $R^{32}$ represents $-CO_2CH_3$ and $R^{33}$ represents $-NH_2$) with a compound of formula (VIIc) in the presence of a base and a carboxyl activating agent. Final formation of the seven-membered ring is conveniently effected by heating the reaction mixture in the presence of molecular seives and acetic acid in an inert solvent such as an ether, for example, dioxan. Alternatively a dehydrating agent such as polyphosphoric acid may be used.

Similarly, A may be prepared in the reverse orientation by interchanging the group represented by $R^{32}$ and $R^{33}$.

Compounds of general formula (I) wherein Het represents

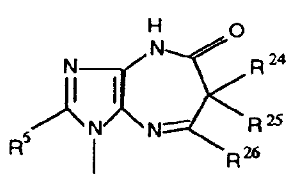

may be prepared by reacting a compound of formula (Ic) (wherein $R^{32}$ and $R^{33}$ both represent $-NH_2$) with a compound of formula (VIId) (wherein L is a leaving group such as a halogen atom, for example, a chlorine atom) using standard procedure of amide formation followed by dehydration to effect ring closure. This method will produce a mix of compounds of general formula (I) wherein A is in the clockwise and the anti-clockwise orientation.

Compounds of general formula (I) wherein Het represents

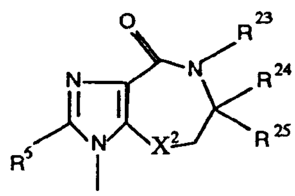

in which $X^2$ is sulphur, may be prepared by the reaction of a compound of formula (Ic) (wherein $R^{32}$ represents $-CO_2R^{12}$ and $R^{33}$ represents -SH) with a compound of formula (VIIe) (wherien L is a leaving group such as a halogen atom, for example, a chlorine atom) in the presence of a carboxyl activating agent, followed by ring closuie by heating the reaction mixture in an alcoholic solvent.

Similarly A may be prepared in the reverse orientation by interchanging the groups represented by $R^{32}$ and $R^{33}$.

In another general process (H) a compound of general formula (I) in which Het represents structure (e) may be obtained by treating a compound of formula (Id)

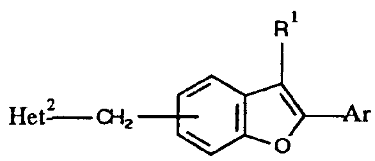

(Id)

(wherein $R^1$ and Ar are as defined in general formula (I) and $Het^2$ represents the group

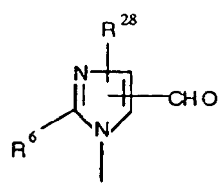

wherein $R^6$ and $R^{28}$ are as defined in general formula (I)) with a compound of general formula (VIIIa)

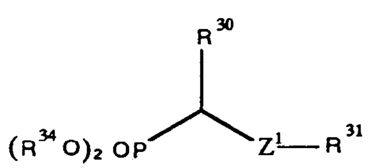

(VIIIa)

(wherein $R^{30}$, $R^{31}$ and $Z^1$ are as defined in general formula (I) and $R^{34}$ is a $C_{1-4}$alkyl group) followed by the removal of any protecting groups where present, as described above.

The reaction is effected in the presence of a base such as sodium hydride or sodium methoxide, conveniently in a solvent such as an ether e.g. tetrahydrofuran, or a substituted amide e.g. dimethylformamide, at a temperature between room temperature and 100°C.

Alternatively a compound of general formula (I) may be obtained by treating a compound of formula (Id) as defined above with a compound of formula (VIIIb)

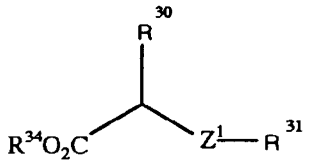

(VIIIb)

(wherein $R^{30}$, $R^{31}$, $R^{34}$ and $Z^1$ are as defined in formula (VIIIa)) followed by the removal of any protecting groups where present as described above.

The reaction is effected in a solvent such as an aromatic hydrocarbon e.g. toluene, or an ether e.g. tetrahydrofuran in the presence of a base such as potassium hydroxide or lithium diisopropylamide.

In the processes (A), (B), (C), (D), (E), (F), (G) and (H) described above, the compounds of general formula (I) may be obtained in the form of a salt, conveniently in the form of a physiologically acceptable salt. Where desired, such salts may be converted into the corresponding free acids or free bases using conventional methods.

Physiologically acceptable salts of the compounds of general formula (I) may be prepared by reacting a compound of general formula (I) with an appropriate acid or base in the presence of a suitable solvent such as acetonitrile, acetone, chloroform, ethyl acetate or an alcohol, e.g. methanol, ethanol or isopropanol.

Physiologically acceptable salts may also be prepared from other salts, including other physiologically acceptable salts, of the compounds of general formula (I), using conventional methods.

The intermediate compounds of general formula (II) may be prepared from a compound of formula (IX)

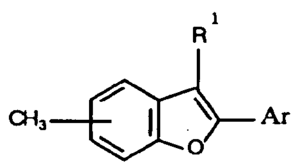

(IX)

using any suitable reagent well known in the art for converting the methyl on the 6-membered ring into the group $-CH_2L$ (wherein L is as defined above). Thus, for example, when L is a halogen atom, a compound of formula (IX) can be converted into a compound of general formula (II) using N-chloro amides, tert-butyl hypochlorite or N-bromosuccinimide. Halogenation of the side chain may be catalysed by light, thus the reaction can be illuminated with a suitable artificial light source, and preferably in the presence of a free radical initiator such as azobisisobutyronitrile (AIBN) or benzoyl peroxide.

Compounds of formula (IX) wherein $R^1$ is a halogen atom, for example, a bromine atom, may be prepared by halogenation of a compound of formula (IX) wherein $R^1$ represents a hydrogen atom, using for example, bromine, in a suitable solvent such as a halogenated hydrocarbon, e.g. carbon tetrachloride.

Compounds of formula (IX) may be prepared by reaction of a compound of formula (X)

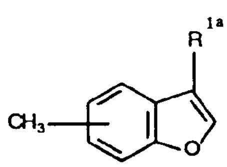

(X)

(wherein $R^{1a}$ represents a hydrogen atom or a group selected from $C_{1-6}$alkyl or $C_{2-6}$alkenyl) with a compound of formula (XI)

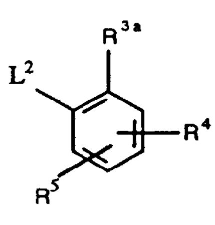

(XI)

(wherein $L^2$ represents a bromine or iodine atom or the group $-OSO_2CF_3$, $R^4$ and $R^5$ are as defined in general formula (I) and $R^{3a}$ is as defined for $R^3$ in general formula (I) or is a protected derivative thereof).

The compound of formula (X) is first treated with an alkyl lithium compound such as n-butyl lithium at a reduced temperature, for example, between -100°C and 0°C in a solvent such as an ether (e.g. tetrahydrofuran). The mixture is then treated with a tri- alkylborate such as triisopropylborate and the temperature conveniently brought up to room temperature. Subsequently, water may be added and the mixture treated with a mineral acid such as sulphuric acid thus producing a compound of formula (Xa)

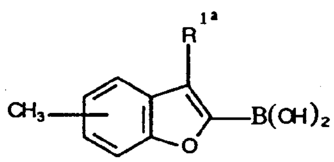

(Xa)

The intermediate compound of formula (Xa) is then reacted with a compound of formula (XI) in the presence of a palladium (0) compound such as tetrakis(triphenylphosphine) palladium (0) in a solvent such as an ether (e.g. dimethoxyethane), and in the presence of a base such as sodium carbonate or thallium hydroxide. The reaction is conveniently effected at an elevated temperature, such as the reflux temperature of the solvent.

Compounds of formula (IX) in which the substituent $R^3$ in the group Ar represents a C-linked tetrazolyl group may be prepared from a precursor of a compound of formula (IX) wherein the substituent $R^3$ represents a nitrile group using the reagents and conditions described in process (C).

Similarly, intermediates of formula (XI) wherein $R^{3a}$ represents a C-linked tetrazolyl group may be prepared from a compound of formula (XIa)

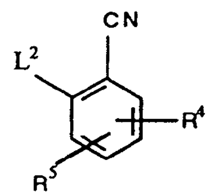

(XIa)

(followed where necessary by protection of any reactive groups), using methods well-known in the art such as those described in process (C).

Compounds of formula (IX) in which the substituent $R^3$ in the group Ar is $-NHSO_2CF_3$ may be prepared from a precursor of a compound of formula (IX) wherein the substituent $R^3$ is an amine group using the reagents and conditions described in process (D).

Similarly, intermediates of formula (XI) wherein $R^{3a}$ represents $-NHSO_2CF_3$ may be prepared from a compound of formula (XIb),

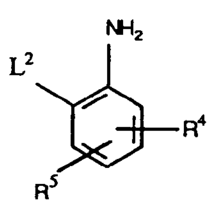

(XIb)

(followed where necessary by the protection of any reactive group) using methods well known in the art such as those described in process (D).

Compounds of formula (IX) may also be prepared by an intramolecular cyclisation reaction of a compound of formula (XII)

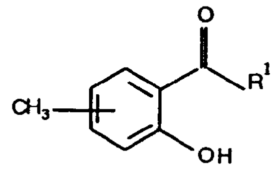

(XII)

(wherein $R^1$ is as previously defined with the exception of CHO, $COR^2$, where $R^2$ is $C_{1-6}$alkoxy or $-NR^{14}R^{15}$, and halogen) with a suitably substituted benzene of formula (XIII)

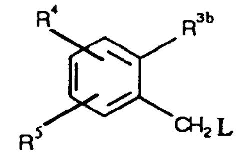

(XIII)

(wherein L is as previously defined and $R^{3b}$ is as defined for $R^{3a}$ in formula (XI) with the exception of $-CO_2H$ and $-NHSO_2CF_3$ or is a nitrile group suitable for subsequent conversion into a tetrazolyl group or is a nitro group suitable for conversion into $-NHSO_2CF_3$), in the presence of a base such as sodium hydride or potassium carbonate. The cyclisation is a two step reaction which requires one equivalent of base per step. It will be appreciated however that the reaction can be effected in the presence of two equivalents of base to avoid the need to isolate the intermediate. The reaction is conveniently effected in a solvent such as an ether e.g tetrahydrofuran, an alcohol e.g ethanol or a substituted amide e.g dimethylformide, at a temperature between room temperature and the reflux temperature of the solvent.

Intermediates of formula (V) may be prepared from a compound of formula (II) using methods well known in the art, for example, using the Gabriel synthesis where a halide of formula (II) is reacted with potassium phthalimide and the product hydrolysed to give the primary amine. Alternatively, a halide of formula (II) may be treated with an azide, for example, sodium azide followed by reduction of the azide thus formed to give the primary amine.

The intermediates of formula (IIIc) may be prepared from an imidazole of formula (XIV)

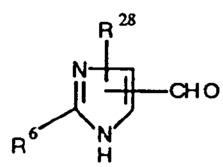

(XIV)

(wherein $R^6$ and $R^{28}$ are as defined in general formula (I)) by reaction with a compound of general formula (VIIIa) or (VIIIb) using the reagents and conditions described in process (H).

Compounds of general formula (Id) may be made by processes analogues to those described herein as (A) to (D) except that the 5- position substituent on the imidazole Het is an aldehyde group.

It will be appreciated that compounds of formula (IX) in which $R^1$ represents a hydrogen or halogen atom may also be converted into compounds of formula (IX) in which $R^1$ represents the group methyl (via hydrogenolysis of the Mannich base), -CHO or $-COR^2$ (wherein $R^2$ is as defined in general formula (I)) using techniques well known in the art, such as those described in "Heterocyclic Chemistry" by J.A. Joule and G.F. Smith, Van Nostrand Reinhold Company, London (1972), "Heterocyclic Chemistry" by A. Albert, 2nd Edition, The Athlone Press, London (1968), "Heterocyclic Compounds", Vol. 29 by A. Mustafa, John Wiley and Sons Inc., New York (1974), "Heterocyclic Compounds", Vol. 2 by R.C. Elderfield, John Wiley and Sons Inc., New York (1951) and "Advances inHeterocyclic Chemistry", Vol. 29 by A.R. Ratritsky and A.J. Boulton, Academic Press, New York (1981).

Intermediates of formula (IIIa) (VIa) and (VIb) are either known compounds or may be prepared by methods well known in the art, see for instance, "Comprehensive Organic Chemistry", Vol.4 by D Barton and W.D. Ollis, Pergamon Press, Oxford (1979).

Intermediates of formula (IIIb) may be prepared as described in European Patent Specification no. 0401030A and the methods described therein are incorporated by reference herein.

Intermediates of formula (VIIIa) may be prepared as described in European Patent Specification No.

0403159A and 0425211A or by methods analogous to those described therein.

Intermediates of formulae (VIIa)-(VIIe), (VIIIb), (X), (XI), (XIa), (XIb), (XII), (XIII) and (XIV) are either known compounds or may be prepared by methods analogous to those used for the preparation of the known compounds.

The following examples illustrate the invention. Temperatures are in °C. "Dried" refers to drying using magnesium sulphate. Thin layer chromatography (T.l.c.) was carried out over silica and column chromatography was carried out on silica (Merck 9385 unless otherwise stated), using one of the following solvent systems : A - ether:hexane, B - ether: petroleum ether, C - dichloromethane:ethanol, D-dichloromethane:ether, or E-ether:acetic acid, F - dichloromethane:ethanol:ammonia. The following abbreviations are used : THF - tetrahydrofuran; DME - dimethoxyethane; AIBN - azobisisobutyronitrile; DMF - dimethylformamide; TMEDA - tetramethylethylenediamine; NBS - N-bromosuccinimide; DMAP- 4-dimethylaminopyridine; DEAD - diethyl azodicarboxylate.

Intermediate 1

5-Methylbenzofuran-2-boronic acid

n-Butyl lithium (1.7M, 35.16ml) was added dropwise to a stirred solution of TMEDA (9.58ml) and 5-methylbenzofuran (8.22g) in ether (250ml) maintaining the temperature below - 60°C throughout. The solution was warmed to about -10°C over 45 minutes and stirred at this temperature for 30 minutes. A precipitate formed on warming. The suspension was cooled and triisopropylborate (43ml) was added, maintaining the temperature below -60°C. The solution was warmed gradually to room temperature before quenching with 2N HCl (70ml). The mixture was extracted with ether (3x50ml) and the combined organic extracts washed with 2N HCl (4x30ml), water (2x30ml) and dried before evaporation to give the title compound as an orange solid (12.75g). T.l.c. System A (1:1), Rf 0.3.

Intermediate 2

2-(5-Methyl-2-benzofuranyl)benzonitrile

Intermediate 1 (20g) was added to a stirred solution of 2-bromobenzonitrile (10.34g) and tetrakistriphenylphosphine palladium (0) (1.5g) in DME (200ml) and 8% $NaHCO_3$ (50ml) at reflux under nitrogen. Further catalyst (1.5g) was added and the reaction continued overnight. The reaction was cooled to room temperature and diluted with ether (200ml). The organic layer was separated, washed with water (3x100ml) and dried. Filtration and evaporation gave a white solid which was purified by chromatography eluting with System A (1:9) to give the title compound (10.58g) as a white solid. T.l.c. System A (1:9), Rf 0.45.
Intermediate 2 was also prepared by the alternative two-step reaction:

a) 2-Hydroxy-5-methylbenzaldehyde

p-Cresol (100g) in dry THF(100ml) was added dropwise to a mechanically stirred, freshly prepared solution of ethyl magnesium bromide [magnesium (25.0g) and bromoethane (75ml)] in THF (500ml) under nitrogen at a rate which maintained a slow reflux (about 30mins). After a further 30mins toluene (1.21) was added, followed by 1,3-dimethyl-3,4,5,6-tetrahydro-2(1H)-pyrimidone (125ml), and paraformaldehyde (70g). The mixture was then heated at reflux for 16h. The mixture was concentrated by distillation and aqueous hydrochloric acid (2M, 600ml) then added. Water (600ml) was added and the mixture filtered through "hyflo". The organic phase was separated, dried, filtered and concentrated in vacuo to give a brown oil. The oil was steam distilled and the product extracted from the distillate with ether (1 litre). The organic extract was dried, filtered and concentrated in vacuo to give a pale yellow slurry which was cooled to -10°C, triturated with ether (precooled to -78°C, 100ml), filtered off rapidly and washed with ether (precooled to -78°C) to give the title compound as colourless needles, (131.4g).
T.l.c. System A (1:5) Rf 0.5.

b) 2-(5-Methyl-2-benzofuranyl)benzonitrile

A solution of the product of step (a) (130g) in dry DMF (400ml) was added dropwise to a solution of sodium methoxide (56.2g) in ethanol (400ml) mechanically stirred under nitrogen. After a further 20mins, a solution of 2-(bromomethyl)benzonitrile (182.2g) in dry DMF (400ml) was added dropwise. The mixture was then heated

to 75°C for 30min. The solution was allowed to cool for 1h. A slurry of sodium methoxide (56.2g) in dry DMF (100ml) was added and the mixture heated at reflux for 1.5h. The mixture was concentrated in vacuo and then poured into iced water. The solid was collected, and then triturated with methanol to give the title compound (Intermediate 2) as a beige solid (149.4g).
T.l.c. System A (1:9) Rf 0.4.

Intermediate 3

5-[2-(5-Methyl-2-benzofuranyl)phenyl]-1H-tetrazole

A suspension of Intermediate 2 (94g) in tri-n-butyl tin azide (268g) was heated at 100-125°C for 1.25h under nitrogen. The resulting solution was then heated at 155-160°C for 2h under nitrogen, then poured into a solution of aqueous sodium hydroxide (0.8N, 3070ml). This solution was extracted with ether. The aqueous phase was acidified to pH1 with 5N hydrochloric acid and the resulting precipitate filtered, washed with water and dried under vacuum. The solid was dissolved in ethyl acetate, washed with brine and dried. The solvent was evaporated to give the title compound as a buff-coloured solid (100.3g).
T.L.C. System A (1:1), Rf 0.2.

Intermediate 4

5-[2-(3-Bromo-5-methyl-2-benzofuranyl)phenyl]-1H-tetrazole

A solution of bromine (58g), in carbon tetrachloride (140ml) was added dropwise over 35min to a mechanically stirred solution of Intermediate 3 (50g) in dry dioxan (2090ml) at room temperature under nitrogen. The resulting solution was stirred at room temperature for 3h, then cyclohexene (63ml) was added. Another preparation of the product was carried out simultaneously on the same scale as described above, and at this stage they were combined. The solvent was evaporated and the residual brown oil (260g) partitioned between ether and aqueous sodium hydroxide. The alkaline solution was acidified to pH1 with hydrochloric acid, then extracted with ethyl acetate. The cotined ethyl acetate extracts were washed with brine, dried and evaporated to give a buff solid (125g) which was triturated under hot toluene, cooled and filtered off to give the title compound as a cream coloured solid (101.8g). T.l.c. ether:petroleum ether:acetic acid (50:50:1), Rf 0.27.

Intermediate 5

5[2-(3-Bromo-5-methyl-2-benzofuranyl)phenyl]-2-(triphenylmethyl)- 2H-tetrazole

Triethylamine (57.4g) was added to a mechanically stirred suspension of Intermediate 4 (101g) in dry dichloromethane (2.9 litres) at room temperature under nitrogen. Triphenylmethyl chloride (79.3g) followed by DMAP (1.0g) were added at room temperature and the mixture stirred for 3h under nitrogen. The reaction mixture was washed with water, then brine and dried. The solvent was filtered and concentrated to a volume of about 1.2 litres then filtered through silica (Merck 9385, 14cm diam. column). Elution with dichloromethane gave a colourless solid (158.4g) which was triturated with ether and filtered to give the title compound as a colourless solid (147.9g).
T.l.c. dichloromethane:hexane (1:1), Rf 0.28

Intermediate 6

5-[2-[3-Bromo-5-(bromomethyl)-2-benzofuranyl]phenyl]-2- (triphenylmethyl)-2H-tetrazole

Intermediate 5 (74g) was dissolved in carbon tetrachloride (2050ml) by heating the suspension to reflux. The resulting colourless solution was allowed to cool to 50°C then NBS (22.1g) was added, followed by benzoyl peroxide (1.1g). The reaction mixture was heated at reflux for 3.25h, under nitrogen, then allowed to cool to room temperature. The reaction mixture was washed with water then brine. Another preparation of the product was carried out simultaneously on the same scale as described above, and at this stage they were combined and dried. The solvent was evaporated to give a colourless solid (168g) which was triturated with ether/methanol (1:1) and filtered to give the title compound as a colourless solid (160.8g).
T.l.c. dichloromethane:hexane (1:1), Rf 0.15.

Intermediate 7

3-[[3-Bromo-2-[2-[2-(triphenylmethyl)-2H-tetrazol-5-yl]phenyl]-5- benzofuranyl]methyl]-2-ethyl-4(3H)-pyrimidinone

60% Sodium hydride (15mg,) was added to a solution of 2-ethyl-4-(3H)-pyrimidinone (300mg,) in anhydrous DMF (25ml) and the mixture stirred under nitrogen until gaseous evolution ceased (ca 20min) and for a further 15min Intermediate 6 (2.00g,) was added and the resulting suspension stirred overnight to give a brown solution. The solution was added to saturated aqueous ammonium chloride (100ml) and extracted with dichloromethane (3x50ml). The combined extracts were washed with water (3X50ml), dried and concentrated in vacuo to give an orange oil. Purification by Flash column chromatography eluting with ether/dichloromethane (2:1) gave the title compound as a white glass (260mg,)
T.l.c. ether:dichloromethane (2:1) Rf = 0.43

Intermediate 8

Ethyl N-cyanopentaneimidate

Cyanamide (1.275g) was added to a solution of ethyl pentaneimidate hydrochloride (5g) in ethanol (8ml) and the mixture stirred at room temperature under nitrogen for 18h. Precipitated ammonium chloride was filtered off and the filtrate evaporated in vacuo. The residue was purified by flash column chromatography eluting with ether to give the title compound as a colourless mobile oil (3.37g)
T.l.c ether:petroleum ether (1:1) Rf 0.7.

Intermediate 9

N-[1-(Cyanoimino)pentyl]glycine methyl ester

Methyl glycine hydrochloride (2.93g) and triethylamine (9.85g) was added to a solution of Intermediate 8 (3g) in methanol (6ml) at room temperature. The mixture was stirred for 18h, then filtered. The filtrate was evaporated in vacuo to give a pale yellow gum (4g). The crude material was purified by flash column chromatography eluting with ethyl acetate/hexane (1:1) to give the title compound as a pale yellow oil (3.77g).
T.l.c. ethyl acetate:hexane (1:1) Rf 0.3

Intermediate 10

N-[[3-Bromo-2-[2-[2-(triphenylmethyl)-2H-tetrazol-5-yl]phenyl]-5- benzofuranyl]methyl]-N-[1-(cyanoimino)pentyl]glycine methyl ester

A solution of Intermediate 9 (0.5g) in dry DMF (1ml) was added dropwise to a suspension of sodium hydride (60% dispersion 122mg washed with hexane (2x3ml)) at room temperature under nitrogen. The mixture was stirred for 15 mins then a suspension of Intermediate 6 (2.45g) in dry DMF (8ml) was added in one portion and the mixture stirred overnight at room temperature. The pale yellow suspension was partitioned between ethyl acetate (3x50ml) and brine/water 1:1 (50ml) and the cotined organic extracts washed with brine/water 1:1 (2x50ml) and dried. The solvent was evaporated to give a pale yellow foam (2.9g). The crude material was purified by flash column chromatography eluting with petroleum ether:ethyl acetate to give the title compound as a pale yellow foam (837mg).
T.l.c. hexane:ethyl acetate (1:1) Rf 0.5

Intermediate 11

Methyl 4-amino-1-[[3-bromo-2-[2-[2-(triphenylmethyl)-2H-tetrazol-5-yl]-5-benzofuranyl]methyl]-2-butyl-1H-imidazole-5-carboxylate

A solution of Intermediate 10 (0.4g) in dry DMF (1ml) was added dropwise to a suspension of sodium hydride (60% disperesion in oil; 24mg) in dry DMF (1ml) at room temperature under nitrogen. The mixture was stirred for 4h, then partitioned between brine/water 1:1 (5ml) and ethyl acetate (3x10ml). The cotined organic extracts were washed with brine/water 1:1 (1x20ml) and dried. The solvent was evaporated to give a pale yellow solid

(0.365g). The crude material was purified by flash column chromatography eluting with ethyl acetate:petroleum ether (1:1) to give the title compound as a pale yellow solid (216mg).
T.l.c. ethyl acetate:petroleum ether (1:1) Rf 0.25

Intermediate 12

Methyl 1-[[3-bromo-2-[2-[2-(triphenylmethyl)-2H-tetrazol-5-yl] phenyl]-5-benzofuranyl]methyl]-2-butyl-4-(methylamino)-1H-imidazole -5-carboxylate

A suspension of Intermediate 11 (7.06g) in dry DMF (80ml) was heated to 65° to effect dissolution. The solution was cooled to room temperature, then sodium hydride (463mg) was added portionwise over 5mins. The mixture was stirred for 30mins, then methyl iodide (1.52g, 0.67ml) was added. The solution was stirred for 18h at room temperature under nitrogen. A further portion of methyl iodide (0.33ml) was added and stirring was continued for 4h. The mixture was partitioned between ethyl acetate (3x200ml) and pH 6.8 phosphate buffer (200ml). The combined organic extracts were washed with brine/water (1:1) (3x200ml) and dried. The solvent was evaporated to give a pale yellow foam. The crude material was purified by flash column chromatography eluting with ethyl acetate:petroleum ether (1:1) to give the title compound as a pale yellow solid (2-42g).
T.l.c. ethyl acetate:petroleum ether (1:1) Rf 0.5.

Intermediate 13

Methyl 1-[[3-bromo-2-[2-[2-(triphenylmethyl)-2H-tetrazol-5-yl] phenyl]-5-benzofuranyl]methyl]-2-butyl-4-[[[[(1,1-dimethylethoxy) carbonyl]amino]acetyl]methylamino]-1H-imidazole-5-carboxylate

Dicyclohexylcarbodiimide (1.28g) in dry chloromethane (10ml) was added dropwise over 15 mins to a solution of N-(tert-butoxycarbonyl) glycine (2.77g) in dry dichloromethane (50ml) at 0° under nitrogen. The mixture was stirred at 0° for 0.5h, then a solution of Intermediate 12 (1.0g) in dry dichloromethane (10ml) was added dropwise over 10mins at 0° and the mixture allowed to warm to room temperature overnight. Ethyl acetate (100ml) was added and the mixture filtered. The filtrate was washed with sodium bicarbonate solution (8%; 2x100ml), then brine (1x100ml) and dried. The solvent was evaporated to give a pale yellow gum. The crude material was purified by short-path column chromatography eluting with ethyl acetate:petroleum ether (1:1) to give the title compound as a colourless foam (788mg)
T.l.c. ethyl acetate:petroleum ether (1:1) Rf 0.2

Intermediate 14

Methyl 1-[[3-bromo-2-[2-(1H-tetrazol-5-yl)phenyl]-5-benzofuranyl] methyl]-2-butyl-4-[[[[(1,1-dimethylethoxy)carbonyl]amino]acetyl] methylamino]-1H-imidazole-5-carboxylate

Conc. Hydrochloric acid (2 drops) was added to a solution of Intermediate 13 (788mg) in methanol (10ml) and the mixture stirred for 3h at room temperature. Sodium bicarbonate (8%; 3ml) was added and the methanol evaporated in vacuo. The residue was extracted with ether (3x20ml), then acidified to pH2 with 2N HCl (1ml) and extracted with ethyl acetate (3x20ml). The cotined ethyl acetate extracts were washed with brine (1x20ml) and dried. The solvent was evaporated to give the title compound as a colourless foam (516mg)
T.l.c. dichloromethane:ether:acetic acid (75:25:1) Rf 0.15

Intermediate 15

1-[[3-bromo-2-[2-(1H-tetrazol-5-yl)phenyl]-5-benzofuranyl]methyl]-2-butyl-4-[[[[(1,1-dimethylethoxy)carbonyl]amino]acetyl]methylamino] -1H-imidazole-5-carboxylic acid

Potassium hydroxide (132mg) in water (0.3ml) was added to a solution of Intermediate 14 (710mg) in methanol (20ml) and the mixture stirred at room temperature for 16h. A further portion of potassium hydroxide (132mg) in water (0.3ml) was added and the mixture warmed at 55° for 3h. The solvent was evaporated in vacuo and the residue dissolved in water (10ml), then acidified to pH 1 with 2N HCl and extracted with ethyl acetate (3x25ml). The combined organic extracts were washed with brine (1x20ml) and dried. The solvent was evaporated to give the title compound as a colourless foam (0.7g).
T.l.c. dichloromethane:methanol (5:1) Rf 0.4

Intermediate 16

1-[[3-Bromo-2-[2-[2-(triphenylmethyl)-2H-tetrazol-5-yl]phenyl]-5-benzofuranyl]methyl]-2-butyl-1H-imidazole-5-carboxaldehyde

A mixture of 2-butyl-1H-imidazole-5-carboxaldehyde (3.0g), Intermediate 6 (13.5g), potassium carbonate (3.3g) and DMF (200ml) was stirred at 85°C for 3 hours. When the mixture had cooled water (150ml) was added. The mixture was extracted with ethyl acetate (3x120ml) and the organics were combined, washed with brine (3x120ml), dried and concentrated in vacuo. The residue was purified by flash column chromatography, eluting with dichloromethane/hexane/ether, (3:3:1) to afford title compound (4.1g) as a white foam.
T.l.c. dichlomethane:ether:hexane (1:1:1) Rf = 0.20

Intermediate 17

(E)-Methyl α-[[1-[[3-bromo-2-[2-[2-(triphenylmethyl)-2H-tetrazol-5- yl]phenyl]-5-benzofuranyl]methyl]-2-butyl-1H-imidazol-5- yl]methylene]-2-thiophenepropanoate

A suspension of sodium hydride (69mg) and trimethyl 3-(2-thienyl)-2-phosphonopropionate (0.40g) in dry THF (6ml) was heated to 60° and stirred for 45 minutes. A solution of Intermediate 16 (0.54g) in THF (10ml) was added and stirring under nitrogen continued for 3 hours. A further portion of sodium hydride (0.54g) and the propionate (3.15g) in dry THF (20ml) at 60° was added and stirring continued for 1½ hours at 65°. After cooling, water (80ml) was added and the suspension extracted with ethyl acetate (3x80ml). The organics were combined, dried and concentrated in vacuo. The residue was purified by flash column chromatography, eluting with ether/hexane (2:1) to afford the title compound (175mg) as a foam.
T.l.c. ether:methanol (96:4) Rf = 0.40

Intermediate 18

(E)-α-[[1-[[3-Bromo-2-[2-[2-(triphenylmethyl)-2H-tetrazol-5-yl]phenyl]-5-benzofuranyl]methyl]-2-butyl-1H-imidazol-5- yl]methylene]-2-thiophenepropanoic acid

A solution of Intermediate 17 (590mg), aqueous sodium hydroxide (2N, 4ml) and ethanol (20ml) was stirred at room temperature for 24h. The solvent was removed in vacuo and after adding water (45ml) the mixture was acidified to pH5 (2N HCl). The mixture was extracted with ethyl acetate (3x50ml) and the cotined organic extracts were dried and concentrated in vacuo to afford the title compound, (420mg).
T.l.c. System F 96:4:0.5 Rf=0.40

Intermediate 19

(E)-1-[3-[1-[[3-Bromo-2-[2-[2-(triphenylmethyl)-2H-tetrazol-5- yl]phenyl]-5-benzofuranyl]methyl]-2-butyl-1H-imidazol-5-yl]-1-oxo-2-(2-thienylmethyl)-2-propenyl]-1H-imidazole

A solution of Intermediate 18 (420mg), 1,1′-carbonyl-diimidazole (440mg) and dry THF (20ml) was stirred under nitrogen for 48h. The solvent was removed in vacuo and the residue purified by flash column chromatography (50:1) to afford the title compound (160mg).
T.l.c. System F (96:4:0.5) Rf=0.43

Intermediate 20

(E)-α-[[1-[[3-Bromo-2-[2-[2-(triphenylmethyl)-2H-tetrazol-5-yl]phenyl]-5-benzofuranyl]methyl]-2-butyl-1H-imidazol-5-yl]methylene]-2-thiophenepropanamide

A mixture of Intermediate 19, (160mg), ammonia (880; 4ml) and ethanol (5ml) was stirred at room temperature for 16h. The solution was concentrated in vacuo and the residue partitioned between ethyl acetate (20ml) and water/brine 1:1 (3x15ml). The organic phases were dried and concentrated in vacuo and the material purified by flash column chromatography eluting with System F (100:2:0.5) to afford the title compound (75mg).
T.l.c. System F (96:4:0.5) Rf=0.38

Intermediate 21

1,1-Dimethylethyl [2-[3-bromo-5-[(2-ethyl-1,6-dihydro-4,5-dimethyl-6-oxo-1-pyrimidinyl)methyl]-2-benzofuranyl]phenyl]carbamate

A mixture of 2-ethyl-5,6-dimethyl-4(3H)-pyrimidone (1.5g) and sodium hydride (60% dispersion in oil 0.4g) in dry DMF (25ml) was stirred at ambient temperature for 1h. 1,1-Dimethyl[2-[3-bromo-5-(bromomethyl)-2-benzofuranyl]phenyl]carbamate (described in European Patent Specification No. 0434249A, published 26th June 1991) (4.2g) was added and stirring was continued at ambient temperature overnight. The mixture was dissolved in dichloromethane (150ml) washed with 5% aqueous lithium chloride solution (3x150ml), dried and evaporated in vacuo. The residue was purified by flash column chromatography eluting with System A (3:2) to give the title compound as a white foam (2.0g).
T.l.c. System A (3:2) Rf=0.3

Intermediate 22

3-[[2-(2-Aminophenyl)-3-bromo-5-benzofuranyl]methyl]-2-ethyl-5,6-dimethyl-4(3H)-pyrimidinone

A solution of Intermediate 21 (1.9g) in dry dichloromethane (40ml) at 0-5°C was treated with trifluoroacetic acid (10ml). The mixture was stirred at ambtient temperature for 4h. The solvent was removed in vacuo. The residue was dissolved in dichloromethane (75ml) and washed with aqueous sodium carbonate solution (1M; 50ml), dried and evaporated to give the title compound as a white foam (1.5g).

| | |
|---|---|
| Assay Found: | C,60.8; H,4.9; N,8.9; |
| $C_{23}H_{22}BrN_3O_2$ requires: | C,61.1; H,4.9; N,9.3% |

Example 1

3-[[3-Bromo-2-[2-(1H-tetrazol-5-yl)phenyl]-5-benzofuranyl]methyl]-2-ethyl-4(3H)-pyrimidinone

Conc. Hydrochloric acid (0.5ml) was added to a solution of Intermediate 7 (240mg) in (1:1) methanol/THF (25ml). After standing under nitrogen at room temperature for 3h, 8% w/v sodium hydrogen carbonate (25ml) was added, and the resultant mixture concentrated in vacuo to 25ml. The solution was extracted with ether (3x25ml) and the extracts discarded. The aqueous phase was acidified to pH4 with 2N hydrochloric acid and extracted with ethyl acetate (3x25ml). The combined ethyl acetate extracts were washed with brine (25ml), dried, and concentrated in vacuo to give a white solid. Purification by flash column chromatography eluting with 5% acetic acid in ether gave the title compound as a white solid (113mg).m.p.140-145°C.
T.l.c (5% acetic acid in ether) Rf = 0.13

Example 2

Methyl 1-[[3-bromo-2-[2-(1H-tetrazol-5-yl)phenyl]-5-benzofuranyl] methyl]-2-butyl-4-(methylamino)-1H-imidazole-5-carboxylate

Conc. Hydrochloric acid (3 drops) was added dropwise to a solution of Intermediate 12 (0.5g) in methanol:dichloromethane (2:1) (30ml) and the mixture stirred at room temperature for 3h. Sodium bicarbonate (8%; 3ml) was added and the methanol evaporated in vacuo. The residue was partitioned between water (20ml) and ether (3x20ml). The aqueous phase was neutralised with hydrochloric acid (1ml) to pH 7 and then extracted with ethyl acetate (3x20ml). The cotined ethyl acetate extracts were washed with brine (1x30ml) and dried. The solvent was evaporated to give the title compound as a colourless foam (345mg) mp 138-140°
T.l.c. dichloromethane:methanol (5:1) Rf 0.35.

Example 3

Methyl 4-[(aminoacetyl)methylamino]-1-[[3-bromo-2-(1H-tetrazol-5-yl)phenyl]-5-benzofuranyl]methyl]-2-butyl-1H-imidazole-5-carboxylate

Trifluoroacetic acid (0.5ml) was added dropwise to a solution of Intermediate 14 (455mg) in dry dichlomethane (5ml) at room temperature under nitrogen. The mixture was stirred for 3h, then, taken to dryness in vacuo. The

residue was partitioned between sodium bicarbonate solution (8%; 10ml) and ether/methanol (20:1) (3x 10ml). The aqueous phase was then taken to pH 6.5 with phosphate buffer (5ml) and extracted with ethyl acetate (3x15ml). The cotined organic extract were washed with brine (1x20ml) and dried. The solvent was evaporated to give the title compound as a colourless solid (270mg) mp 150-155°
T.l.c dichloromethane:methanol (5:1) Rf 0.25

Example 4

4-[(Aminoacetyl)methylamino]-1-[[3-bromo-2-[2-(1H-tetrazol-5-yl) phenyl]-5-benzofuranyl]methyl]-2-butyl-1H-imidazole-5-carboxylic acid trifluoroacetate salt (1:1)

Trifluoroacetic acid (0.5ml) was added dropwise to a solution of Intermediate 15 (658mg) in dry dichloromethane (10ml) and the solution stirred at room temperature under nitrogen for 5h. A further portion of trifluroacetic acid (0.5ml) was added and stirring continued for 16h. The solvent was evaporated to give the title compound as a colourless foam (0.67g).
T.l.c dichloromethane:methanol (5:1) Rf 0.5
n.m.r. ($CD_3OD$) δ 0.87 (3H,t), 1.35 (2H,m), 1.61 (2H,m), 2.78 (2H,t), 3.28 (3H,s), 3.62 (2H,s), 5.82 (2H,s), 7.18 (2H,m), 7.40 (1H,d), 7.68-7.98 (4H,m).

Example 5

1-[[3-Bromo-2-[2-(1H-tetrazol-5-yl)phenyl]-5-benzofuranyl]methyl]-2-butyl-1,4,6,7-tetrahydro-4-methyl-imidazo[4,5-e][1,4]diazepine-5,8-dione

Triethylamine (0.21g) was added to a solution of the product of Example 4 (250mg) in dry dichloromethane (15ml) at room temperature under nitrogen and the mixture stirred for 15mins. 1,1-Carbonyldiimidazole (337mg) was added and the mixture heated under reflux for 64h. The solvent was evaporated and the residue partitioned between 1M HCl (10ml) and ethyl acetate (3x15ml). The cotined organic extracts were washed with brine (1x20ml) and dried. The solvent was evaporated to give a colourless foam (0.25g). The crude material was purified by flash column chromatography eluting with dichloromethane/methanol (10:1) to give the title compound as a colourless foam (94mg) m.p. 138-143°
n.m.r. (400 MHz; $CD_3OD$) δ 0.87 (t,3H), 1.35 (m,2H), 1.58 (m,2H), 2.74 (t,2H), 3.44(s,3H), 3.84(s,2H), 5.70(s,2H), 7.21 (dd1H), 7.29 (s,1H), 7.38(d,1H), 7.75 (m,2H), 7.87 (dd,1H), 7.95 (dd,1H).

Example 6

Methyl 4-amino-1-[[3-bromo-2-[2-(1H-tetrazol-5-yl)phenyl]-5-benzofuranyl]methyl]-2-butyl-1H-imidazole-5-carboxylate

Intermediate 11 (0.3g) in a methanol (15ml) was treated with conc. hydrochloric acid (0.3ml) according to the method of Example 2 to give the title compound as a colourless foam (138mg).
T.l.c. dichloromethane:methanol (5:1) Rf = 0.65
n.m.r. (250MHz; DMSO) δ 0.84 (t,3H), 1.31 (m,2H), 1.56 (m,2H), 2.58 (m,2H), 3.67 (s,3H), 5.56 (s,2H), 5.80 (br.s, 2H), 7.10 (dd +s, 2H), 7.51 (d, IH), 7.76 (m, 2H), 7.86(m,1H), 7.98 (m, 1H).

Example 7

(E)-Methyl α-[[1-[[3-bromo-2-[2-(1H-tetrazol-5-yl)phenyl]-5-benzofuranyl]methyl]-2-butyl-1H-imidazol-5-yl]methylene]-2-thiophenepropanoate

A solution of Intermediate 17 (155mg) in ethanol (5ml) and concentrated hydrochloric acid (0.2ml) was stirred at room temperature for 16 hours. The pH was adjusted to pH9 ($Na(CO_3)_2$) and the solvent removed in vacuo. The residue was partitioned between water (15ml) and ether (3x15ml). The aqueous layer was acidified to $pH_4$ (2N HCl) and extracted with ethyl acetate (3x15ml). The ethyl acetate fractions were cotined, dried and concentrated to afford the title compound (60mg). m.p. 192-195°C
T.l.c. dichloromethane:ethanol:ammonia (80:20:2) Rf = 0.57

Example 8

(E)-α-[[1-[[3-Bromo-2-[2-(1H-tetrazol-5-yl)phenyl]-5-benzofuranyl]methyl]-2-butyl-1H-imidazol-5-yl]methylene]-2-thiophenepropanoic acid

A solution of the product of Example 7 (55mg), ethanol (4ml) and 2N sodium hydroxide (0.5ml) was stirred at room temperature for 24hours. The pH was adjusted to pH4 (2N HCl) and the mixture diluted with water (5ml) and cooled in ice. The suspension was filtered to afford the title compound (50mg).
T.l.c. dichloromethane:ethanol:ammonia (40:30:1) Rf = 0.09
m.p. 205-208°

Example 9

(a) (E)-α-[[1-[[3-Bromo-2-[2-(1H-tetrazol-5-yl)phenyl]-5-benzofuranyl]methyl]-2-butyl-1H-imidazol-5-yl]methylene]-2-thiophenepropanamide

(b) (Z)-α-[[1-[[3-Bromo-2-[2-(1H-tetrazol-5-yl)phenyl]-5-benzofuranyl]methyl]-2-butyl-1H-imidazol-5-yl]methylene]-2-thiophenepropanamide

A solution of Intermediate 20 (73mg), concentrated hydrochloric acid (0.1ml) and ethanol (8ml) was stirred at room temperature for 20h. The solution was basified to pH9 ($Na(CO_3)_2$) and concentrated in vacuo. The residue was partitioned between water (8ml) and ether (3x5ml). The aqueous phase was then acidified to pH3 (2N HCl) and extracted with ethyl acetate (3x8ml). The insoluble material was removed by filtration to afford the title compound (a) (24mg).

| | |
|---|---|
| Mass Spec. | $MH^+$ (calc.) 643.5 |
| | $MH^+$ (obs.) 643.5 |
| n.m.r. | (DMSO) δ8.03-7.88 (2H,2xm), 7.88-7.77 (2H,m), 7.63 (1H,s), 7.55 (1H,d), 7.4-7.1 (5H,dd,d, vbrs,brs,dd), 6.95 (1H,dd), 6.76 (1H,brd), 5.7 (2H,s), 4.08 (2H,s), 2.95 (2H,t), 1.58 (2H,m), 1.30 (2H,m), 0.84 (3H,t). |

The cotined ethyl acetate fractions were dried and concentrated in vacuo to afford the title compound (b) (10mg).

| | |
|---|---|
| Mass Spec. | $MH^+$ (calc.) 643.5 |
| | $MH^+$ (obs.) 643.4 |
| n.m.r. | (DMSO) δ8.03-7.83 (2H,2xm), 7.83-7.73 (2H,m), 7.68 (1H,brs), 7.53 (1H,d), 7.32 (1H,dd), 7.22 (2H,s), 7.15-7.00 (3H,brs,vbrs,dd), 6.93 (1H,dd), 6.82 (1H,brd), 5.53 (2H,s), 4.03 (2H,s), 2.68 (2H,t), 1.58 (2H,m), 1.30 (2H,m), 0.83 (3H,t). |

Example 10

N-[2-[3-Bromo-5-[(2-ethyl-1,6-dihydro-4,5-dimethyl-6-oxo-1-pyrimidinyl)methyl]-2-benzofuranyl]phenyl]-2,2,2-trifluoromethanesulphonamide

A mixture of the Intermediate 22 (0.8g), dry triethylamine (0.31ml) in dry dichloromethane (20ml) was treated with a solution of trifluoromethanesulphonic anhydride (1M; 2ml) in dry dichloromethane dropwise over a 5min period at -70°. The mixture was stirred at -70° for 20min and water (5ml) was cautiously added. The mixture was warmed to ambient temperature and the product was extracted with dichloromethane (50ml). The organic solution was washed with water (3x50ml), dried, filtered and evaporated in vacuo. The residue was crystallized from methyl acetate/hexane to give the title compound as an off-white solid (0.65g), m.p. 182-4°.

| | |
|---|---|
| Assay Found: | C,49.6; H,3.7; N,7.1; |
| $C_{24}H_{21}BrF_3N_3O_4S$ requires: | C,49.3; H,3.6; N,7.2% |

The compounds of the invention are tested in vitro for angiotensin II receptor antagonism. Aortic strips are obtained from male New Zealand white rabbits and prepared for recording isometric contractions in response to cumulative addition of angiotensin II. The potencies of test antagonists are assessed by measuring their abilities to displace the angiotensin II cumulative concentration response curve. The method used is that of Ackerly et al., Proc. Natl. Acad. Sci., 74(12), pp5725-28 (1977) with the exception that the final composition of the physiological salt solution is as given below in Table 1:

TABLE 1

| Ingredient | Amount (mM) |
|---|---|
| $Na^+$ | 143.4 |
| $K^+$ | 5.9 |
| $Mg^{2+}$ | 0.6 |
| $Ca^{2+}$ | 1.3 |
| $Cl^-$ | 124.5 |
| $HPO^{4-}$ | 1.2 |
| $SO_4^{2-}$ | 0.6 |
| $HCO_3^-$ | 25.0 |
| glucose | 11.1 |
| indomethacin | 0.005 |
| ascorbic acid | 0.1 |

The tissues are initially challenged with $K^+$ (80mM) and then washed at 0, 5, 10 and 15 minutes after the response to $K^+$ has plateaued. After a further 45 minutes an angiotensin II cumulative response curve is constructed (0.1nM to 0.1μM in 10-fold increments) and the tissues are washed as before. A second, third and fourth angiotensin II cumulative response curve (0.1nM to 0.1μM in 3-fold increments) is then constructed at hourly intervals (15 minutes washing after each curve followed by 45 minutes equilibration). The compounds of the invention (30μM) are tested for angiotensin II receptor antagonism by application 45 minutes before construction of the fourth angiotensin II curve. The third and fourth angiotensin II curves are expressed graphically and a concentration ratio (CR) is calculated by dividing the angiotensin II $EC_{50}$ value obtained in the presence of the test antagonist (i.e. fourth curve) by the angiotensin II $EC_{50}$ value obtained in the absence of the test antagonist (i.e. third curve).

The potency of the test antagonist is expressed as a pKb which is calculated from the equation :

$$pKb = -\log\left[\frac{CR-1}{[antagonist]}\right]$$

which is a rearrangement of equation 4 described by Furchgott, in Handbook of Exp. Pharmacol., 33, p290 (1972) (eds. Blaschko and Muscholl).

If a compound supresses the maximum response to angiotensin II, a pKb is estimated using the double reciprocal plot technique for insurmountable antagonists, described by T.P. Kenakin, Pharmacol. Rev., 36(3), pp165-222 (esp. 203-204) (1984).

Compounds of the invention will desirably exhibit a pKb in the range between 5 and 12. Thus we have found that the compounds of the invention inhibit the action of the hormone angiotensin II and are therefore useful in the treatment of conditions in which it is desirable to inhibit angiotensin II activity. In particular, the compounds of the Examples are active in the above test.

There is thus provided as a further aspect of the invention a compound of general formula (I) or a physiologically acceptable salt, solvate or metabolically labile ester thereof for use in the treatment of conditions associated with excessive or unregulated angiotensin II activity.

In a further or alternative aspect of the invention there is provided a compound of general formula (I) or a physiologically acceptable salt, solvate or metabolically labile ester thereof for the manufacture of a therapeutic agent for the treatment of conditions associated with excessive or unregulated angiotensin II activity.

There is also provided in a further or alternative aspect of the invention a method for the treatment of conditions associated with excessive or unregulated angiotensin II activity in a mammal including man comprising

administration of an effective amount to a mammal in need of such treatment a compound of general formula (I) or a physiologically acceptable salt, solvate or metabolically labile ester thereof.

In addition, by virtue of their antagonistic activity at angiotensin II receptors, compounds of the present invention will be of value in the treatment of conditions associated with activation of the Renin-Angiotensin System.

There is thus provided a further aspect of the present invention a compound of general formula (I) or a physiologically acceptable salt, solvate or metabolically labile ester thereof for use in the treatment of a condition associated with activation of the Renin-Angiotensin system.

In a further or alternative aspect of the present invention there is provided a compound of general formula (I) or a physiologically acceptable salt, solvate or metabolically labile ester thereof for the manufacture of a therapeutic agent for the treatment of a condition associated with activation of the Renin- Angiotensin System.

There is also provided in a further or alternative aspect of the present inventions a method for the treatment of a condition associated with the activation of the Renin-Angiotensin System in a mammal including man comprising administration of an effective amount to a mammal in need of such treatment of a compound of general formula (I) or a physiologically acceptable salt, solvate or metabolically labile ester thereof.

The following examples illustrate pharmaceutical formulations according to the invention. The term "active ingredient" is used herein to represent a compound of formula (I).

Pharmaceutical Example 1

Oral Tablet A

| | |
|---|---|
| Active Ingredient | 700mg |
| Sodium starch glycollate | 10mg |
| Microcrystalline cellulose | 50mg |
| Magnesium stearate | 4mg |

Sieve the active ingredient and microcrystalline cellulose through a 40 mesh screen and blend in a appropriate blender. Sieve the sodium starch glycollate and magnesium stearate through a 60 mesh screen, add to the powder blend and blend until homogeneous. Compress with appropriate punches in an automatic tablet press. The tablets may be coated with a thin polymer coat applied by the film coating techniques well known to those skilled in the art. Pigments may be incorporated in the film coat.

Pharmaceutical Example 2

Oral Tablet B

| | |
|---|---|
| Active Ingredient | 500mg |
| Lactose | 100mg |
| Maize Starch | 50mg |
| Polyvinyl pyrrolidone | 3mg |
| Sodium starch glycollate | 10mg |
| Magnesium stearate | 4mg |
| Tablet Weight | 667mg |

Sieve the active ingredient, lactose and maize starch through a 40 mesh screen and blend the powders in a suitable blender. Make an aqueous solution of the polyvinyl pyrrolidone (5 - 10% w/v). Add this solution to the blended powders and mix until granulated; pass the granulate through a 12 mesh screen and dry the granules in a suitable oven or fluid bed dryer. Sieve the remaining components through a 60 mesh screen and blend them with the dried granules. Compress, using appropriate punches, on an automatic tablet press.

The tablets may be coated with a thin polymer coat applied by film coating techniques well known to those skilled in art. Pigments may be incorporated in the film coat.

Pharmaceutical Example 3

Inhalation Cartridge

| | |
|---|---|
| Active Ingredient | 1mg |
| Lactose | 24mg |

Blend active ingredient, particle size reduced to a very fine particle size (weight mean diameter ca. 5μm)

with the lactose in a suitable powder blender and fill the powder blender into No. 3 hard gelatin capsules.

The contents of the cartridges may be administered using a powder inhaler.

Pharmaceutical Example 4

Injection Formulation

| | | % w/v |
|---|---|---|
| Active ingredient | | 1.00 |
| Water for injections B.P. | to | 100.00 |

Sodium chloride may be added to adjust the tonicity of the solution and the pH may be adjusted to that of maximum stability and/or to facilitate solution of the active ingredient using dilute acid or alkali or by the addition of suitable buffer salts. Antioxidants and metal chelating salts may also be included.

The solution is prepared, clarified and filled into appropriate sized ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen.

## Claims

1. A compound of the general formula (I):

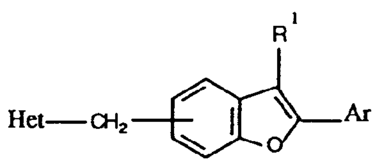

(I)

or a physiologically acceptable salt, solvate or metabolically labile ester thereof
wherein
$R^1$ represents a hydrogen atom or a halogen atom or a group selected from $C_{1-6}$alkyl, $C_{2-6}$alkenyl, fluoro$C_{1-6}$alkyl, $C_{1-6}$alkoxy ,-CHO, $-CO_2H$ or $-COR^2$;
Ar represents the group

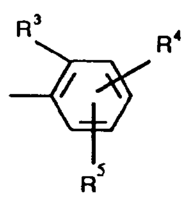

;

$R^2$ represents a group selected from $C_{1-6}$alkyl, $C_{2-6}$alkenyl, $C_{1-6}$alkoxy or the group $-NR^{14}R^{15}$;
$R^3$ represents a group selected from $-CO_2H$, $-NHSO_2CF_3$ or a C-linked tetrazolyl group;
$R^4$ and $R^5$ which may be the same or different each independently represent a hydrogen atom or a halogen atom or a $C_{1-6}$alkyl group;
Het represents a group selected from

(a) 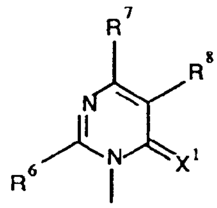 , (b) 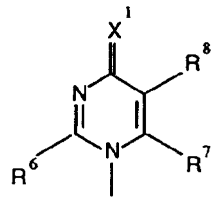

(c) 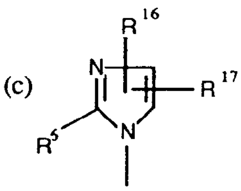 , (d) 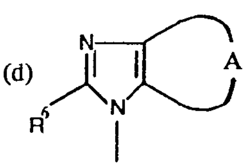 or

(e) 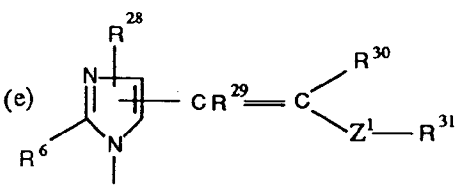

A represents, when read in a clockwise or anti-clockwise direction, a group selected from

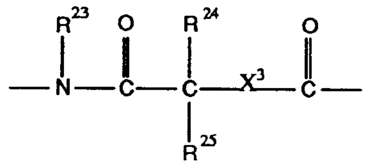 ,

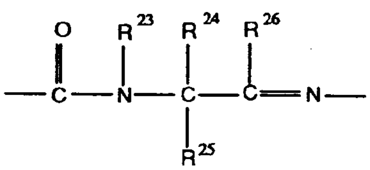 , 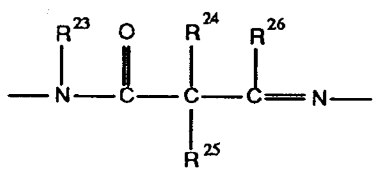 ,

or 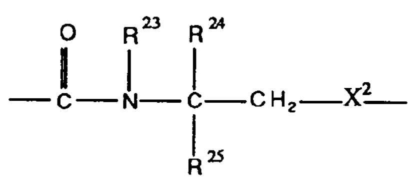 ;

$R^6$ represents a hydrogen atom or a group selected from $C_{1-6}$alkyl, $C_{2-6}$alkenyl $C_{1-6}$alkylthio, $C_{1-6}$alkoxy, $C_{3-7}$cycloalkyl or $C_{3-7}$cycloalkyl$C_{1-4}$alkyl;
$R^7$ represents a hydrogen atom, a halogen atom or a group selected from $C_{1-6}$alkyl, $C_{2-6}$alkenyl, fluoro$C_{1-6}$alkyl $-(O)_m(CH_2)_nR^{10}$, $-(CH_2)_pCOR^{11}$ or $-(CH_2)_qNR^{12}COR^{13}$;
$R^8$ represents a hydrogen atom, a halogen atom or a group selected from $C_{1-6}$alkyl, $C_{2-6}$alkenyl, fluoro$C_{1-6}$alkyl, $-(CH_2)_rR^{10}$ or $-(CH_2)_pCOR^{11}$;
$R^9$ represents a hydrogen atom or a $C_{1-4}$alkyl group;
$R^{10}$ represents a group selected from hydroxy, $C_{1-6}$alkoxy, $C_{1-6}$alkylthio, phenyl or the group $-NR^{14}R^{15}$;
$R^{11}$ represents a hydrogen atom or a group selected from hydroxy, $C_{1-6}$alkyl, $C_{1-6}$alkoxy, phenyl, phenoxy or the group $-NR^{14}R^{15}$;
$R^{12}$ represents a hydrogen atom or a $C_{1-6}$alkyl group,

$R^{13}$ represents a hydrogen atom or a group selected from $C_{1-6}$alkyl, $C_{1-6}$alkoxy, phenyl, phenoxy or the group $-NR^{14}R^{15}$;

$R^{14}$ and $R^{15}$, which may be the same or different, each independently represent a hydrogen atom or a $C_{1-4}$alkyl group or $-NR^{14}R^{15}$ forms a saturated heterocyclic ring which has 5 or 6 ring members and may optionally contain in the ring one oxygen atom;

$R^{16}$ represents a group selected from $-NR^{18}COR^{19}$, $-NHCOR^{19}$, $-NR^{18}SO_2R^{19}$, $-NHSO_2(C_{1-4}alkyl)$ or $-NR^9R^{20}$;

$R^{17}$ represents a group selected from $-CO_2R^{12}$, $-CONHSO_2R^{21}$ or $-CONR^9R^{20}$;

$R^{18}$ represents a group selected from $C_{1-6}$alkyl, aryl or arylmethyl, wherein 'aryl' represents a phenyl group optionally substituted by 1 or 2 substituents selected from a halogen atom or a group selected from $-NO_2$, $-CF_3$, $C_{1-4}$alkyl, $C_{1-4}$alkoxy, $-NR^9R^9$, $-NHCO_2(C_{1-4}alkyl)$, $-CO_2R^9$ or hydroxy;

$R^{19}$ represents a group selected from aryl (as defined above), fluoro$C_{1-4}$alkyl, $-NR^9R^9$, $C_{3-5}$cycloalkyl, heteroaryl,

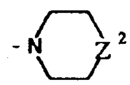

or $C_{1-4}$alkyl optionally substituted by aryl (as defined above), $-NR^9R^9$, $-NCO_2R^{22}$ or $-CO_2R^9$, wherein 'heteroaryl' represents a 5 or 6 membered aromatic ring containing from 1 to 3 heteroatoms selected from the group consisting of N, O or S, said aromatic ring being optionally substituted by 1 or 2 substituents selected from a halogen atom or a group selected from hydroxy, -SH, $C_{1-4}$alkyl, $C_{1-4}$alkoxy, $-CF_3$, $-NO_2$, $-CO_2R^9$ or $-NR^9R^9$;

$R^{20}$ represents a hydrogen atom or a group selected from aryl (as defined above) or $C_{1-6}$alkyl optionally substituted by a group selected from aryl (as defined above), hydroxy, $-CO_2R^9$ or $-NR^9R^9$;

$R^{21}$ represents a group selected from aryl (as defined above), heteroaryl (as defined above), $C_{3-7}$cycloalkyl, fluoro$C_{1-4}$alkyl or a $C_{1-4}$alkyl group optionally substituted by a halogen atom or a group selected from aryl (as defined above), heteroaryl (as defined above), hydroxy, -SH, $C_{1-4}$alkyl, $C_{1-4}$alkoxy, $C_{1-4}$alkylthio, $-CF_3$, $-NO_2$, $-CO_2R^9$, $-NR^9R^9$, $-PO_3H$, $-PO(OH)(C_{1-4}alkoxy)$;

$R^{22}$ represents a hydrogen atom or a group selected from $C_{1-6}$alkyl, $C_{3-7}$cycloalkyl, aryl or arylmethyl (wherein 'aryl' is as defined above);

$R^{23}$ represents a hydrogen atom or a $C_{1-6}$alkyl group optionally substituted by a hydroxy group;

$R^{24}$ and $R^{25}$, which may be the same or different, each independently represent a hydrogen atom or a group selected from $-CO_2R^{12}$, a $C_{1-6}$alkyl, $C_{2-6}$alkenyl or $C_{2-6}$alkynyl group each of which is optionally substituted by a group selected from hydroxy, $C_{1-4}$alkoxy, $-NR^9R^9$, $-CONR^9R^9$, $-CO_2R^{27}$, $-OC(O)R^{27}$, guanidino or $C_{1-4}$alkylthio, a phenyl or phenyl-$C_{1-4}$alkyl group, each of which is optionally substituted by a halogen atom or a group selected from hydroxy, $C_{1-4}$alkyl or $C_{1-4}$alkoxy, or an imidazolyl-$C_{1-4}$alkyl or indolyl-$C_{1-4}$alkyl group;

$R^{26}$ and $R^{27}$, which may be the same or different, each independently represent a hydrogen atom or a group selected from $C_{1-6}$alkyl, phenyl or benzyl;

$R^{28}$ represents a hydrogen atom or a halogen atom or a group selected from $C_{1-6}$alkyl, $C_{2-6}$alkenyl or fluoro$C_{1-6}$alkyl;

$R^{29}$ and $R^{30}$, which may be the same or different, each independently represent a hydrogen atom or a group selected from $C_{1-6}$alkyl, $C_{3-7}$cycloalkyl, thienyl-Y-, pyrazolyl-Y-, imidazolyl-Y, thiazolyl-Y, furyl-Y-, pyrrolyl-Y-, triazolyl-Y-, oxazolyl-Y-, isoxazolyl-Y, pyridyl-Y-, phenyl-Y- or tetrazolyl-Y- wherein each aryl or heterocyclic ring is optionally substituted by a halogen atom or group selected from $C_{1-6}$alkyl, fluoro$C_{1-6}$alkyl, $C_{1-6}$alkoxy, hydroxy, $-NR^{27}R^{27}$, $-CO_2R^{27}$, $-CONR^{27}R^{27}$, $-SO_2NHR^{27}$ or $-SO_3H$;

$R^{31}$ represents $-COR^{11}$, a C-linked tetrazol-5-yl group or the group $-NHSO_2CF_3$;

$X^1$ represents an oxygen atom or sulphur atom or the group $-NR^9-$;

$X^2$ represents an oxygen or sulphur atom or the group $-N(R^{26})-$;

$X^3$ represents an oxygen atom or the group $-N(R^{23})-$;

Y represents a bond or an oxygen or sulphur atom, or a $C_{1-6}$alkyl linkage which is optionally substituted by phenyl or benzyl wherein each aryl ring is optionally substituted by a halogen atom or a group selected from $-NO_2$, $-CF_3$, $C_{1-4}$alkyl, $C_{1-4}$alkoxy, -CN or $CO_2R^{27}$;

$Z^1$ represents a bond or a methylene, ethylene or vinyl linkage, each being optionally substituted by a

Cl,qalkyl group; and
$Z^2$ represents an oxygen or sulphur atom or a group selected from $-N(R^9)-$, $-S(O)-$ or $-S(O)_2-$;
m represents zero or 1;
n represents zero or an integer from 1 to 4;
p represents zero or an integer from 1 to 4;
q represents an integer from 1 to 4; and
r represents an integer from 1 to 4.
provided that when m is 1, n is an integer from 1 to 4.

**2.** A compound as claimed in Claim 1 or a physiologically acceptable salt, solvate or metabolically labile ester thereof
wherein
Het represents either group (a) or group (b);
$R^6$ represents a hydrogen atom, a $C_{1-5}$alkyl group, particularly a $C_{2-4}$alkyl group, especially an ethyl, n-propyl or n-butyl group, a $C_{3-5}$alkyenyl group, a $C_{3-5}$cycloalkyl group, especially a cyclopropyl or cyclobutyl group, or a $C_{3-5}$cycloalkyl$C_{1-2}$alkyl group, especially a cyclopropylmethyl group;
$R^7$ represents a hydrogen atom a $C_{1-4}$alkyl, especially a methyl group, $-(O)_m(CH_2)_nR^{10}$ or $-(CH_2)_pCOR^{11}$;
$R^8$ represents a hydrogen atom, a halogen, especially chlorine, atom, a $C_{1-4}$alkyl, especially a methyl, group, $-(CH_2)_rR^{10}$ or $-(CH_2)_pCOR^{11}$;
$R^{10}$ represents hydroxy, a $C_{1-4}$alkoxy, especially a methoxy, group, a $C_{1-4}$alkylthio, especially a methylthio group, phenyl or $-NR^{14}R^{15}$;
$R^{11}$ represents a hydroxy, a $C_{1-4}$alkoxy, especially a methoxy or ethoxy, group or $-NR^{14}R^{15}$;
$R^{14}$ and $R^{15}$, which may be the same or different, each independently represent a hydrogen atom or a $C_{1-4}$alkyl, especially a methyl or, ethyl, group or $-NR^{14}R^{15}$ forms a morpholino group;
$X^1$ represents an oxygen atom;
m represents zero or 1;
n represents zero, 1 or 2;
p represents zero, 1 or 2, especially zero or 1; and
r represents 1 or 2.

**3.** A compound as claimed in either Claim 1 or Claim 2 or a physiologically acceptable salt, solvate or metabolically labile ester thereof
wherein
Het represents either group (a) or group (b);
$R^6$ represents a $C_{2-4}$alkyl, especially an ethyl or n-propyl, group;
$R^7$ represents a hydrogen atom, a $C_{1-4}$alkyl, especially a methyl, group, or $-(CH_2)_pCOR^{11}$;
$R^8$ represents a hydrogen atom, a halogen, especially a chlorine, atom, or a $C_{1-4}$alkyl, especially a methyl, group;
$R^{11}$ represents hydroxy or $-NR^{14}R^{15}$;
$R^{14}$ and $R^{15}$ represents a hydrogen atom or a $C_{1-4}$alkyl, especially a methyl or ethyl, group;
$X^1$ represents an oxygen atom; and
p represents zero.

**4.** A compound as claimed in Claim 1 or a physiologically acceptable salt, solvate or metabolically labile ester thereof
wherein
Het represents the group (c);
$R^9$ represents a hydrogen atom;
$R^{16}$ represents the group $-NR^9R^{20}$; and
$R^{20}$ represents a hydrogen atom or a $C_{1-6}$alkyl group optionally substituted by a hydroxy group.

**5.** A compound as claimed in either Claim 1 or Claim 4 or a physiologically acceptable salt, solvate or metabolically labile ester thereof
wherein
Het represents the group (c);
$R^9$ represents a hydrogen atom;
$R^{12}$ represents a hydrogen atom or a $C_{1-4}$alkyl group;

$R^{17}$ represents the group $-CO_2R^{12}$ or $-CONR^9R^{20}$; and
$R^{20}$ represents a hydrogen atom or a $C_{1-6}$alkyl group optionally substituted by a hydroxy group.

6. A compound as claimed in Claim 1 or a physiologically acceptable salt, solvate or metabolically labile ester thereof
wherein
Het represents the group (d);
A represents the group

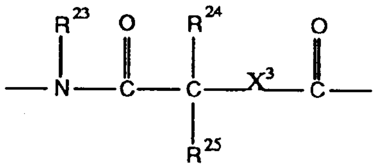

or

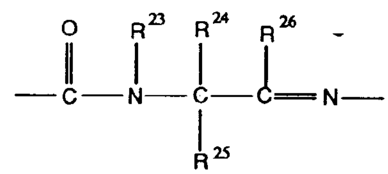

;

$R^{23}$ represents a hydrogen atom;
$R^{24}$ represents a hydrogen atom;
$R^{25}$ represents a hydrogen atom, a $C_{1-6}$alkyl group, optionally substituted by a group selected from hydroxy, amino, guanidino, $C_{1-4}$alkylthio, $-CONR^9R^9$ (wherein $-NR^9R^9$ represents an $NH_2$ group), $-CO_2R^{27}$ (wherein $R^{27}$ represents a hydrogen atom or a $C_{1-4}$alkyl group or $-OC(O)R^{27}$ (wherein $R^{27}$ represents a hydrogen atom or a $C_{1-4}$alkyl group), or a phenyl, benzyl, 4-hydroxybenzyl, 4-imidazolylmethyl or 3-indolylmethyl group;
$R^{26}$ represents a hydrogen atom; and
$X^3$ represents $-N(R^{23})-$.

7. A compound as claimed in Claim 1 wherein Het represents the group (e) and $R^{28}$ represents a hydrogen atom, a halogen atom, particularly a chlorine atom, or a $C_{1-4}$alkyl group, particularly a methyl group.

8. A compound as claimed in either Claim 1 or Claim 7 wherein Het represents the group (e) and $R^{29}$ represents a hydrogen atom or a $C_{1-4}$alkyl group, particularly a methyl or ethyl group.

9. A compound as claimed in any one of Claims 1, 7 and 8 wherein Het represents the group (e) and $R^{30}$ represents a group selected from thienyl-Y-, imidazolyl-Y-, furyl-Y-, Pyridyl-Y-, or phenyl-Y- wherein each aryl or heterocyclic group is optionally substituted by a hydroxy group or a $C_{1-4}$alkyl, particularly a methyl, group or $C_{1-4}$alkoxy, particularly a methoxy, group, and Y represents a bond or a methylene linkage.

10. A compound as claimed in any one of Claims 1, 7, 8 and 9 wherein Het represents the group (e) and $R^{31}$ represents the group $COR^{11}$ wherein $R^{11}$ represents a hydroxy or $C_{1-4}$alkoxy, particularly methoxy or ethoxy, group or the group $-NR^{14}R^{15}$, particularly wherein $R^{14}$ and $R^{15}$ each independently represents a hydrogen atom or a $C_{1-4}$alkyl, particularly a methyl or ethyl, group, or $R^{31}$ represents a C-linked tetrazol-5-yl group, and $Z^1$ represents a bond or methylene linkage.

11. A compound as claimed in any one of Claims 1 to 10 wherein $R^6$ represents a hydrogen atom or a group selected from $C_{1-5}$alkyl, $C_{3-5}$alkenyl, $C_{1-5}$alkoxy, $C_{3-5}$cycloalkyl or $C_{3-5}$cycloalkyl$C_{1-2}$alkyl, particularly a $C_{2-4}$alkyl group, for example, an ethyl, n-propyl or n-butyl group.

12. A compound as claimed in any one of Claims 1 to 11 wherein $R^1$ represents a hydrogen atom or a halogen atom or a group selected from $C_{1-6}$alkyl, fluoro$C_{1-6}$alkyl or $C_{1-6}$alkoxy, particularly a bromine atom.

13. A compound as claimed in any one of Claims 1 to 12 wherein the group Het-$CH_2$- is attached at the 5- or 6-position on the benzofuran ring, particularly at the 5-position on the benzofuran ring.

14. A compound as claimed in any one of Claims 1 to 13 wherein $R^3$ represents the group $-NHSO_2CF_3$ or a C-linked tetrazolyl group or the group $-NHSO_2CF_3$.

15. A compound as claimed in any one of Claims 1 to 14 wherein $R^4$ and $R^5$ each independently represent a hydrogen atom or a halogen atom, particularly, $R^4$ and $R^5$ each represent a hydrogen atom.

16. A compound as claimed in Claim 1 or a physiologically acceptable salt, solvate or metabolically labile ester thereof
wherein
$R^1$ represents a halogen atom;
Ar represents the group

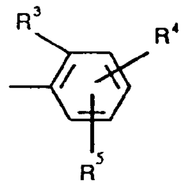
;

$R^3$ represents the group $-NHSO_2CF_3$ or a C-linked tetrazolyl group;
$R^4$ and $R^5$ each independently represent a hydrogen atom;
Het represents a group selected from

(a) 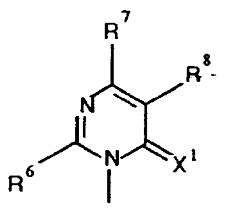 , (c) 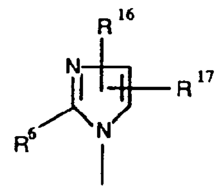 ,

(d) 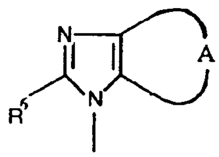 or (e) 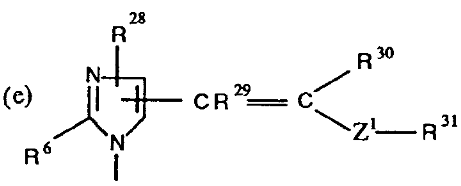

A represents, when read in a clockwise direction, the group

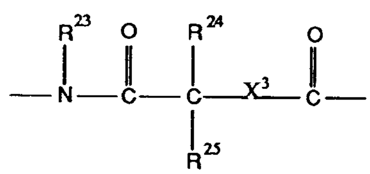
;

$R^6$ represents a $C_{1-6}$alkyl group;
$R^7$ represents a hydrogen atom or a $C_{1-6}$ alkyl group;
$R^8$ represents a hydrogen atom or a $C_{1-6}$alkyl group;
$R^{16}$ represents a group selected from $-NR^{18}COR^{19}$ or $-NR^9R^{20}$ (wherein $R^9$ represents a hydrogen atom or a $C_{1-4}$alkyl group and $R^{20}$ represents a hydrogen atom or a $C_{1-6}$alkyl group);
$R^{17}$ represents the group $-CO_2R^{12}$ (wherein $R^{12}$ represents a hydrogen atom or a $C_{1-6}$alkyl group);
$R^{23}$ represents a hydrogen atom or $C_{1-6}$alkyl group;
$R^{24}$ and $R^{25}$ each independently represent a hydrogen atom;
$R^{28}$ represents a hydrogen atom;
$R^{29}$ represents a hydrogen atom;
$R^{30}$ represents the group thienyl-Y-;
$R^{31}$ represents the group $-COR^{11}$ (wherein $R^{11}$ represents a hydrogen atom or a group selected from $C_{1-6}$alkyl or $-NH_2$);
$X^1$ represents an oxygen atom;

$X^3$ represents the group $-N(R^{23})-$;
Y represents a bond; and
$Z^1$ represents a bond.

**17.** A compound as claimed in Claim 1 selected from

3-[[3-bromo-2-[2-(1H-tetrazol-5-yl)phenyl]-5-benzofuranyl]methyl]-2-ethyl-4(3H)-pyrimidinone;

3-[[3-bromo-2-[2-(1H-tetrazol-5-yl)phenyl]-5-benzofuranyl]methyl]-2-ethyl-6-methyl-4(3H)-pyrimidinone;

3-[[3-bromo-2-[2-(1H-tetrazol-5-yl)phenyl]-5-benzofuranyl]methyl]-2-ethyl-5-methyl-4(3H)-pyrimidinone;

3-[[3-bromo-2-[2-(1H-tetrazol-5-yl)phenyl]-5-benzofuranyl]methyl]-2-ethyl-5,6-dimethyl-4(3H)-pyrimidinone;

3-[[3-bromo-2-[2-[[(trifluoromethyl)sulphonyl]amino]phenyl]-5-benzofuranyl]methyl]-2-ethyl-4(3H)-pyrimidinone;

3-[[3-bromo-2-[2-[[(trifluoromethyl)sulphonyl]amino]phenyl]-5-benzofuranyl]methyl]-2-ethyl-6-methyl-4(3H)-pyrimidinone;

3-[[3-bromo-2-[2-[[(trifluoromethyl)sulphonyl]amino]phenyl]-5-benzofuranyl]methyl]-2-ethyl-5-methyl-4(3H)-pyrimidinone;

3-[[3-bromo-2-[2-[[(trifluoromethyl)sulphonyl]amino]phenyl]-5-benzofuranyl]methyl]-2-ethyl-5,6-dimethyl-4(3H)-pyrimidinone;

3-[[3-bromo-2-[2-(1H-tetrazol-5-yl)phenyl]-5-benzofuranyl]methyl]-2-propyl-4(3H)-pyrimidinone;

3-[[3-bromo-2-[2-(1H-tetrazol-5-yl)phenyl]-5-benzofuranyl]methyl]-6-methyl-2-propyl-4(3H)-pyrimidinone;

3-[[3-bromo-2-[2-(1H-tetrazol-5-yl)phenyl]-5-benzofuranyl]methyl]-5-methyl-2-propyl-4(3H)-pyrimidinone;

3-[[3-bromo-2-[2-(1H-tetrazol-5-yl)phenyl]-5-benzofuranyl]methyl]-5,6-dimethyl-2-propyl-4(3H)-pyrimidinone;

3-[[3-bromo-2-[2-[[(trifluoromethyl)sulphonyl]amino]phenyl]-5-benzofuranyl]methyl]-2-propyl-4(3H)-pyrimidinone;

3-[[3-bromo-2-[2-[[(trifluoromethyl)sulphonyl]amino]phenyl]-5-benzofuranyl]methyl]-6-methyl-2-propyl-4(3H)-pyrimidinone;

3-[[3-bromo-2-[2-[[(trifluoromethyl)sulphonyl]amino]phenyl]-5-benzofuranyl]methyl]-5-methyl-2-propyl-4(3H)-pyrimidinone;

3-[[3-bromo-2-[2-[[(trifluoromethyl)sulphonyl]amino]phenyl]-5-benzofuranyl]methyl]-5,6-dimethyl-2-propyl-4(3H)-pyrimidinone;

1-[[3-bromo-2-[2-(1H-tetrazol-5-yl)phenyl]-5-benzofuranyl]methyl]-2-ethyl-4(1H)-pyrimidinone;

1-[[3-bromo-2-[2-(1H-tetrazol-5-yl)phenyl]-5-benzofuranyl]methyl]-2-propyl-4(1H)-pyrimidinone;

1-[[3-bromo-2-[2-[[(trifluoromethyl)sulphonyl]amino]phenyl]-5-benzofuranyl]methyl]-2-ethyl-4(1H)-pyrimidinone;

1-[(3-bromo-2-[2-[[(trifluoromethyl)sulphonyl]amino]phenyl]-5-benzofuranyl]methyl]-2-propyl-4(1H)-pyrimidinone;

3-[[3-bromo-2-[2-(1H-tetrazol-5-yl)phenyl]-5-benzofuranyl]methyl]-2-ethyl-3,6-dihydro-6-oxo-4-pyrimidinecarboxylic acid;

3-[[3-bromo-2-[2-(1H-tetrazol-5-yl)phenyl]-5-benzofuranyl]methyl]-3,6-dihydro-6-oxo-2-propyl-4(3H)-pyrimidinecarboxylic acid;

3-[[3-bromo-2-[2-(1H-tetrazol-5-yl)phenyl]-5-benzofuranyl]methyl]-5-chloro-2-ethyl-3,6-dihydro-6-oxo-4-pyrimidinecarboxylic acid;

3-[[3-bromo-2-[2-(1H-tetrazol-5-yl)phenyl]-5-benzofuranyl]methyl]-5-chloro-3,6-dihydro-6-oxo-2-propyl-4(3H)-pyrimidinecarboxylic acid;

3-[[3-bromo-2-[2-[[(trifluoromethyl)sulphonyl]amino]phenyl]-5-benzofuranyl]methyl]-2-ethyl-3,6-dihydro-6-oxo-4-pyrimidinecarboxamide;

3-[[3-bromo-2-[2-[[(trifluoromethyl)sulphonyl]amino]phenyl]-5-benzofuranyl]methyl]-2-ethyl-3,6-dihydro-N-methyl-6-oxo-4-pyrimidine carboxamide;

3-[[3-bromo-2-[2-[[(trifluoromethyl)sulphonyl]amino]phenyl]-5-benzofuranyl]methyl]-N,2-diethyl-3,6-dihydro-6-oxo-4-pyrimidine carboxamide;

3-[[3-bromo-2-[2-[[(trifluoromethyl)sulphonyl]amino]phenyl]-5-benzofuranyl]methyl]-3,6-dihydro-6-oxo-2-propyl-4-pyrimidine carboxamide;

3-[[3-bromo-2-[2-[[(trifluoromethyl)sulphonyl]amino]phenyl]-5-benzofuranyl]methyl]-3,6-dihydro-N-methyl-6-oxo-2-propyl-4-pyrimidinecarboxamide;

3-[[3-bromo-2-[2-[[(trifluoromethyl)sulphonyl]amino]phenyl]-5-benzofuranyl]methyl]-N-ethyl-3,6-dihydro-6-oxo-2-propyl-4-pyrimidinecarboxamide;

3-[[3-bromo-2-[2-[[(trifluoromethyl)sulphonyl]amino]phenyl]-5-benzofuranyl]methyl]-5-chloro-2-ethyl-3,6-dihydro-6-oxo-4-pyrimidinecarboxamide;

3-[[3-bromo-2-[2-[[(trifluoromethyl)sulphonyl]amino]phenyl]-5-benzofuranyl]methyl]-5-chloro-2-ethyl-3,6-dihydro-N-methyl-6-oxo-4-pyrimidinecarboxamide;

3-[[3-bromo-2-[2-[[(trifluoromethyl)sulphonyl]amino]phenyl]-5-benzofuranyl]methyl]-5-chloro-N,2-diethyl-3,6-dihydro-6-oxo-4-pyrimidinecarboxamide;

3-[[3-bromo-2-[2-[[(trifluoromethyl)sulphonyl]amino]phenyl]-5-benzofuranyl]methyl]-5-chloro-3,6-dihydro-6-oxo-2-propyl-4-pyrimidinecarboxamide;

3-[[3-bromo-2-[2-[[(trifluoromethyl)sulphonyl]amino]phenyl]-5-benzofuranyl]methyl]-5-chloro-3,6-dihydro-N-methyl-6-oxo-2-propyl-4-pyrimidinecarboxamide;

3-[[3-bromo-2-[2-[[(trifluoromethyl)sulphonyl]amino]phenyl]-5-benzofuranyl]methyl]-5-chloro-N-ethyl-3,6-dihydro-6-oxo-2-propyl-4-pyrimidinecarboxamide;

1-[[3-bromo-2-[2-(1H-tetrazol-1-yl)phenyl]-5-benzofuranyl]methyl]-2-ethyl-1,4,6,7-tetrahydro-4-methyl-5,8-dioxoimidazo[4,5-e][1,4]diazepine-6-carboxylic acid;

1-[[3-bromo-2-[2-(1H-tetrazol-1-yl)phenyl]-5-benzofuranyl]methyl]-1,4,6,7-tetrahydro-4-methyl-5,8-dioxo-2-propylimidazo[4,5-e][1,4]diazepine-6-carboxylic acid;

1-[[3-bromo-2-[2-[[(trifluoromethyl)sulphonyl]amino]phenyl]-5-benzofuranyl]methyl]-2-ethyl-1,4,6,7-tetrahydro-4-methyl-5,8-dioxoimidazo[4,5-e][1,4]diazepine-6-carboxylic acid;

1-[[3-bromo-2-[2-[[(trifluoromethyl)sulphonyl]amino]phenyl]-5-benzofuranyl]methyl]-1,4,6,7-tetrahydro-4-methyl-5,8-dioxo-2-propylimidazo[4,5-e][1,4]diazepine-6-carboxylic acid;

1-[[3-bromo-2-[2-(1H-tetrazol-1-yl)phenyl]-5-benzofuranyl]methyl]-2-ethyl-1,4,6,7-tetrahydro-4-methylimidazo[4,5-e][1,4]diazepine-5,8- dione;

1-[[3-bromo-2-[2-(1H-tetrazol-1-yl)phenyl]-5-benzofuranyl]methyl]-1,4,6,7-tetrahydro-4-methyl-2-propylimidazo[4,5-e][1,4]diazepine-5,8-dione;

N-[2-[3-bromo-5-[(2-ethyl-1,4,6,7-tetrahydro-4-methyl-5,8-dioxoimidazo[4,5-e][1,4]diazepin-1-yl)methyl]-2-benzofuranyl]phenyl]-2,2,2-trifluoromethanesulphonamide;

N-[2-[3-bromo-5-[(1,4,6,7-tetrahydro-4-methyl-5,8-dioxo-2-propylimidazo[4,5-e][1,4]diazepin-1-yl)methyl]-2-benzofuranyl]phenyl]-2,2,2-trifluoromethanesulphonamide;

4-amino-1-[[3-bromo-2-[2-[[(trifluoromethyl)sulphonyl]amino]phenyl]-5-benzofuranyl]methyl]-2-ethyl-1H-imidazole-5-carboxamide;

4-amino-1-[[3-bromo-2-[2-[[(trifluoromethyl)sulphonyl]amino]phenyl]-5-benzofuranyl]methyl]-2-propyl-1H-imidazole-5-carboxamide;

1-[[3-bromo-2-[2-[[(trifluoromethyl)sulphonyl]amino]phenyl]-5-benzofuranyl]methyl]-2-ethyl-4-(methylamino)-1H-imidazole-5-carboxamide;

1-[[3-bromo-2-[2-[[(trifluoromethyl)sulphonyl]amino]phenyl]-5-benzofuranyl]methyl]-4-(methylamino)-2-propyl-1H-imidazole-5-carboxamide;

1-[[3-bromo-2-[2-[[(trifluoromethyl)sulphonyl]amino]phenyl]-5-benzofuranyl]methyl]-4-(dimethylamino)-2-ethyl-1H-imidazole-5-carboxamide;

1-[[3-bromo-2-[2-[[(trifluoromethyl)sulphonyl]amino]phenyl]-5-benzofuranyl]methyl]-4-(dimethylamino)-2-propyl-1H-imidazole-5-carboxamide;

4-amino-1-[[3-bromo-2-[2-[[(trifluoromethyl)sulphonyl]amino]phenyl]-5-benzofuranyl]methyl]-2-ethyl-N-methyl-1H-imidazole-5-carboxamide;

4-amino-1-[[3-bromo-2-[2-[[(trifluoromethyl)sulphonyl]amino]phenyl]-5-benzofuranyl]methyl]-N,2-diethyl-1H-imidazole-5-carboxamide;

4-amino-1-[[3-bromo-2-[2-[[(trifluoromethyl)sulphonyl]amino]phenyl]-5-benzofuranyl]methyl]-N-ethyl-2-propyl-1H-imidazole-5-carboxamide;

1-[[3-bromo-2-[2-[[(trifluoromethyl)sulphonyl]amino]phenyl]-5-benzofuranyl]methyl]-N,2-diethyl-4-(methylamino)-1H-imidazole-5-carboxamide;

1-[[3-bromo-2-[2-[[(trifluoromethyl)sulphonyl]amino]phenyl]-5-benzofuranyl]methyl]-N-ethyl-4-(methylamino)-2-propyl-1H-imidazole-5-carboxamide;

1-[[3-bromo-2-[2-[[(trifluoromethyl)sulphonyl]amino]phenyl]-5-benzofuranyl]methyl]-4-(dimethylamino)-N,2-diethyl-1H-imidazole-5-carboxamide;

1-[[3-bromo-2-[2-[[(trifluoromethyl)sulphonyl]amino]phenyl]-5-benzofuranyl]methyl]-4-(dimethylamino)-N-ethyl-2-propyl-1H-imidazole-5-carboxamide;

4-amino-1-[[3-bromo-2-[2-[[(trifluoromethyl)sulphonyl]amino]phenyl]-5-benzofuranyl]methyl]-N-methyl-2-propyl-1H-imidazole-5-carboxamide;

1-[[3-bromo-2-[2-[[(trifluoromethyl)sulphonyl]amino]phenyl]-5-benzofuranyl]methyl]-2-ethyl-N-methyl-4-(methylamino)-1H-imidazole-5-carboxamide;

1-[[3-bromo-2-[2-[[(trifluoromethyl)sulphonyl]amino]phenyl]-5-benzofuranyl]methyl]-N-methyl-4-(methylamino)-2-propyl-1H-imidazole-5-carboxamide;

1-[[3-bromo-2-[2-[[(trifluoromethyl)sulphonyl]amino]phenyl]-5-benzofuranyl]methyl]-4-(dimethylamino)-2-ethyl-N-methyl-1H-imidazole-5-carboxamide;

1-[[3-bromo-2-[2-[[(trifluoromethyl)sulphonyl]amino]phenyl]-5-benzofuranyl]methyl]-4-(dimethylamino)-N-methyl-2-propyl-1H-imidazole-5-carboxamide;

(E)-α-[[1-[[3-bromo-2-[2-(1H-tetrazol-5-yl)phenyl]-5-benzofuranyl]methyl]-2-propyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-4-chloro-2-propyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-4-methyl-2-propyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-ethyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-4-chloro-2-ethyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-ethyl-4-methyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-butyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-butyl-4-chloro-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-butyl-4-methyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-2-propyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-4-chloro-2-propyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-4-methyl-2-propyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-2-ethyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-4-chloro-2-ethyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-2-ethyl-4-methyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-2-propyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-4-chloro-2-propyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-4-methyl-2-propyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-2-ethyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-4-chloro-2-ethyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-2-ethyl-4-methyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-2-propyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-4-chloro-2-propyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-4-methyl-2-propyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-2-ethyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-4-chloro-2-ethyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-2-ethyl-4-methyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-2-propyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-4-chloro-2-propyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-4-methyl-2-propyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-2-ethyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-4-chloro-2-ethyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-2-ethyl-4-methyl-1H-imidazol-5-yl]methylene]-N-ethyl-2-thiophenepropanamide;

or a physiologically acceptable salt, solvate or metabolically labile ester thereof.

**18.** A process for the preparation of a compound as claimed in any one of Claims 1 to 17 or a physiologically acceptable salt, solvate or metabolically labile ester thereof which comprises:

(A), where Het represents group (a), (c) or (e), treating a compound of general formula (II)

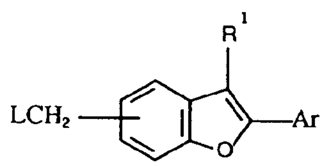
(II)

wherein L is a leaving group and $R^1$ and Ar are as defined in Claim 1, with a heterocycle of formula (IIIa), (IIIb) or (IIIc)

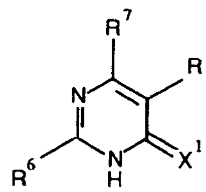
(IIIa)

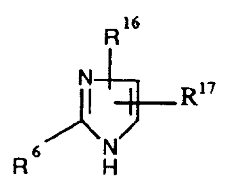
(IIIb)

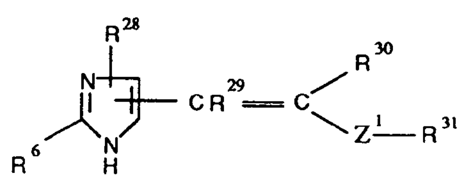
(IIIc)

wherein $R^6$, $R^7$, $R^8$, $R^{16}$, $R^{17}$, $R^{28}$, $R^{29}$, $R^{30}$, $R^{31}$, $X^1$ and $Z^1$ are as defined in Claim 1 followed, if necessary, by the removal of any protecting groups where present; or

(B) deprotecting a protected intermediate of general formula (IV)

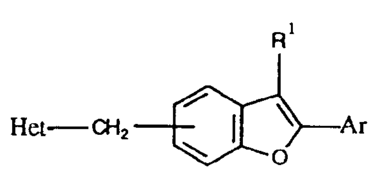
(IV)

wherein $R^1$, Ar and het are as defined in Claim 1, except that at least one reactive group is blocked by a protecting group; or

(C), where the substituent $R^3$ in the group Ar represents a C-linked tetrazolyl group, by reacting a compound of general formula (Ia)

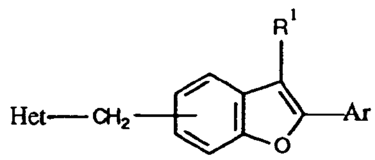

(Ia)

wherein $R^1$, Ar and Het are as defined in Claim 1 except that in the group Ar, $R^3$ represents a nitrile group, with a suitable azide, followed, if necessary, by the removal of any protecting groups where present; or

(D), where the substituent $R^3$ in the group Ar represents $-NHSO_2CF_3$, by reacting a compound of general formula (Ib)

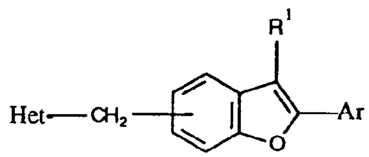

(Ib)

wherein $R^1$, Ar and Het are as defined in Claim 1 except that in the group Ar, $R^3$ represents an amino group, with trifluoromethanesulphonic anhydride or trifluoromethylsulphonyl chloride, followed, if necessary, by the removal of any protecting groups where present; or

(E), where Het represents the group (b), by treating a compound of formula (II) with a heterocycle of formula (VIa)

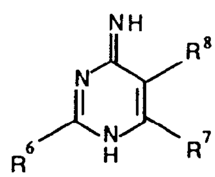

(VIa)

wherein $R^6$, $R^7$ and $R^8$ are as defined in Claim 1, followed by hydrolysis of the amine using aqueous hydroxide to give the pyrimidinone, followed, if necessary, by the removal of any protecting groups where present; or

(F), where Het represents the group (a), by treating a compound of formula (V)

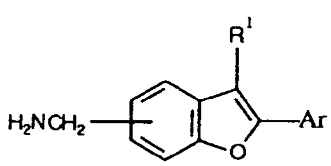

(V)

wherein $R^1$ and Ar are as defined in Claim 1 with a compound of formula (VIb)

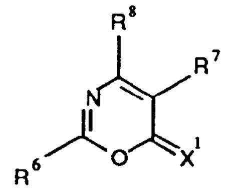

(VIb)

wherein $R^6$, $R^7$, $R^8$ and $X^1$ are as defined in Claim 1, optionally in the presence of a dehydrating agent, followed, if necessary, by the removal of any protecting groups where present; or
(G), where Het represents the group (d), by treating a compound of formula (Ic)

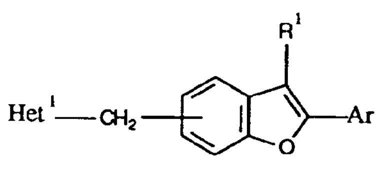

(Ic)

wherein $R^1$ and Ar are as defined in Claim 1 and $Het^1$ represents the group

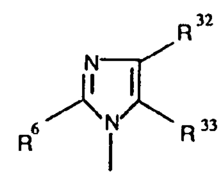

wherein $R^6$ is as defined in Claim 1 and $R^{32}$ and $R^{33}$ represent, respectively, $-CONR^{23}$ and $-NHR^{23}$; $-NHR^{23}$ and $-CONR^{23}$; $-CO_2R^{12}$ and $-NHR^{23}$; $-NHR^{23}$ and $-CO_2R^{12}$; $-CO_2R^{12}$ and halogen; halogen and $-CO_2R^{12}$; -SH and $-CO_2R^{12}$; $-CO_2R^{12}$ and -SH, with one of the compounds of general formula (VIIa) to (VIIe)

(a) 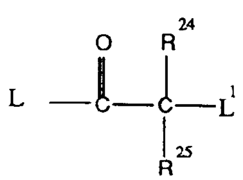 ,

(b) 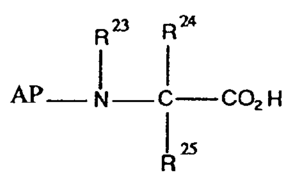 ,

(c) 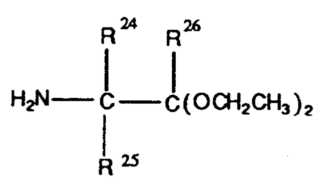 ,

(d) 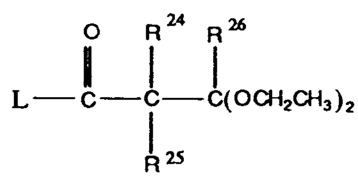 ,

(e) 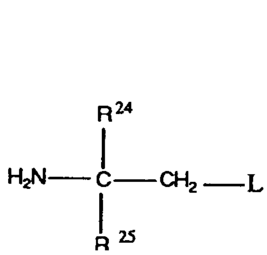

(VII)

wherein $R^{23}$, $R^{24}$, $R^{25}$ and $R^{26}$ are as defined in Claim 1, L and $L^1$ are leaving groups and AP is a suitable amino-protecting group, followed, if necessary, by the removal of any protecting group where present; or

(H), where Het represents the group (e), by treating a compound of formula (Id)

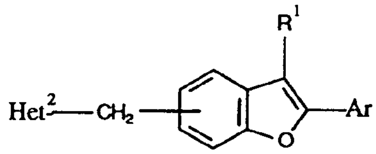

(Id)

wherein $R^1$ and Ar are as defined in Claim 1 and $Het^2$ represents the group

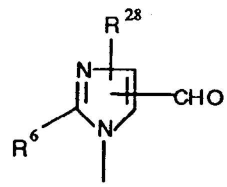

wherein $R^6$ and $R^{28}$ are as defined in Claim 1, with a compound of general formula (VIIIa) or (VIIb)

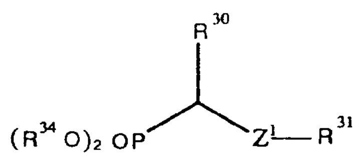

(VIIIa)

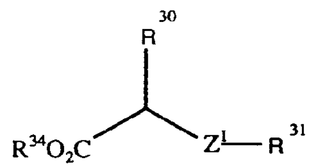

(VIIIb)

wherein $R^{30}$, $R^{31}$ and $Z^1$ are as defined in Claim 1 and $R^{34}$ is a $C_{1-4}$alkyl group, followed, if necessary, by the removal of any protecting groups where present;
and when the compound of general formula (I) is obtained as a mixture of enantiomers optionally resolving the mixture to obtain the desired enantiomer;
and/or, if desired, converting the resulting compound of general formula (I) or a salt thereof into a physiologically acceptable salt, solvate or metabolically labile ester thereof.

**19.** A pharmaceutical composition comprising at least one compound of general formula (I) as defined in any one of Claims 1 to 17 or a physiologically acceptable salt, solvate or metabolically labile ester thereof, together with at least one physiologically acceptable carrier or excipient.

**20.** A compound of general formula (I) as claimed in any one of Claims 1 to 17 or a physiologically acceptable salt, solvate or metabolically labile ester thereof for use in therapy, for example,
(i) for use in the treatment or prophylaxis of hypertension; or
(ii) for use in the treatment or prophylaxis of congestive heart failure, acute or chronic heart failure, aortic or cardiac insufficiency, post-myocardial infarction, renal insufficiency and renal failure (for example, as a result of diabetic nephropathy, glomerular nephritis, scleroderma or renal crisis), proteinuria, Bartter's syndrome, secondary hyperaldosteronism, Reynaud's syndrome, cerebrovascular insufficiency, peripheral vascular disease, diabetic retinopathy, atherogenesis and for the improvement of vascular compliance; or
(iii) for use in the treatment or prophylaxis of cognitive disorders such as dementia (e.g. Alzheimer's disease) and other CNS disorders, such as anxiety disorders, schizophrenia, depression and alcohol or drug (e.g. cocaine) dependency;
(iv) for use in the treatment of conditions associated with excessive or unregulated angiotensin II activity: or
(v) for use in the treatment of a condition associated with activation of the Renin-Angiotensin System.

**21.** A compound of general formula (Ia)

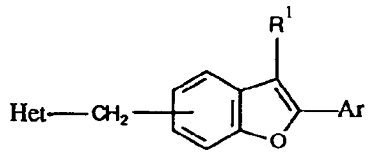

(Ia)

or an acid addition salt thereof
wherein
$R^1$, Ar and Het are as defined in Claim 1 except that in the group Ar, $R^3$ represents a nitrile group.

**22.** A compound of general formula (Ib)

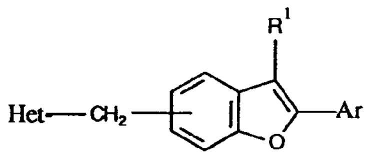

(Ib)

or an acid addition salt thereof
wherein
$R^1$, Ar and Het are as defined in Claim 1 except that in the group Ar, $R^3$ represents an amino group.

**Claims for the following Contracting States: ES, GR**

**1.** A process for the preparation of a compound of the general formula (I):

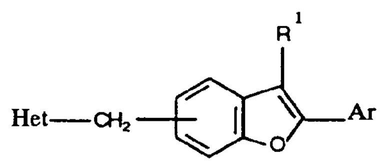

(I)

or a physiologically acceptable salt, solvate or metabolically labile ester thereof
wherein
$R^1$ represents a hydrogen atom or a halogen atom or a group selected from $C_{1-6}$alkyl, $C_{2-6}$alkenyl, fluoro$C_{1-6}$alkyl, $C_{1-6}$alkoxy ,-CHO, $-CO_2H$ or $-COR^2$;
Ar represents the group

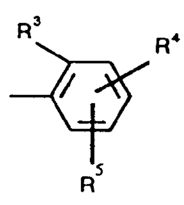

;

$R^2$ represents a group selected from $C_{1-6}$alkyl, $C_{2-6}$alkenyl, $C_{1-6}$alkoxy or the group $-NR^{14}R^{15}$;
$R^3$ represents a group selected from $-CO_2H$, $-NHSO_2CF_3$ or a C-linked tetrazolyl group;
$R^4$ and $R^5$ which may be the same or different each independently represent a hydrogen atom or a halogen atom or a $C_{1-6}$alkyl group;
Het represents a group selected from

(a) 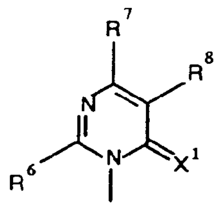 , (b) 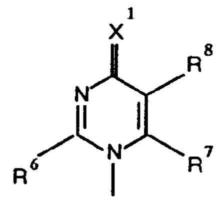

(c) 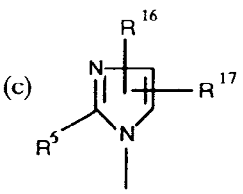 , (d) 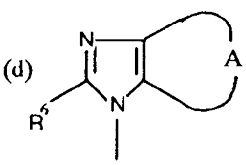 or

(e) 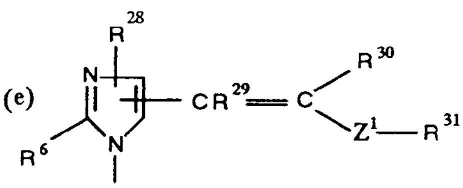

A represents, when read in a clockwise or anti-clockwise direction, a group selected from

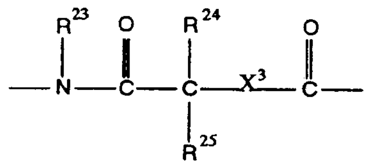 ,

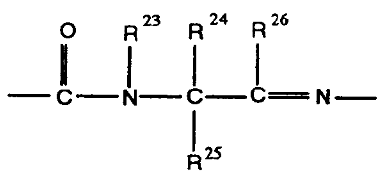 , 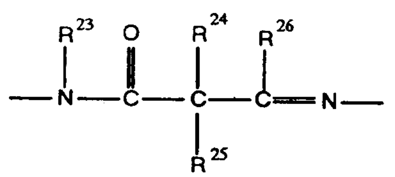 ,

or 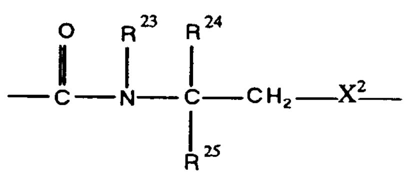 ;

$R^6$ represents a hydrogen atom or a group selected from $C_{1-6}$alkyl, $C_{2-6}$alkenyl, $C_{1-6}$alkylthio, $C_{1-6}$alkoxy, $C_{3-7}$cycloalkyl or $C_{3-7}$cycloalkyl$C_{1-4}$alkyl;

$R^7$ represents a hydrogen atom, a halogen atom or a group selected from $C_{1-6}$alkyl, $C_{2-6}$alkenyl, fluoro$C_{1-6}$alkyl $-(O)_m(CH_2)_nR^{10}$, $-(CH_2)_pCOR^{11}$ or $-(CH_2)_qNR^{12}COR^{13}$;

$R^8$ represents a hydrogen atom, a halogen atom or a group selected from $C_{1-6}$alkyl, $C_{2-6}$alkenyl, fluoro$C_{1-6}$alkyl, $-(CH_2)_rR^{10}$ or $-(CH_2)_pCOR^{11}$;

$R^9$ represents a hydrogen atom or a $C_{1-4}$alkyl group;

$R^{10}$ represents a group selected from hydroxy, $C_{1-6}$alkoxy, $C_{1-6}$alkylthio, phenyl of the group $-NR^{14}R^{15}$;

$R^{11}$ represents a hydrogen atom of a group selected from hydroxy, $C_{1-6}$alkyl, $C_{1-6}$alkoxy, phenyl, phenoxy or the group $-NR^{14}R^{15}$;

$R^{12}$ represents a hydrogen atom or a $C_{1-6}$alkyl group,

$R^{13}$ represents a hydrogen atom or a group selected from $C_{1-6}$alkyl, $C_{1-6}$alkoxy, phenyl, phenoxy or the group $-NR^{14}R^{15}$;

$R^{14}$ and $R^{15}$, which may be the same or different, each independently represent a hydrogen atom or a

$C_{1-4}$alkyl group or $-NR^{14}R^{15}$ forms a saturated heterocyclic ring which has 5 or 6 ring members and may optionally contain in the ring one oxygen atom;
$R^{16}$ represents a groups selected from $-NR^{18}COR^{19}$, $-NHCOR^{19}$, $-NR^{18}SO_2R^{19}$, $-NHSO_2(C_{1-4}$alkyl) or $-NR^9R^{20}$;
$R^{17}$ represents a group selected from $-CO_2R^{12}$, $-CONHSO_2R^{21}$ or $-CONR^9R^{20}$;
$R^{18}$ represents a group selected from $C_{1-6}$alkyl, aryl or arylmethyl, wherein 'aryl' represents a phenyl group optionally substituted by 1 or 2 substituents selected from a halogen atom or a group selected from $-NO_2$, $-CF_3$, $C_{1-4}$alkyl, $C_{1-4}$alkoxy, $-NR^9R^9$, $-NHCO_2(C_{1-4}$alkyl), $-CO_2R^9$ or hydroxy;
$R^{19}$ represents a group selected from aryl (as defined above), fluoro$C_{1-4}$alkyl, $-NR^9R^9$, $C_{3-5}$cycloalkyl, heteroalkyl,

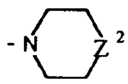

or $C_{1-4}$alkyl optionally substituted by aryl (as defined above), $-NR^9R^9$, $-NCO_2R^{22}$ or $-CO_2R^9$, wherein 'heteroaryl' represents a 5 or 6 membered aromatic ring containing from 1 to 3 heteroatoms selected from the group consisting of N, O or S, said aromatic ring being optionally substituted by 1 or 2 substituents selected from a halogen atom or a group selected from hydroxy, -SH, $C_{1-4}$alkyl, $C_{1-4}$alkoxy, $-CF_3$, $-NO_2$, $-CO_2R^9$ or $-NR^9R^9$;
$R^{20}$ represents a hydrogen atom or a group selected from aryl (as defined above) or $C_{1-6}$alkyl optionally substituted by a group selected from aryl (as defined above), hydroxy, $-CO_2R^9$ or $-NR^9R^9$;
$R^{21}$ represents a group selected from aryl (as defined above), heteroaryl (as defined above), $C_{3-7}$cycloalkyl, fluoro$C_{1-4}$alkyl or a $C_{1-4}$alkyl group optionally substituted by a halogen atom or a group selected from aryl (as defined above), heteroaryl (as defined above), hydroxy, -SH, $C_{1-4}$alkyl, $C_{1-4}$alkoxy, $C_{1-4}$alkylthio, $-CF_3$, $-NO_2$, $-CO_2R^9$, $-NR^9R^9$, $-PO_3H$, $-PO(OH)(C_{1-4}$alkoxy);
$R^{22}$ represents a hydrogen atom or a group selected from $C_{1-6}$alkyl, $C_{3-7}$cycloalkyl, aryl or arylmethyl (wherein 'aryl' is as defined above);
$R^{23}$ represents a hydrogen atom or a $C_{1-6}$alkyl group optionally substituted by a hydroxy group;
$R^{24}$ and $R^{25}$, which may be the same or different, each independently represent a hydrogen atom or a group selected from $-CO_2R^{12}$, a $C_{1-6}$alkyl, $C_{2-6}$alkenyl or $C_{2-6}$alkynyl group each of which is optionally substituted by a group selected from hydroxy, $C_{1-4}$alkoxy, $-NR^9R^9$, $-CONR^9R^9$, $-CO_2R^{27}$, $-OC(O)R^{27}$, guanidino or $C_{1-4}$alkylthio, a phenyl or phenyl-$C_{1-4}$alkyl group, each of which is optionally substituted by a halogen atom or a group selected from hydroxy, $C_{1-4}$alkyl or $C_{1-4}$alkoxy, or an imidazolyl-$C_{1-4}$alkyl or indolyl-$C_{1-4}$alkyl group;
$R^{26}$ and $R^{27}$, which may be the same or different, each independently represent a hydrogen atom or a group selected from $C_{1-6}$alkyl, phenyl or benzyl;
$R^{28}$ represents a hydrogen atom or a halogen atom or a group selected from $C_{1-6}$alkyl, $C_{2-6}$alkenyl or fluoro$C_{1-6}$alkyl;
$R^{29}$ and $R^{30}$, which may be the same or different, each independently represent a hydrogen atom or a group selected from $C_{1-6}$alkyl, $C_{3-7}$cycloalkyl, thienyl-Y-, pyrazolyl-Y-, imidazolyl-Y, thiazolyl-Y, furyl-Y-, pyrrolyl-Y-, triazolyl-Y-, oxazolyl-Y-, isoxazolyl-Y, pyridyl-Y-, phenyl-Y- or tetrazolyl-Y- wherein each aryl or heterocyclic ring is optionally substituted by a halogen atom or group selected from $C_{1-6}$alkyl, fluoro$C_{1-6}$alkyl, $C_{1-6}$alkoxy, hydroxy, $-NR^{27}R^{27}$, $-CO_2R^{27}$, $-CONR^{27}R^{27}$, $-SO_2NHR^{27}$ or $-SO_3H$;
$R^{31}$ represents $-COR^{11}$, a C-linked tetrazol-5-yl group or the group $-NHSO_2CF_3$;
$X^1$ represents an oxygen atom or sulphur atom or the group $-NR^9$-;
$X^2$ represents an oxygen or sulphur atom or the group $-N(R^{26})$-;
$X^3$ represents an oxygen atom or the group $-N(R^{23})$-;
Y represents a bond or an oxygen or sulphur atom, or a $C_{1-6}$alkyl linkage which is optionally substituted by phenyl or benzyl wherein each aryl ring is optionally substituted by a halogen atom or a group selected from $-NO_2$, $-CF_3$, $C_{1-4}$alkyl, $C_{1-4}$alkoxy, -CN or $CO_2R^{27}$;
$Z^1$ represents a bond or a methylene, ethylene or vinyl linkage, each being optionally substituted by a $C_{1-4}$alkyl group; and
$Z^2$ represents an oxygen or sulphur atom or a group selected from $-N(R^9)$-, -S(O)- or $-S(O)_2$-;
m represents zero or 1;

n represents zero or an integer from 1 to 4;
p represents zero or an integer from 1 to 4;
q represents an integer from 1 to 4; and
r represents an integer from 1 to 4.
provided that when m is 1, n is an integer from 1 to 4,
which comprises:

(A), where Het represents group (a), (c) or (e), treating a compound of general formula (II)

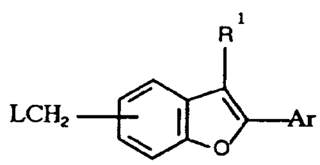

(II)

wherein L is a leaving group and $R^1$ and Ar are as defined in general formula (I), with a heterocycle. of formula (IIIa), (IIIb) or (IIIc)

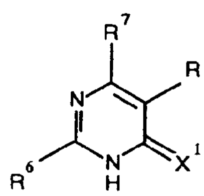

(IIIa)

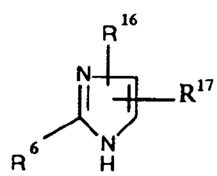

(IIIb)

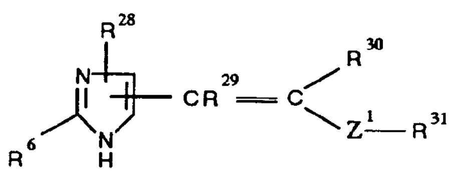

(IIIc)

wherein $R^6$, $R^7$, $R^8$, $R^{16}$, $R^{17}$, $R^{28}$, $R^{29}$, $R^{30}$, $R^{31}$, $X^1$ and $Z^1$ are as defined in general formula (I), followed, if necessary, by the removal of any protecting groups where present; or

(B) deprotecting a protected intermediate of general formula (IV)

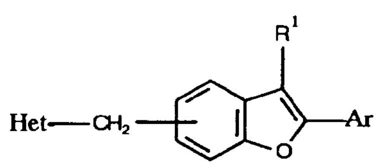

(IV)

wherein $R^1$, Ar and Het are as defined in general formula (I), except that at least one reactive group is blocked by a protecting group; or

(C), where the substituent $R^3$ in the group Ar represents a C-linked tetrazolyl group, by reacting a compound of general formula (Ia)

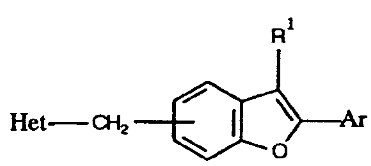

(Ia)

wherein $R^1$, Ar and Het are as defined in general formula (I) except that in the group Ar, $R^3$ represents a nitrile group, with a suitable azide, followed, if necessary, by the removal of any protecting groups where present; or

(D), where the substituent $R^3$ in the group Ar represents $-NHSO_2CF_3$, by reacting a compound of general formula (Ib)

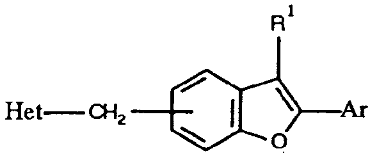

(Ib)

wherein $R^1$, Ar and Het are as defined in general formula (I) except that in the group Ar, $R^3$ represents an amino group, with trifluoromethanesulphonic anhydride or trifluoromethylsulphonyl chloride, followed, if necessary, by the removal of any protecting groups where present; or

(E), where Het represents the group (b), by treating a compound of formula (II) with a heterocycle of formula (VIa)

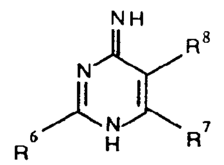

(VIa)

wherein $R^6$, $R^7$ and $R^8$ are as defined in general formula (I), followed by hydrolysis of the amine using aqueous hydroxide to give the pyrimidinone, followed, if necessary, by the removal of any protecting groups where present; or

(F), where Het represents the group (a), by treating a compound of formula (V)

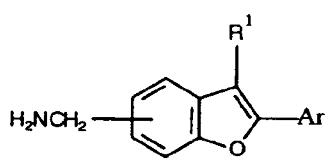

(V)

wherein $R^1$ and Ar are as defined in general formula (I) with a compound of formula (VIb)

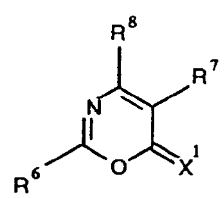

(VIb)

wherein $R^6$, $R^7$, $R^8$ and $X^1$ are as defined in general formula (I), optionally in the presence of a dehydrating agent, followed, if necessary, by the removal of any protecting groups where present; or

(G), where Het represents the group (d), by treating a compound of formula (Ic)

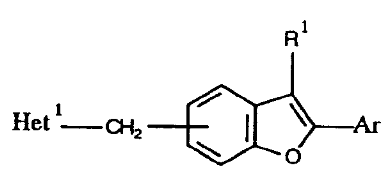

(Ic)

wherein $R^1$ and Ar are as defined in general formula (I) and $Het^1$ represents the group

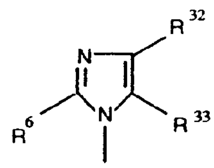

wherein $R^6$ is as defined in general formula (I) and $R^{32}$ and $R^{33}$ represent, respectively, $-CONR^{23}$ and $-NHR^{23}$; $-NHR^{23}$ and $-CONR^{23}$; $-CO_2R^{12}$ and $-NHR^{23}$; $-NHR^{23}$ and $-CO_2R^{12}$; $-CO_2R^{12}$ and halogen; halogen and $-CO_2R^{12}$; -SH and $-CO_2R^{12}$; $-CO_2R^{12}$ and -SH, with one of the compounds of general formula (VIIa) to (VIIe)

(a) 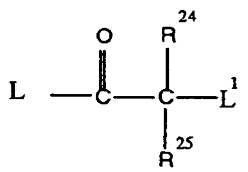 , (b) 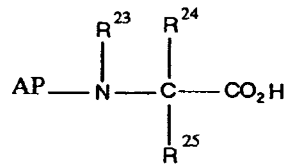 ,

(c) 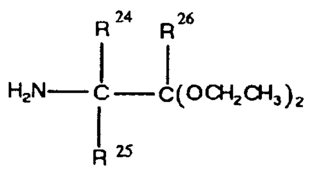 , (d) 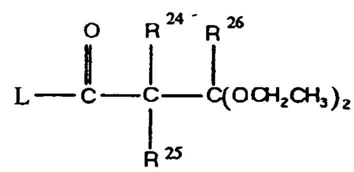 ,

(e) 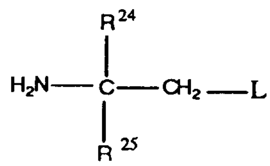 (VII)

wherein $R^{23}$, $R^{24}$, $R^{25}$ and $R^{26}$ are as defined in general formula (I), L and $L^1$ are leaving groups and AP is a suitable amino-protecting group, followed, if necessary, by the removal of any protecting group where present; or

(H), where Het represents the group (e), by treating a compound of formula (Id)

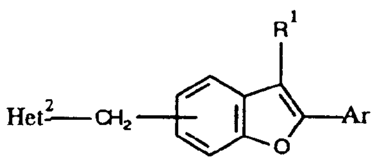 (Id)

wherein $R^1$ and Ar are as defined in general formula (I) and $Het^2$ represents the group

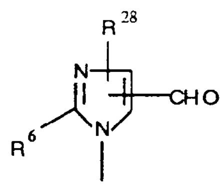

wherein $R^6$ and $R^{28}$ are as defined in general formula (I), with a compound of general formula (VIIIa) or (VIIb)

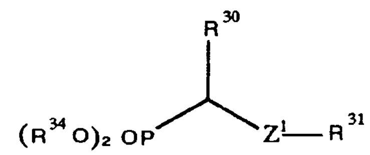 (VIIIa) 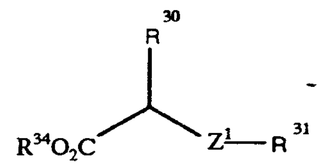 (VIIIb)

wherein $R^{30}$, $R^{31}$ and $Z^1$ are as defined in general formula (I) and $R^{34}$ is a $C_{1-4}$alkyl group, followed, if necessary, by the removal of any protecting groups where present;
and when the compound of general formula (I) is obtained as a mixture of enantiomers optionally resolving the mixture to obtain the desired enantiomer;
and/or, if desired, converting the resulting compound of general formula (I) or a salt thereof into a physiologically acceptable salt, solvate or metabolically labile ester thereof.

2. A process as claimed in Claim 1 for the preparation of a compound of general formula (I) or a physiologically acceptable salt, solvate or metabolically labile ester thereof
wherein
Het represents either group (a) or group (b);
$R^6$ represents a hydrogen atom, a $C_{1-5}$alkyl group, particularly a $C_{2-4}$alkyl group, especially an ethyl, n-propyl or n-butyl group, a $C_{3-5}$alkyenyl group, a $C_{3-5}$cycloalkyl group, especially a cyclopropyl or cyclobutyl group, or a $C_{3-5}$cycloalkyl$C_{1-2}$alkyl group, especially a cyclopropylmethyl group;
$R^7$ represents a hydrogen atom a $C_{1-4}$alkyl, especially a methyl group, $-(O)_m(CH_2)_nR^{10}$ or $-(CH_2)_pCOR^{11}$;
$R^8$ represents a hydrogen atom, a halogen, especially chlorine, atom, a $C_{1-4}$alkyl, especially a methyl, group, $-(CH_2)_rR^{10}$ or $-(CH_2)_pCOR^{11}$;
$R^{10}$ represents hydroxy, a $C_{1-4}$alkoxy, especially a methoxy, group, a $C_{1-4}$alkylthio, especially a methylthio group, phenyl or $-NR^{14}R^{15}$;
$R^{11}$ represents a hydroxy, a $C_{1-4}$alkoxy, especially a methoxy or ethoxy, group or $-NR^{14}R^{15}$;
$R^{14}$ and $R^{15}$, which may be the same or different, each independently represent a hydrogen atom or a $C_{1-4}$alkyl, especially a methyl or, ethyl, group or $-NR^{14}R^{15}$ forms a morpholino group;
$X^1$ represents an oxygen atom;
m represents zero or 1;
n represents zero, 1 or 2;
p represents zero, 1 or 2, especially zero or 1; and
r represents 1 or 2.

3. A process as claimed in either Claim 1 or Claim 2 for the preparation of a compound of general formula (I) or a physiologically acceptable salt, solvate or metabolically labile ester thereof
wherein
Het represents either group (a) or group (b);
$R^6$ represents a $C_{2-4}$alkyl, especially an ethyl or n-propyl, group;
$R^7$ represents a hydrogen atom, a $C_{1-4}$alkyl, especially a methyl, group, or $-(CH_2)_pCOR^{11}$;
$R^8$ represents a hydrogen atom, a halogen, especially a chlorine, atom, or a $C_{1-4}$alkyl, especially a methyl, group;
$R^{11}$ represents hydroxy or $-NR^{14}R^{15}$;
$R^{14}$ and $R^{15}$ represents a hydrogen atom or a $C_{1-4}$alkyl, especially a methyl or ethyl, group;
$X^1$ represents an oxygen atom; and
p represents zero.

4. A process as claimed in Claim 1 for the preparation of a compound of general formula (I) or a physiologically acceptable salt, solvate or metabolically labile ester thereof
wherein
Het represents the group (c);
$R^9$ represents a hydrogen atom;
$R^{16}$ represents the group $-NR^9R^{20}$; and
$R^{20}$ represents a hydrogen atom or a $C_{1-6}$alkyl group optionally substituted by a hydroxy group.

5. A process as claimed in either Claim 1 or Claim 4 for the preparation of a compound of general formula

(I) or a physiologically acceptable salt, solvate or metabolically labile ester thereof
wherein
Het represents the group (c);
$R^9$ represents a hydrogen atom;
$R^{12}$ represents a hydrogen atom or a $C_{1-4}$alkyl group;
$R^{17}$ represents the group $-CO_2R^{12}$ or $-CONR^9R^{20}$; and
$R^{20}$ represents a hydrogen atom or a $C_{1-6}$alkyl group optionally substituted by a hydroxy group.

6. A process as claimed in Claim 1 for the preparation of a compound of general formula (I) or a physiologically acceptable salt, solvate or metabolically labile ester thereof
wherein
Het represents the group (d);
A represents the group

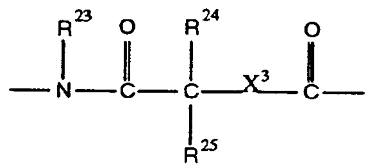

or

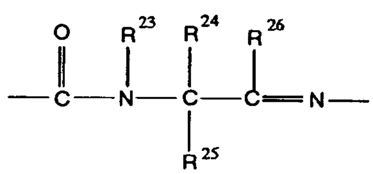

;

$R^{23}$ represents a hydrogen atom;
$R^{24}$ represents a hydrogen atom;
$R^{25}$ represents a hydrogen atom, a $C_{1-6}$alkyl group, optionally substituted by a group selected from hydroxy, amino, guanidino, $C_{1-4}$alkylthio, $-CONR^9R^9$ (wherein $-NR^9R^9$ represents an $NH_2$ group), $-CO_2R^{27}$ (wherein $R^{27}$ represents a hydrogen atom or a $C_{1-4}$alkyl group or $-OC(O)R^{27}$ (wherein $R^{27}$ represents a hydrogen atom or a $C_{1-4}$alkyl group), or a phenyl, benzyl, 4-hydroxybenzyl, 4-imidazolylmethyl or 3-indolylmethyl group;
$R^{26}$ represents a hydrogen atom; and
$X^3$ represents $-N(R^{23})-$.

7. A process as claimed in Claim 1 for the preparation of a compound of general formula (I) wherein Het represents the group (e) and $R^{28}$ represents a hydrogen atom, a halogen atom, particularly a chlorine atom, or a $C_{1-4}$alkyl group, particularly a methyl group.

8. A process as claimed in either Claim 1 or Claim 7 for the preparation of a compound of general formula (I) wherein Het represents the group (e) and $R^{29}$ represents a hydrogen atom or a $C_{1-4}$alkyl group, particularly a methyl or ethyl group.

9. A process as claimed in any one of Claims 1, 7 and 8 for the preparation of a compound of general formula (I) wherein Het represents the group (e) and $R^{30}$ represents a group selected from thienyl-Y-, imidazolyl-Y-, furyl-Y-, Pyridyl-Y-, or phenyl-Y- wherein each aryl or heterocyclic group is optionally substituted by a hydroxy group or a $C_{1-4}$alkyl, particularly a methyl, group or $C_{1-4}$alkoxy, particularly a methoxy, group, and Y represents a bond or a methylene linkage.

10. A process as claimed in any one of Claims 1, 7, 8 and 9 for the preparation of a compound of general formula (I) wherein Het represents the group (e) and $R^{31}$ represents the group $COR^{11}$ wherein $R^{11}$ represents a hydroxy or $C_{1-4}$alkoxy, particularly methoxy or ethoxy, group or the group $-NR^{14}R^{15}$, particularly wherein $R^{14}$ and $R^{15}$ each independently represents a hydrogen atom or a $C_{1-4}$alkyl, particularly a methyl or ethyl, group, or $R^{31}$ represents a C-linked tetrazol-5-yl group, and $Z^1$ represents a bond or methylene linkage.

11. A process as claimed in any one of Claims 1 to 10 for the preparation of a compound of general formula (I) wherein $R^6$ represents a hydrogen atom or a group selected from $C_{1-5}$alkyl, $C_{3-5}$alkenyl, $C_{1-5}$alkoxy, $C_{3-5}$cycloalkyl or $C_{3-5}$cycloalkyl$C_{1-2}$alkyl, particularly a $C_{2-4}$alkyl group, for example, an ethyl, n-propyl or n-butyl group.

12. A process as claimed in any one of Claims 1 to 11 for the preparation of a compound of general formula

(I) wherein $R^1$ represents a hydrogen atom or a halogen atom or a group selected from $C_{1-6}$alkyl, fluoro$C_{1-6}$alkyl or $C_{1-6}$alkoxy, particularly a bromine atom.

**13.** A process as claimed in any one of Claims 1 to 12 for the preparation of a compound of general formula (I) wherein the group Het-$CH_2$- is attached at the 5- or 6-position on the benzofuran ring, particularly at the 5-position on the benzofuran ring.

**14.** A process as claimed in any one of Claims 1 to 13 for the preparation of a compound of general formula (I) wherein $R^3$ represents the group $-NHSO_2CF_3$ or a C-linked tetrazolyl group or the group $-NHSO_2CF_3$.

**15.** A process as claimed in any one of Claims 1 to 14 for the preparation of a compound of general formula (I) wherein $R^4$ and $R^5$ each independently represent a hydrogen atom or a halogen atom, particularly, $R^4$ and $R^5$ each represent a hydrogen atom.

**16.** A process as claimed in Claim 1 for the preparation of a compound of general formula (I) or a physiologically acceptable salt, solvate or metabolically labile ester thereof
wherein
$R^1$ represents a halogen atom;
Ar represents the group

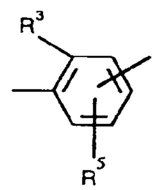 ;

$R^3$ represents the group $-NHSO_2CF_3$ or a C-linked tetrazolyl group;
$R^4$ and $R^5$ each independently represent a hydrogen atom;
Het represents a group selected from

(a) 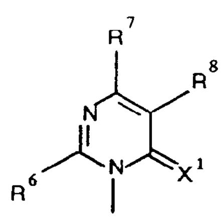 , (c) 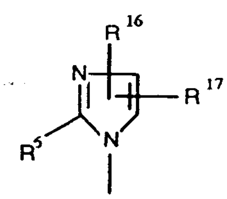 ,

(d) 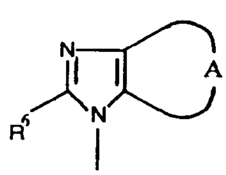 or (e) 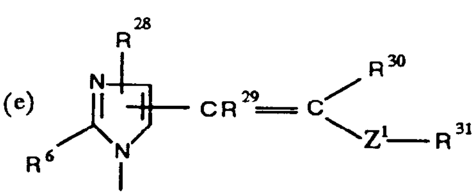

A represents, when read in a clockwise direction, the group

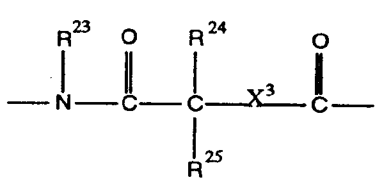 ;

$R^6$ represents a $C_{1-6}$alkyl group;
$R^7$ represents a hydrogen atom or a $C_{1-6}$ alkyl group;
$R^8$ represents a hydrogen atom or a $C_{1-6}$alkyl group;
$R^{16}$ represents a group selected from $-NR^{18}COR^{19}$ or $-NR^9R^{20}$ (wherein $R^9$ represents a hydrogen atom or a $C_{1-4}$alkyl group and $R^{20}$ represents a hydrogen atom or a $C_{1-6}$alkyl group);
$R^{17}$ represents the group $-CO_2R^{12}$ (wherein $R^{12}$ represents a hydrogen atom or a $C_{1-6}$alkyl group);
$R^{23}$ represents a hydrogen atom or $C_{1-6}$alkyl group;
$R^{24}$ and $R^{25}$ each independently represent a hydrogen atom;
$R^{28}$ represents a hydrogen atom;
$R^{29}$ represents a hydrogen atom;
$R^{30}$ represents the group thienyl-Y-;
$R^{31}$ represents the group $-COR^{11}$ (wherein $R^{11}$ represents a hydrogen atom or a group selected from $C_{1-6}$alkyl or $-NH_2$);
$X^1$ represents an oxygen atom;
$X^3$ represents the group $-N(R^{23})-$;
Y represents a bond; and
$Z^1$ represents a bond.

**17.** A process as claimed in Claim 1 for the preparation of a compound selected from
3-[[3-bromo-2-[2-(1H-tetrazol-5-yl)phenyl]-5-benzofuranyl]methyl]-2-ethyl-4(3H)-pyrimidinone;
3-[[3-bromo-2-[2-(1H-tetrazol-5-yl)phenyl]-5-benzofuranyl]methyl]-2-ethyl-6-methyl-4(3H)-pyrimidinone;
3-[[3-bromo-2-[2-(1H-tetrazol-5-yl)phenyl]-5-benzofuranyl]methyl]-2-ethyl-5-methyl-4(3H)-pyrimidinone;
3-[[3-bromo-2-[2-(1H-tetrazol-5-yl)phenyl]-5-benzofuranyl]methyl]-2-ethyl-5,6-dimethyl-4(3H)-pyrimidinone;
3-[[3-bromo-2-[2-[[(trifluoromethyl)sulphonyl]amino]phenyl]-5-benzofuranyl]methyl]-2-ethyl-4(3H)-pyrimidinone;
3-[[3-bromo-2-[2-[[(trifluoromethyl)sulphonyl]amino]phenyl]-5-benzofuranyl]methyl]-2-ethyl-6-methyl-4(3H)-pyrimidinone;
3-[[3-bromo-2-[2-[[(trifluoromethyl)sulphonyl]amino]phenyl]-5-benzofuranyl]methyl]-2-ethyl-5-methyl-4(3H)-pyrimidinone;
3-[[3-bromo-2-[2-[[(trifluoromethyl)sulphonyl]amino]phenyl]-5-benzofuranyl]methyl]-2-ethyl-5,6-dimethyl-4(3H)-pyrimidinone;
3-[[3-bromo-2-[2-(1H-tetrazol-5-yl)phenyl]-5-benzofuranyl]methyl]-2-propyl-4(3H)-pyrimidinone;
3-[[3-bromo-2-[2-(1H-tetrazol-5-yl)phenyl]-5-benzofuranyl]methyl]-6-methyl-2-propyl-4(3H)-pyrimidinone;
3-[[3-bromo-2-[2-(1H-tetrazol-5-yl)phenyl]-5-benzofuranyl]methyl]-5-methyl-2-propyl-4(3H)-pyrimidinone;
3-[[3-bromo-2-[2-(1H-tetrazol-5-yl)phenyl]-5-benzofuranyl]methyl]-5,6-dimethyl-2-propyl-4(3H)-pyrimidinone;
3-[[3-bromo-2-[2-[[(trifluoromethyl)sulphonyl]amino]phenyl]-5-benzofuranyl]methyl]-2-propyl-4(3H)-pyrimidinone;
3-[[3-bromo-2-[2-[[(trifluoromethyl)sulphonyl]amino]phenyl]-5-benzofuranyl]methyl]-6-methyl-2-propyl-4(3H)-pyrimidinone;
3-[[3-bromo-2-[2-[[(trifluoromethyl)sulphonyl]amino]phenyl]-5-benzofuranyl]methyl]-5-methyl-2-propyl-4(3H)-pyrimidinone;
3-[[3-bromo-2-[2-[[(trifluoromethyl)sulphonyl]amino]phenyl]-5-benzofuranyl]methyl]-5,6-dimethyl-2-propyl-4(3H)-pyrimidinone;
1-[[3-bromo-2-[2-(1H-tetrazol-5-yl)phenyl]-5-benzofuranyl]methyl]-2-ethyl-4(1H)-pyrimidinone;
1-[[3-bromo-2-[2-(1H-tetrazol-5-yl)phenyl]-5-benzofuranyl]methyl]-2-propyl-4(1H)-pyrimidinone;
1-[[3-bromo-2-[2-[[(trifluoromethyl)sulphonyl]amino]phenyl]-5-benzofuranyl]methyl]-2-ethyl-4(1H)-pyrimidinone;
1-[[3-bromo-2-[2-[[(trifluoromethyl)sulphonyl]amino]phenyl]-5-benzofuranyl]methyl]-2-propyl-4(1H)-pyrimidinone;
3-[[3-bromo-2-[2-(1H-tetrazol-5-yl)phenyl]-5-benzofuranyl]methyl]-2-ethyl-3,6-dihydro-6-oxo-4-pyrimidinecarboxylic acid;

3-[[3-bromo-2-[2-(1H-tetrazol-5-yl)phenyl]-5-benzofuranyl]methyl]-3,6-dihydro-6-oxo-2-propyl-4(3H)-pyrimidinecarboxylic acid;

3-[[3-bromo-2-[2-(1H-tetrazol-5-yl)phenyl]-5-benzofuranyl]methyl]-5-chloro-2-ethyl-3,6-dihydro-6-oxo-4-pyrimidinecarboxylic acid;

3-[[3-bromo-2-[2-(1H-tetrazol-5-yl)phenyl]-5-benzofuranyl]methyl]-5-chloro-3,6-dihydro-6-oxo-2-propyl-4(3H)-pyrimidinecarboxylic acid;

3-[[3-bromo-2-[2-[[(trifluoromethyl)sulphonyl]amino]phenyl]-5-benzofuranyl]methyl]-2-ethyl-3,6-dihydro-6-oxo-4-pyrimidinecarboxamide;

3-[[3-bromo-2-[2-[[(trifluoromethyl)sulphonyl]amino]phenyl]-5-benzofuranyl]methyl]-2-ethyl-3,6-dihydro-N-methyl-6-oxo-4-pyrimidinecarboxiamide;

3-[[3-bromo-2-[2-[[(trifluoromethyl)sulphonyl]amino]phenyl]-5-benzofuranyl]methyl]-N,2-diethyl-3,6-dihydro-6-oxo-4-pyrimidinecarboxamide;

3-[[3-bromo-2-[2-[[(trifluoromethyl)sulphonyl]amino]phenyl]-5-benzofuranyl]methyl]-3,6-dihydro-6-oxo-2-propyl-4-pyrimidinecarboxamide;

3-[[3-bromo-2-[2-[[(trifluoromethyl)sulphonyl]amino]phenyl]-5-benzofuranyl]methyl]-3,6-dihydro-N-methyl-6-oxo-2-propyl-4-pyrimidinecarboxamide;

3-[[3-bromo-2-[2-[[(trifluoromethyl)sulphonyl]amino]phenyl]-5-benzofuranyl]methyl]-N-ethyl-3,6-dihydro-6-oxo-2-propyl-4-pyrimidinecarboxamide;

3-[[3-bromo-2-[2-[[(trifluoromethyl)sulphonyl]amino]phenyl]-5-benzofuranyl]methyl]-5-chloro-2-ethyl-3,6-dihydro-6-oxo-4-pyrimidinecarboxamide;

3-[[3-bromo-2-[2-[[(trifluoromethyl)sulphonyl]amino]phenyl]-5-benzofuranyl]methyl]-5-chloro-2-ethyl-3,6-dihydro-N-methyl-6-oxo-4-pyrimidinecarboxamide;

3-[[3-bromo-2-[2-[[(trifluoromethyl)sulphonyl]amino]phenyl]-5-benzofuranyl]methyl]-5-chloro-N,2-diethyl-3,6-dihydro-6-oxo-4-pyrimidinecarboxamide;

3-[[3-bromo-2-[2-[[(trifluoromethyl)sulphonyl]amino]phenyl]-5-benzofuranyl]methyl]-5-chloro-3,6-dihydro-6-oxo-2-propyl-4-pyrimidinecarboxamide;

3-[[3-bromo-2-[2-[[(trifluoromethyl)sulphonyl]amino]phenyl]-5-benzofuranyl]methyl]-5-chloro-3,6-dihydro-N-methyl-6-oxo-2-propyl-4-pyrimidinecarboxamide;

3-[[3-bromo-2-[2-[[(trifluoromethyl)sulphonyl]amino]phenyl]-5-benzofuranyl]methyl]-5-chloro-N-ethyl-3,6-dihydro-6-oxo-2-propyl-4-pyrimidinecarboxamide;

1-[[3-bromo-2-[2-(1H-tetrazol-1-yl)phenyl]-5-benzofuranyl]methyl]-2- ethyl-1,4,6,7-tetrahydro-4-methyl-5,8-dioxoimidazo[4,5-e][1,4]diazepine-6-carboxylic acid;

1-[[3-bromo-2-[2-(1H-tetrazol-1-yl)phenyl]-5-benzofuranyl]methyl]-1,4,6,7-tetrahydro-4-methyl-5,8-dioxo-2-propylimidazo[4,5-e][1,4]diazepine-6-carboxylic acid;

1-[[3-bromo-2-[2-[[(trifluoromethyl)sulphonyl]amino]phenyl]-5-benzofuranyl]methyl]-2-ethyl-1,4,6,7-tetrahydro-4-methyl-5,8-dioxoimidazo[4,5-e][1,4]diazepine-6-carboxylic acid;

1-[[3-bromo-2-[2-[[(trifluoromethyl)sulphonyl]amino]phenyl]-5-benzofuranyl]methyl]-1,4,6,7-tetrahydro-4-methyl-5,8-dioxo-2-propylimidazo[4,5-e][1,4]diazepine-6-carboxylic acid;

1-[[3-bromo-2-[2-(1H-tetrazol-1-yl)phenyl]-5-benzofuranyl]methyl]-2-ethyl-1,4,6,7-tetrahydro-4-methylimidazo[4,5-e][1,4]dia2epine-5,8-dione;

1-[[3-bromo-2-[2-(1H-tetrazol-1-yl)phenyl]-5-benzofuranyl]methyl]-1,4,6,7-tetrahydro-4-methyl-2-propylimidazo[4,5-e][1,4]diazepine-5,8-dione;

N-[2-[3-bromo-5-[(2-ethyl-1,4,6,7-tetrahydro-4-methyl-5,8-dioxoimidazo[4,5-e][1,4]diazepin-1-yl)methyl]-2-benzofuranyl]phenyl]-2,2,2-trifluoromethanesulphonamide;

N-[2-[3-bromo-5-[(1,4,6,7-tetrahydro-4-methyl-5,8-dioxo-2-propylimidazo[4,5-e][1,4]diazepin-1-yl)methyl]-2-benzofuranyl]phenyl]-2,2,2-trifluoromethanesulphonamide;

4-amino-1-[[3-bromo-2-[2-[[(trifluoromethyl)sulphonyl]amino]phenyl]-5-benzofuranyl]methyl]-2-ethyl-1H-imidazole-5-carboxiamide;

4-amino-1-[[3-bromo-2-[2-[[(trifluoromethyl)sulphonyl]amino]phenyl]-5-benzofuranyl]methyl]-2-propyl-1H-imidazole-5-carboxamide;

1-[[3-bromo-2-[2-[[(trifluoromethyl)sulphonyl]amino]phenyl]-5-benzofuranyl]methyl]-2-ethyl-4-(methylamino)-1H-imidazole-5-carboxiamide;

1-[[3-bromo-2-[2-[[(trifluoromethyl)sulphonyl]amino]phenyl]-5-benzofuranyl]methyl]-4-(methylamino)-2-propyl-1H-imidazole-5-carboxamide;

1-[[3-bromo-2-[2-[[(trifluoromethyl)sulphonyl]amino]phenyl]-5-benzofuranyl]methyl]-4-(dimethylamino)-2-ethyl-1H-imidazole-5-carboxamide;

1-[[3-bromo-2-[2-[[(trifluoromethyl)sulphonyl]amino]phenyl]-5-benzofuranyl]methyl]-4-(dimethylamino)-2-propyl-1H-imidazole-5-carboxamide;

4-amino-1-[[3-bromo-2-[2-[[(trifluoromethyl)sulphonyl]amino]phenyl]-5-benzofuranyl]methyl]-2-ethyl-N-methyl-1H-imidazole-5-carboxamide;

4-amino-1-[[3-bromo-2-[2-[[(trifluoromethyl)sulphonyl]amino]phenyl]-5-benzofuranyl]methyl]-N,2-diethyl-1H-imidazole-5-carboxamide;

4-amino-1-[[3-bromo-2-[2-[[(trifluoromethyl)sulphonyl]amino]phenyl]-5-benzofuranyl]methyl]-N-ethyl-2-propyl-1H-imidazole-5-carboxamide;

1-[[3-bromo-2-[2-[[(trifluoromethyl)sulphonyl]amino]phenyl]-5-benzofuranyl]methyl]-N,2-diethyl-4-(methylamino)-1H-imidazole-5-carboxamide;

1-[[3-bromo-2-[2-[[(trifluoromethyl)sulphonyl]amino]phenyl]-5-benzofuranyl]methyl]-N-ethyl-4-(methylamino)-2-propyl-1H-imidazole-5-carboxamide;

1-[[3-bromo-2-[2-[[(trifluoromethyl)sulphonyl]amino]phenyl]-5-benzofuranyl]methyl]-4-(dimethylamino)-N,2-diethyl-1H-imidazole-5-carboxamide;

1-[[3-bromo-2-[2-[[(trifluoromethyl)sulphonyl]amino]phenyl]-5-benzofuranyl]methyl]-4-(dimethylamino)-N-ethyl-2-propyl-1H-imidazole-5-carboxamide;

4-amino-1-[[3-bromo-2-[2-[[(trifluoromethyl)sulphonyl]amino]phenyl]-5-benzofuranyl]methyl]-N-methyl-2-propyl-1H-imidazole-5-carboxamide;

1-[[3-bromo-2-[2-[[(trifluoromethyl)sulphonyl]amino]phenyl]-5-benzofuranyl]methyl]-2-ethyl-N-methyl-4-(methylamino)-1H-imidazole-5-carboxamide;

1-[[3-bromo-2-[2-[[(trifluoromethyl)sulphonyl]amino]phenyl]-5-benzofuranyl]methyl]-N-methyl-4-(methylamino)-2-propyl-1H-imidazole-5-carboxamide;

1-[[3-bromo-2-[2-[[(trifluoromethyl)sulphonyl]amino]phenyl]-5-benzofuranyl]methyl]-4-(dimethylamino)-2-ethyl-N-methyl-1H-imidazole-5-carboxamide;

1-[[3-bromo-2-[2-[[(trifluoromethyl)sulphonyl]amino]phenyl]-5-benzofuranyl]methyl]-4-(dimethylamino)-N-methyl-2-propyl-1H-imidazole-5-carboxamide;

(E)-α-[[1-[[3-bromo-2-[2-(1H-tetrazol-5-yl)phenyl]-5-benzofuranyl]methyl]-2-propyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-4-chloro-2-propyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-4-methyl-2-propyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-ethyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-4-chloro-2-ethyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-ethyl-4-methyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-butyl-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-butyl-4-chloro-1H-imidazol-5-yl]methylene]-2-thiophenepropanoic acid;

(E)-α-[[1-[[3-bromo-2-[2-(1H-tetrazol-5-yl)phenyl]-5-benzofuranyl]methyl]-2-butyl-4-methyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-2-propyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-4-chloro-2-propyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-4-methyl-2-propyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-2-ethyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-4-chloro-2-ethyl-1H-imidazol-5-yl]methylene]-2-thiophenepropanoic acid;

(E)-α-[[1-[[3-bromo-2-[2-[[(trifluoromethyl)sulphonyl]-amino]phenyl]-5-benzofuranyl]methyl]-2-ethyl-4-methyl-1H-imidazol-5-yl]methylene]-2-thiophenepropanoic acid;

(E)-α-[[1-[[3-bromo-2-[2-[[(trifluoromethyl)sulphonyl]amino]phenyl]-5-benzofuranyl]methyl]-2-propyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-4-chloro-2-propyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-4-methyl-2-propyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-2-ethyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-4-chloro-2-ethyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-2-ethyl-4-methyl-1H-imidazol-5-yl]methylene]-2-thiophenepropanamide;

(E)-α-[[1-[[3-bromo-2-[2-[[(trifluoromethyl)sulphonyl]amino]phenyl]-5-benzofuranyl]methyl]-2-propyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-4-chloro-2-propyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-4-methyl-2-propyl-1H-imidazol-5-yl]methylene]-N-methyl--2-thiophenepropanamide;

(E)-α-[[1-[[3-bromo-2-[2-[[(trifluoromethyl)sulphonyl]amino]phenyl]-5-benzofuranyl]methyl]-2-ethyl-1H-imidazol-5yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-4-chloro-2-ethyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-2-ethyl-4-methyl-1H-imidazol-5-yl]methylene]-N-methyl-2-thiophenepropanamide;

(E)-α-[[1-[[3-bromo-2-[2-[[(trifluoromethyl)sulphonyl]amino]phenyl]-5-benzofuranyl]methyl]-2-propyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-4-chloro-2-propyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-4-methyl-2-propyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-2-ethyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-4-chloro-2-ethyl-1H-imidazol-5-yl]methylene]-N-ethyl-2-thiophenepropanamide;

(E)-α-[[1-[[3-bromo-2-[2-[[(trifluoromethyl)sulphonyl]amino]phenyl]-5-benzofuranyl]methyl]-2-ethyl-4-methyl-1H-imidazol-5-yl]methylene]-N-ethyl-2-thiophenepropanamide;

or a physiologically acceptable salt, solvate or metabolically labile ester thereof.

European Patent Office

# EUROPEAN SEARCH REPORT

Application Number

EP 92 30 4404

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-9 100 281 (E.I. DU PONT DE NEMOURS AND COMPANY)<br>* page 5, line 33 - page 15, line 8 *<br>--- | 1,20 | C07D405/14<br>C07D457/04<br>C07D405/06<br>A61K31/55<br>A61K31/505<br>A61K31/415 |
| D,Y | EP-A-0 425 211 (SMITHKLINE BEECHAM CORPORATION)<br>* abstract; claims 1,6-10; example 6 *<br>--- | 1,18-20 | |
| D,Y | EP-A-0 401 030 (MERCK & CO. INC.)<br>* page 3, line 27 - page 4, line 20; claims 1-6,10 *<br>--- | 1,18-20 | |
| P,Y | EP-A-0 434 249 (GLAXO GROUP LIMITED)<br>* the whole document * | 1,18-20 | |
| P,X | * claims 20,21 *<br>----- | 20,21 | |

TECHNICAL FIELDS SEARCHED (Int. Cl.5)

C07D

The present search report has been drawn up for all claims

| Place of search | Date of completion of the search | Examiner |
|---|---|---|
| THE HAGUE | 07 JULY 1992 | PAISDOR B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)